# FASCICULE DE BREVET EUROPEEN

(11) **EP 1 861 403 B1**
(45) Date de publication et mention de la délivrance du brevet: **02.05.2012**
(21) Numéro de dépôt: 06726093.5
(22) Date de dépôt: 15.03.2006
(51) Int. Cl.: C07D 471/04, A61K 31/437, A61P 35/00, A61P 29/00, A61P 27/02, A61P 19/02, A61P 9/00, A61P 3/04

(54) **NOUVEAUX DERIVES D'IMIDAZO[1,5-a]PYRIDINES, LEUR PROCEDE DE PREPARATION ET LES COMPOSITIONS PHARMACEUTIQUES LES CONTENANT**
NEUE IMIDAZO[1,5-A]PYRIDINDERIVATE, VERFAHREN ZU DEREN HERSTELLUNG UND PHARMAZEUTISCHE ZUSAMMENSETZUNGEN, DIE DIESE ENTHALTEN
NOVEL IMIDAZO[1,5-A]PYRIDINE DERIVATIVES, METHOD FOR PREPARING SAME AND PHARMACEUTICAL COMPOSITIONS CONTAINING SAME

(30) Priorité: 16.03.2005 FR 0502590
(43) Date de publication de la demande: 05.12.2007
(73) Titulaire: SANOFI, 75008 Paris (FR)
(72) Inventeur: BORDES, Marie-Françoise, 31860 Labarthe sur Leze (FR); BONO, Françoise, F-31300 Toulouse (FR); BADORC, Alain, 31120 Roquettes (FR); ALCOUFFE, Chantal, 31120 Roquettes (FR)
(74) Mandataire: Le Coupanec, Pascale A.M.P.
(86) Numéro de dépôt international: PCT/FR2006/000567
(87) Numéro de publication internationale: WO 2006/097625

(56) Documents cités:
- WO-A-03/084956
- THOMPSON A M ET AL: "3-(3,5-DIMETHOXYPHENYL)-1,6-NAPHTHYRIDINE -2,7-DIAMINES AND REALTED 2-UREA DERIVATIVES ARE POTENT AND SELECTIVE INHIBITORS OF THE FGF RECEPTOR-1 TYROSINE KINASE" JOURNAL OF MEDICINAL CHEMISTRY, AMERICAN CHEMICAL SOCIETY. WASHINGTON, US, vol. 22, no. 43, 2000, pages 4200-4211, XP002951686 ISSN: 0022-2623 cité dans la demande

## Description

La présente invention a pour objet de nouveaux dérivés d'imidazo[1,5-a]pyridines, inhibiteurs des FGFs (Fibroblast Growth Factor), leur procédé de préparation et les compositions pharmaceutiques les contenant.

Les FGFs sont une famille de polypeptides synthétisés par un grand nombre de cellules lors du développement embryonnaire et par des cellules des tissus adultes dans diverses conditions pathologiques.

On connaît certains dérivés de naphtyridine diamines et des urées correspondantes qui sont des inhibiteurs sélectifs de FGF-1 (Batley B. et al., Life Sciences, (1998), vol. 62 n°2, pp143-150 ; Thompson A. et al., J. Med. Chem., (2000), vol. 43, pp4200-4211).

Des dérivés d'indolizine, antagonistes de la liaison des FGFs à leurs récepteurs sont décrits dans les demandes de brevets internationales WO 03/084956 et WO 2005/028476.

Il a été maintenant trouvé que des composés, dérivés d'imidazo[1,5-a]pyridines, présente une puissante activité antagoniste de la liaison des FGFs à leurs récepteurs, ainsi qu'une très bonne activité in vivo. En effet et de manière surprenante, dans les modèles in vivo chez la souris, la dose de 10 mg/kg nous permet d'obtenir une activité maximale des composés. Cet effet n'était obtenu qu'à la dose de 50 mg/kg avec la série indolizine décrite dans les demandes de brevets internationales WO 03/084956 et WO 2005/028476.

Ainsi, la présente invention a pour objet des nouveaux dérivés d'imidazo[1,5-a]pyridines de formule I : 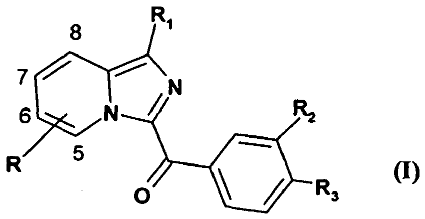 dans laquelle :
- **R**, présent sur les positions 5, 6, 7 ou 8 de l'imidazo[1,5-a]pyridine, représente un atome d'hydrogène, un atome d'halogène, un radical alkyle de 1 à 5 atomes de carbone, un radical hydroxy, un radical alcoxy de 1 à 5 atomes de carbone, un radical -COOR₆ ou un radical de formule :
- -NR₄R₅
- -NH-SO₂-Alk
- -NH-CO-Alk
- -NR₆-CO₂-Alk
- -O-Alk-COOR₆
- -O-Alk-NR₄R₅
- -O-(CH₂)ₙ-Ph
- -CO-NR₄R₅, ou
- -CO-NH-CH(R₇)-(CH₂)ₘ-COOR₆
dans lesquels :
- Alk représente un radical alkyle ou un radical alkylène de 1 à 5 atomes de carbone,
- n représente un nombre entier de 1 à 5,
- m représente un nombre entier de 0 à 4,
- R₄ et R₅ représentent indépendamment l'un de l'autre un atome d'hydrogène, un radical alkyle de 1 à 5 atomes de carbone ou un radical benzyle,
- R₆ représente un atome d'hydrogène ou un radical alkyle de 1 à 5 atomes de carbone,
- R₇ représente un atome d'hydrogène, un radical alkyle de 1 à 5 atomes de carbone ou un radical de formule :
   - -Alk-CONR₄R₅
   - -Alk-OR₆
   - -Alk-NR₄R₅
   - -Ph, ou
   - -CH₂Ph, et
- Ph représente un radical phényle éventuellement substitué par un ou plusieurs groupes choisis parmi les atomes d'halogène, les radicaux alcoxy de 1 à 5 atomes de carbone et les radicaux -COOR₆, où R₆ est tel que défini ci-dessus ;
- **R**₁ représente un atome d'hydrogène, un atome d'halogène, un radical cyano, un radical -COOR₆ ou un radical de formule :
   - -NR₄R₅
   - -NH-SO₂-Alk
   - -NH-CO-CF₃
   - -NH-CO-Ph
   - -NH-CO-Alk
   - -NH-CO₂-Alk
   - -CONR₄R₅
   - un radical phényle éventuellement substitué par un ou plusieurs groupes choisis parmi les atomes d'halogène, les radicaux alkyles de 1 à 5 atomes de carbone, les radicaux alcoxy de 1 à 5 atomes de carbone et les radicaux -COOR₆,
   - un radical hétéroaryle à 5 chaînons comportant un hétéroatome choisi parmi un atome de soufre, un atome d'oxygène ou un atome d'azote, et comportant éventuellement un second atome d'azote, ledit hétéroaryle étant éventuellement substitué par un ou plusieurs groupes choisis parmi les atomes d'halogène, les radicaux alkyles de 1 à 5 atomes de carbone, les radicaux alcoxy de 1 à 5 atomes de carbone et les radicaux -COOR₆, ou
   - un radical hétéroaryle à 6 chaînons comportant 1 ou 2 atomes d'azote et étant éventuellement substitué par un ou plusieurs groupes choisis parmi les atomes d'halogène, les radicaux alkyles de 1 à 5 atomes de carbone, les radicaux alcoxy de 1 à 5 atomes de carbone et les radicaux -COOR₆,
      dans lesquels Alk, Ph, R₄, R₅ et R₆ sont tels que définis dans ce qui précède ;
- **R₂** et **R₃** représentent indépendamment l'un de l'autre un radical hydroxy, un radical alcoxy de 1 à 5 atomes de carbone, un radical amino, un radical -COOR₆, un radical nitro ou un radical de formule :
   - NR₄R₅
   - -NH-CO-Alk
   - -NH-CO-Ph
   - -NH-CO₂-Alk
   - -NH-SO₂-Alk
   - -CO-NR₄R₅, ou
   - -CO-NHOH
   dans lesquels Alk, Ph, R₄, R₅ et R₆ sont tels que définis dans ce qui précède ;
   ou bien **R₂** et **R₃** forment ensemble, avec les atomes de carbone du noyau phényle auquel ils sont rattachés, un cycle carboné à 6 chaînons, comportant un atome d'azote et un autre hétéroatome tel que l'oxygène.

Les composés de formule I peuvent exister à l'état de bases ou salifiés par des acides ou des bases, notamment des acides ou des bases pharmaceutiquement acceptables. De tels sels d'addition font également partie de l'invention.

Les composés selon l'invention peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un radical alkyle : un radical aliphatique saturé, linéaire ou ramifié, pouvant comporter de 1 à 5 atomes de carbone. A titre d'exemple, on peut citer les radicaux méthyle, éthyle, n-propyle, isopropyle, n-butyle, isobutyle, tertbutyle, 2,2-diméthylpropyle;
- un radical alkylène : un radicale alkyle tel que précédemment défini, qui est saturé, linéaire ou ramifié, et qui est divalent. A titre d'exemple, on peut citer les radicaux méthylène, éthylène et propylène ;
- un radical alcoxy : un radical -O-alkyle où le groupe alkyle est tel que précédemment défini et peut comporter de 1 à 5 atomes de carbone. A titre d'exemple, on peut citer les radicaux méthoxy, éthoxy et propoxy ;
- un atome d'halogène : un fluor, un chlore, un brome ou un iode ;
- un hétéroatome : un atome d'azote, d'oxygène ou de soufre ;
- un radical hétéroaryle à 5 chaînons : un radical aromatique cyclique comprenant 5 chaînons et comportant un hétéroatome tel que précédemment défini, ainsi éventuellement qu'un second hétéroatome étant un atome d'azote, le dit radical aromatique étant éventuellement substitué. A titre d'exemple, on peut citer un radical thiènyle, furyle et pyrrolyle ; et
- un radical hétéroaryle à 6 chaînons : un radical aromatique cyclique comprenant 6 chaînons et comportant 1 ou 2 atomes d'azote, éventuellement substitué. A titre d'exemple, on peut citer un radical pyridinyle.

Parmi les composés objets de l'invention, on peut citer un second groupe de composés de formule I dans laquelle :
- **R**, présent sur les positions 6, 7 ou 8 de l'imidazo[1,5-a]pyridine, représente un atome d'hydrogène, un radical alkyle de 1 à 5 atomes de carbone, un radical alcoxy de 1 à 5 atomes de carbone, un radical hydroxy, un radical -COOR₆ ou un radical de formule :
   - -NR₄R₅
   - -NH-SO₂-Alk
   - -NH-CO-Alk
   - -NR₆-CO₂-Alk
   - -O-Alk-COOR₆
   - -O-Alk-NR₄R₅
   - -O-CH₂-Ph
   - -CO-NR₄R₅, ou
   - -CO-NH-CH(R₇)-(CH₂)ₘ-COOR₆
   dans lesquels Alk, Ph, R₄, R₅, R₆, R₇ et m sont tels que définis dans ce qui précède ;
- **R₁** représente un atome d'hydrogène, un atome d'halogène, un radical cyano, un radical -COOR₆ ou un radical de formule :
   - -NR₄R₅
   - -NH-SO₂-Alk
   - -NH-CO-CF₃
   - -NH-CO-Ph
   - -NH-CO-Alk
   - -CO-NR₄R₅
   - un radical phényle éventuellement substitué par un ou deux groupes choisis parmi les atomes d'halogène, les radicaux alcoxy de 1 à 5 atomes de carbone et les radicaux -COOR₆ ;
   - un radical hétéroaryle à 5 chaînons comportant un hétéroatome choisi parmi un atome de soufre, un atome d'oxygène ou un atome d'azote, et comportant éventuellement un second atome d'azote, ledit hétéroaryle étant éventuellement substitué par un ou deux groupes choisis parmi les atomes d'halogène, les radicaux alkyles de 1 à 5 atomes de carbone, les radicaux alcoxy de 1 à 5 atomes de carbone et les radicaux -COOR₆, ou
   - un radical hétéroaryle à 6 chaînons comportant 1 ou 2 atomes d'azote et étant éventuellement substitué par un ou deux groupes choisis parmi les atomes d'halogène, les radicaux alkyles de 1 à 5 atomes de carbone, les radicaux alcoxy de 1 à 5 atomes de carbone et les radicaux -COOR₆,
      où Alk, Ph et R₆ sont tels que définis dans ce qui précède ;
- **R₂** et **R₃** représentent indépendamment l'un de l'autre un radical alcoxy de 1 à 5 atomes de carbone, un radical -COOR₆, un radical amino, un radical nitro ou un radical de formule :
   - -NR₄R₅
   - -NH-CO-Alk
   - -NH-CO-Ph
   - -NH-SO₂-Alk
   dans lesquels Alk, Ph, R₄, R₅ et R₆ sont tels que définis dans ce qui précède.

Parmi ce second groupe de composés selon l'invention, on peut notamment citer ceux dans lesquels R₇ représente un atome d'hydrogène, un radical alkyle de 1 à 5 atomes de carbone ou un radical de formule -Alk-OR₆ ou -CH₂-Ph.

Parmi ce second groupe de composés selon l'invention, on peut également citer ceux dans lesquels m = 0 ou 1.

Parmi les composés objets de l'invention, on peut citer un troisième groupe de composés de formule I dans laquelle :
- **R**, présent sur les positions 6, 7 ou 8 de l'imidazo[1,5-a]pyridine, représente un atome d'hydrogène, un radical alcoxy de 1 à 5 atomes de carbone, un radical hydroxy, un radical -COOR₆ ou un radical de formule :
   - -NR₄R₅
   - -NH-SO₂-Alk
   - -NH-CO-Alk
   - -NR₆-CO₂-Alk
   - -O-Alk-COOR₆
   - -CO-NR₄R₅, ou
   - -CO-NH-CH(R₇)-(CH₂)ₘ-COOR₆
   dans lesquels m représente 0 ou 1, R₇ représente un atome d'hydrogène, un radical alkyle de 1 à 5 atomes de carbone ou un radical de formule -Alk-OR₆ ou -CH₂-Ph, et Alk, R₄, R₅ et R₆ sont tels que définis dans ce qui précède ;
- **R₁** représente un atome d'hydrogène, un atome d'halogène, un radical cyano, un radical -COOR₆ ou un radical de formule :
   - -NR₄R₅
   - -NH-SO₂-Alk
   - -NH-CO-Ph
   - -NH-CO-Alk
   - un radical phényle éventuellement substitué par un ou deux groupes choisis parmi les atomes d'halogène, les radicaux alcoxy de 1 à 5 atomes de carbone et les radicaux -COOR₆,
   - un radical hétéroaryle choisi parmi les radicaux thiényle, furyle et pyrrolyle, le dit hétéroaryle étant éventuellement substitué par un ou deux groupes choisis parmi les radicaux alcoxy de 1 à 5 atomes de carbone et les radicaux -COOR₆, ou
   - un radical pyridinyle éventuellement substitué par un ou deux groupes choisis parmi les radicaux alcoxy de 1 à 5 atomes de carbone et les radicaux -COOR₆ ;
   dans lesquels Alk, Ph, R₄ et R₆ sont tels que définis dans ce qui précède ;
- **R₂** et **R₃** représentent indépendamment l'un de l'autre un radical alcoxy de 1 à 5 atomes de carbone, un radical -COOR₆, un radical nitro, un radical amino, ou un radical de formule NH-CO-Alk, -NH-CO-Ph ou -NH-SO₂Alk ;
dans lesquels Alk, Ph et R₆ sont tels que définis dans ce qui précède.

Parmi l'ensemble des composés de formule I selon l'invention tels que définis ci-avant, on peut notamment citer ceux dans lesquels R₂ représente un radical alcoxy de 1 à 5 atomes de carbone ou un radical -COOR₆, où R₆ est tel que défini dans ce qui précède.

Parmi l'ensemble des composés de formule I selon l'invention tels que définis ci-avant, on peut encore citer ceux dans lesquels R₃ représente un radical nitro, un radical amino ou un radical de formule -NH-CO-Alk, -NH-CO-Ph ou -NH-SO₂Alk, où Alk et Ph sont tels que définis dans ce qui précède. Avantageusement, R₃ représente un radical amino.

Parmi les composés objets de l'invention, on peut citer un quatrième groupe de composés de formule I dans laquelle :
- **R**, présent sur les positions 6, 7 ou 8 de l'imidazo[1,5-a]pyridine, représente un atome d'hydrogène, un radical hydroxy, un radical -COOR₆ ou un radical de formule :
   - -O-Alk-COOR₆
   - -CO-NR₄R₅, ou
   - -CO-NH-CH(R₇)-COOR₆
   dans lesquels R₇ représente un atome d'hydrogène, un radical alkyle de 1 à 5 atomes de carbone ou un radical de formule -Alk-OR₆, et Alk, R₄, R₅ et R₆ sont tels que définis dans ce qui précède ;
- **R₁** représente un atome d'hydrogène, un atome d'halogène, un radical -COOR₆ ou un radical de formule :
   - -NH-CO-Ph
   - un radical phényle éventuellement substitué par un ou deux groupes choisis parmi les atomes d'halogène, les radicaux alcoxy de 1 à 5 atomes de carbone et les radicaux -COOR₆, ou
   - un radical thiényle éventuellement substitué par un ou deux groupes choisis parmi les radicaux alcoxy de 1 à 5 atomes de carbone et les radicaux -COOR₆, dans lesquels Ph et R₆ sont tels que définis dans ce qui précède ;
- **R₂** représente un radical alcoxy de 1 à 5 atomes de carbone ou un radical -COOR₆, où R₆ est tel que défini dans ce qui précède ; et
- **R₃** représente un radical amino.

Parmi les composés objets de l'invention, on peut notamment citer les composés suivants :
- acide 2-amino-5-{(imidazo[1,5-a]pyridin-3-yl)carbonyl}benzoïque ;
- acide 2-amino-5{[1-(4-méthoxyphényl)imidazo[1,5-a]pyridin-3-yl]carbonyl}benzoïque;
- acide 2-amino-5{[1-(3-méthoxyphényl)imidazo[1,5-a]pyridin-3-yl]carbonyl} benzoïque;
- (4-amino-3-méthoxyphényl)(1-bromoimidazo[1,5-a]pyridin-3-yl)méthanone ;
- acide 3-(4-amino-3-méthoxybenzoyl)imidazo [1,5-a]pyridine-8-carboxylique ;
- acide 5-[3-(4-amino-3-méthoxybenzoyl)imidazo[1,5-a]pyridin-1-yl)]thiophène-2-carboxylique ;
- acide 3-(4-amino-3-méthoxybenzoyl)imidazo[1,5-a]pyridine-1-carboxylique ;
- *N*-[3-(4-amino-3-méthoxybenzoyl)imidazo[1,5-a]pyridin-1-yl]-3-méthoxybenzamide ;
- acide 3-(4-amino-3-méthoxybenzoyl)imidazo [1,5-a]pyridine-6-carboxylique ;
- acide 3-(4-amino-3-méthoxybenzoyl)imidazo [1,5-a]pyridine-7-carboxylique.
- acide 3-[3-(4-amino-3-méthoxybenzoyl)imidazo[1,5-a]pyridin-1-yl]benzoïque
- (4-amino-3-méthoxyphényl)[1-(3-fluorophényl)imidazo[1,5-a]pyridin-3-yl]méthanone
- acide 3-{3-(4-amino-3-méthoxybenzoyl)-7-[(méthylamino)carbonyl]imidazo[1,5-a] pyridin-1-yl}benzoïque
- (4-amino-3-méthoxyphényl)(8-hydroxyimidazo [1,5-a]pyridin-3-yl)méthanone
- (4-amino-3-méthoxyphényl)(7-hydroxyimidazo [1,5-a]pyridin-3-yl)méthanone
- acide {[3-(4-amino-3-méthoxybenzoyl)imidazo[1,5-a]pyridin-7-yl]oxy}acétique
- acide 3-(4-amino-3-méthoxybenzoyl)-1-(3-méthoxyphényl)imidazo[1,5-a]pyridine-7-carboxylique
- *N*-{[3-(4-amino-3-méthoxybenzoyl)imidazo[1,5-a]pyridin-7-yl]carbonyl}-D-alaninate de méthyle
- *N*-{[3-(4-amino-3-méthoxybenzoyl)imidazo[1,5-a]pyridin-6-yl]carbonyl}-L-sérine.

Dans ce qui suit, selon les significations des différentes substitutions R, R₁, R₂ et R₃, les composés de formule I seront nommés Ia, Ib, Ic, Id, Ie, If, Ig, Ih, Ii, Ij, Ik, Is, Im, In, Io, Ip, Iq, Ir, It, Iu, Iv, Iw, Ix, Iy, Iz et Iz'.

La présente invention concerne également un procédé de préparation des composés de formule I caractérisé en ce que :
**A)** on condense le composé de formule II : 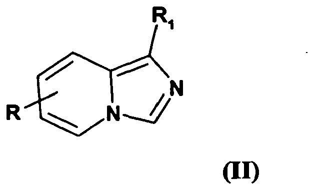 dans laquelle R est tel que défini pour le composé de formule I, mais R est différent d'un radical susceptible de réagir avec les composés de formule III, tel qu'un radical hydroxy, un radical carboxy ou un radical -NR₄R₅, et R est différent d'un radical -NH-CO₂R₆ ou d'un radical -CONR₄R₅, R₁ représentant avantageusement un atome d'hydrogène,
   avec le composé de formule III : 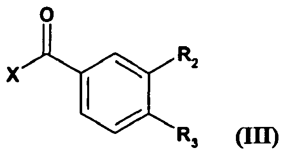 dans laquelle X représente un atome d'halogène et R₂ et R₃ représentent indépendamment l'un de l'autre un radical alcoxy de 1 à 5 atomes de carbone, un radical nitro ou un radical -COOR₆, où R₆ représente un radical alkyle de 1 à 5 atomes de carbone, pour obtenir :
   - les composés de formule Ia, qui sont des composés de formule I dans laquelle R₂ ou R₃ représente un radical nitro, ou
   - les composés de formule Ib, qui sont des composés de formule I dans laquelle R₂ ou R₃ représente un radical -COOR₆, où R₆ représente un radical alkyle de 1 à 5 atomes de carbone,
   et ensuite :
   a) on soumet les composés de formule Ia à une réaction de réduction pour obtenir les composés de formule Id : 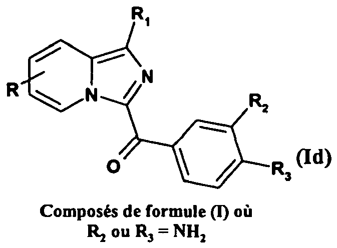 dans laquelle R et R₁ sont tels que définis pour le composé de formule Ia et R₂ ou R₃ représente un radical amino ;
      les composés de formule Id peuvent ensuite être soumis à une réaction d'alkylation, d'acylation ou de sulfonylation pour obtenir les composés de formule Ig : 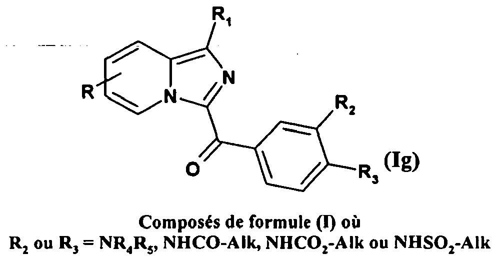 dans laquelle R et R₁ sont tels que définis pour le composé de formule Id et R₂ ou R₃ représente un radical -NR₄R₅, -NHCOAlk, -NHCO₂Alk ou -NHSO₂Alk ;
   b) ou on soumet les composés de formule Ib à une réaction de saponification pour obtenir les composés de formule Ie : 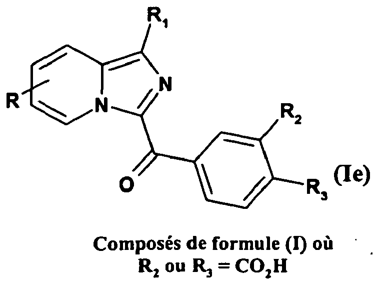
   dans laquelle R et R₁ sont tels que définis pour le composé de formule Ib et R₂ ou R₃ représente un radical carboxy,
   les composés de formule Ie peuvent ensuite être soumis à une réaction de couplage après activation de la fonction carboxy avec par exemple le réactif BOP [hexafluorophosphate de benzotriazol-1-yloxytris (diméthylamino)phosphonium] en présence d'une base telle que la triéthylamine, selon le mode opératoire décrit dans Tetrahedron Letters ; (1975), 14, 1219-1222, puis ajout d'une amine de formule HNR₄R₅ ou de l'hydroxylamine pour obtenir les composés de formule Ih : 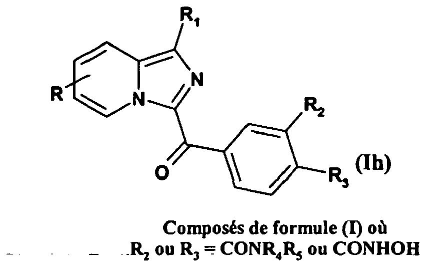 dans laquelle R et R₁ sont tels que définis pour les composés de formule Ie et R₂ ou R₃ représente un radical -CONR₄R₅ ou -CONHOH ;
   **OU**
**B)** on condense le composé de formule II tel que précédemment défini au point A) avec le composé de formule III' : 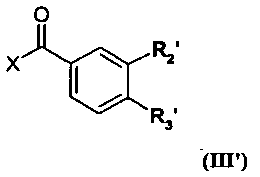 dans laquelle X représente un atome d'halogène et R₂' et R₃' forment ensemble, avec les atomes de carbone du noyau phényle auquel ils sont rattachés, un cycle carboné à 6 chaînons, comportant un atome d'azote et un autre hétéroatome tel que l'oxygène,
   pour obtenir les composés de formule Ic : 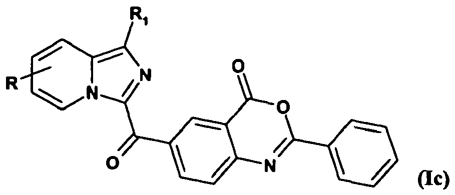 dans laquelle R et R₁ sont tels que définis pour le composé de formule II,
   lesdits composés de formule Ic étant ensuite soumis à une réaction d'alcoolyse pour donner les composés de formule If qui suit : 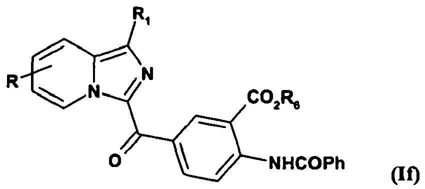 dans laquelle R et R₁ sont tels que définis pour le composé de formule II et R₆ est tel que défini pour le composé de formule I,
   les composés If peuvent ensuite être saponifiés pour obtenir les composés de formules Id ou Ie, dans lesquelles R et R₁ sont tels que définis pour les composés de formule II, R₂ représente un radical -COOH et R₃ représente un radical -NH₂ ;
   **OU**
**C)** on soumet le composé de formule I dans lequel R₁ représente un atome d'hydrogène, tel qu'obtenu précédemment au point A), à une réaction de bromation pour obtenir les composés de formule Ii : 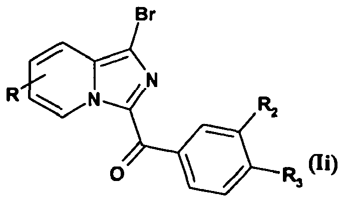 dans laquelle R, R₂ et R₃ sont tels que définis pour le composé de formule I (lorsque R₂ et R₃ ne forment pas ensemble un hétéroaryle) et R₁ représente un atome de brome,
   les composés de formule Ii pour lesquels R est différent d'un atome de brome ou d'un atome d'iode peuvent ensuite être soumis, en présence d'un catalyseur au palladium, d'un ligand et d'une base :
   a) soit à une réaction d'imination avec la benzophénone imine selon les conditions de réaction décrite dans Tetrahedron. ; (2003), 59(22), 3925-3936, suivie d'une réaction d'hydrolyse acide, pour obtenir les composés de formule Ij : 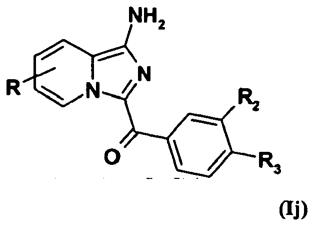 dans laquelle R, R₂ et R₃ sont tels que définis pour les composés de formule Ii et R₁ représente un radical -NH₂,
   b) soit à une réaction de cyanation avec du cyanure de zinc selon les conditions de réaction décrite dans J. Med. Chem. ; (2003), 46, 265-283, pour obtenir les composés de formule Ik: 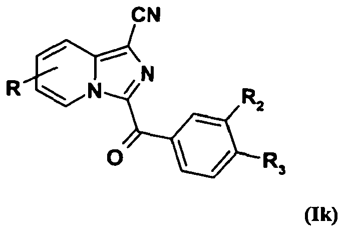 dans laquelle R, R₂ et R₃ sont tels que définis pour les composés de formule Ii et R₁ représente un radical -CN,
      - les composés de formule Ik peuvent ensuite être soumis à une réaction d'hydrolyse basique, afin d'obtenir les composés de formule Im : 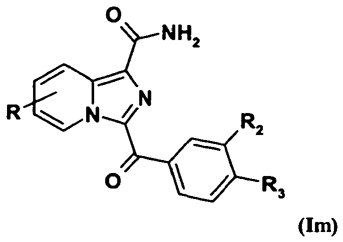 dans laquelle R, R₂ et R₃ sont tels que définis pour les composés de formule Ik et R₁ représente un radical -CONH₂,
      - ou bien encore, les composés de formule Ik sont soumis à une réaction de Pinner [The Chemistry of Amidines and Imidates ; Patai, S., Ed. ; J. Wiley and Sons : New York, (1975) ; 385-489] avec un alcool primaire tel que le méthanol ou l'éthanol en présence de gaz chlorhydrique pour conduire à l'imidoester correspondant, qui par hydrolyse acide conduit aux composés de formule In : 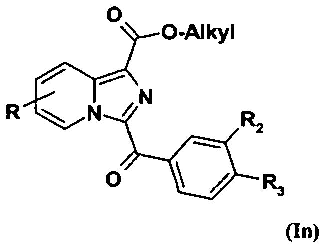
      dans laquelle R, R₂ et R₃ sont tels que définis pour les composés de formule Ik et R₁ représente un radical -CO₂Alk,
      les composés de formule In pouvant eux-même être soumis à une réaction de saponification pour obtenir les composés de formule Io : 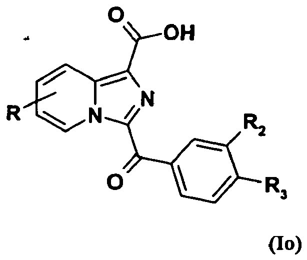 dans laquelle R, R₂ et R₃ sont tels que définis pour les composés de formule Ik et R₁ représente un radical -CO₂H,
   c) soit à une réaction de Suzuki selon les conditions décrites dans Synth. Commun.; (1981), Vol. 11, p513 avec des dérivés phénylboroniques ou hétéroarylboroniques pour obtenir les composés de formule Is : 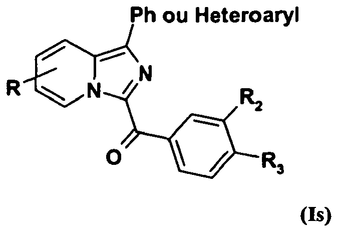 dans laquelle R, R₂ et R₃ sont tels que définis pour le composé de formule Ii et R₁ représente un radical phényle substitué ou un hétéroaryle à 5 ou 6 chainons éventuellement substitué ;
   **OU**
**D)** on soumet les composés de formule Ij dans lequel R₁ représente un radical amino à une réaction d'acylation ou de sulfonylation, pour obtenir les composés de formule Ip : 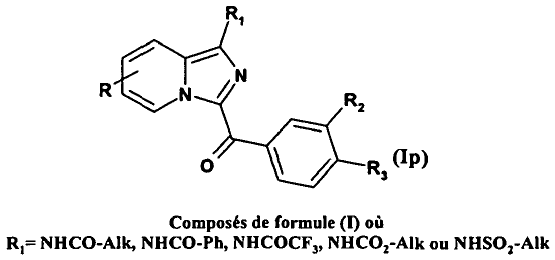 dans laquelle R, R₂ et R₃ sont tels que définis pour les composés de formule Ij et R₁ représente un radical -NHCOAlk, -NHCO₂Alk, -NHSO₂Alk, -NHCOPh ou -NHCOCF₃, dans lesquels Alk et Ph sont tels que définis pour le composé de formule I,
   les composés de formule Ip dans laquelle R₁ représente un radical -NHCOCF₃ pouvant eux-mêmes être soumis à une réaction d'alkylation puis de déprotection, éventuellement suivie d'une autre réaction d'alkylation, pour obtenir les composés de formule Iq : 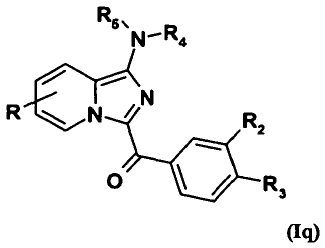 dans laquelle R, R₂, R₃ sont tels que définis pour les composés de formule Ij et R₄ et R₅ sont tels que définis pour le composé de formule I ;
   OU
E) on soumet les composés de formule Ir, dans laquelle R représente un radical -CO₂R₆ et R₆ représente un radical Alk, tels qu'obtenus précédemment au point A) (à savoir par acylation des composés de formule (II), où R = -COOAlk, avec les composés de formule (III)), à une réaction d'hydrolyse acide ou basique pour obtenir les composés de formule It : 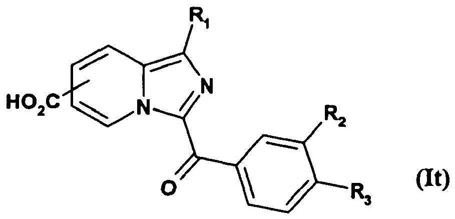 dans laquelle R₁, R₂, R₃ sont tels que définis pour les composés de formule Ir et R représente un radical -COOH,
   les composés de formule It peuvent ensuite être soumis :
   a) soit à une réaction de couplage après activation de la fonction carboxy avec par exemple le réactif BOP [hexafluorophosphate de benzotriazol-1-yloxytris (diméthylamino)phosphonium] en présence d'une base telle que la triéthylamine selon le mode opératoire décrit dans Tetrahedron Letters ; (1975), 14, 1219-1222, puis ajout d'une amine de formule HNR₄R₅ ou d'une amine de formule H₂N-CH(R₇)-(CH₂)ₘ-COOR₆ où R₆ représente un radical Alk, pour obtenir les composés de formule Iu : 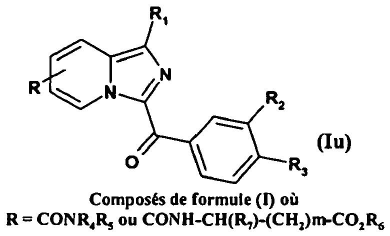 dans laquelle R₁, R₂, R₃ sont tels que définis pour les composés de formule It,
      et lorsque R est un radical -CONH-CH(R₇)-(CH₂)ₘ-COOR₆, où R₆ représente un radical Alk tel que défini pour les composés de formule I, ces composés peuvent être saponifiés pour obtenir les composés de formule Iu où R est un radical -CONH-CH(R₇)-(CH₂)ₘ-COOR₆, où R₆ représente un atome d'hydrogène et R₁, R₂, R₃ sont tels que définis précédemment,
   b) soit être soumis à un réarrangement de Curtius selon le mode opératoire décrit dans Synthesis ; (1990), 295-299 par action de diphénylphosphorylazide en présence de triéthylamine à reflux dans un solvant inerte tel que le toluène puis ajout d'un alcool de formule Alk-OH pour obtenir les composés de formule Iv : 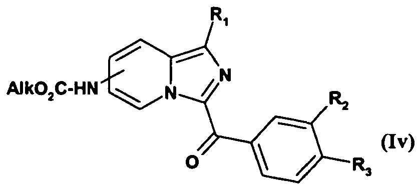 dans laquelle R₁, R₂, R₃ sont tels que définis pour les composés de formule It et R représente un radical -NHCO₂Alk,
      les composés de formule Iv dans lesquels R représente un radical -NH-CO₂-Alk, où Alk représente un radical -tBu, peuvent ensuite conduire aux composés de formule Iw, dans laquelle R₁, R₂, R₃, R₄, R₅ sont tels que définis pour le composé de formule I : 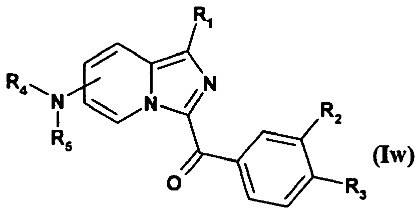
      - par déprotection en milieu acide on obtient les composés de formule Iw où R représente un radical -NH₂,
      - par alkylation suivi d'une déprotection et d'une éventuelle deuxième alkylation on peut obtenir les composés de formule Iw où R représente un radical -NR₄R₅,
         les composés de formule Iw où R représente un radical -NH₂ peuvent ensuite être soit acylés, soit sulfonylés, pour obtenir les composés de formule Ix : 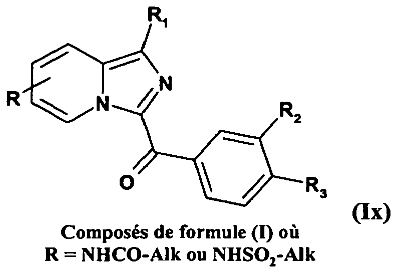
   dans laquelle R₁, R₂, R₃ sont tels que définis pour les composés de formule Iw et R représente un radical -NHCOAlk ou -NHSO₂Alk ;
   **OU**
**F)** on soumet les composés de formule Iy : 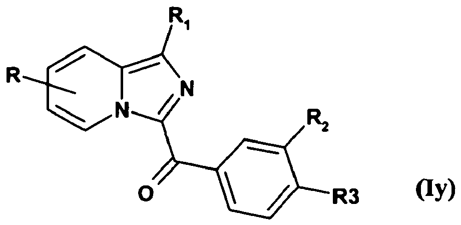 dans laquelle R représente un radical -Obenzyl et R₁, R₂ et R₃ sont tels que définis dans les composés de formule I, à une réaction de débenzylation, par exemple par réaction d'hydrate d'hydrazine dans un solvant protique tel que le méthanol, en présence de palladium sur charbon, pour obtenir les composés de formule Iz : 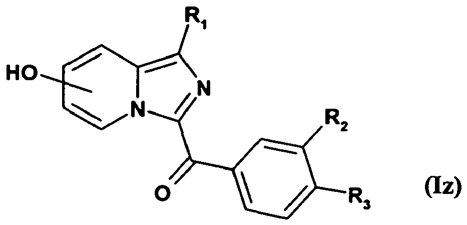 dans laquelle R₁, R₂ et R₃ sont tels que définis pour les composés de formule Iy et R représente un radical hydroxy, et lorsque R₂ ou R₃ représente une fonction nitro on obtient les composés de formule Id dans laquelle R₂ ou R₃ représente un radical NH₂ et R₁ est tel que défini dans les composés de formule I,
   les composés de formule Iz peuvent ensuite être soumis à une réaction de O-alkylation sélective par action à température ambiante d'un halogénure d'alkyle dans un solvant polaire tel que le diméthylformamide, en présence d'un carbonate alcalin, pour obtenir les composés de formule Iz' 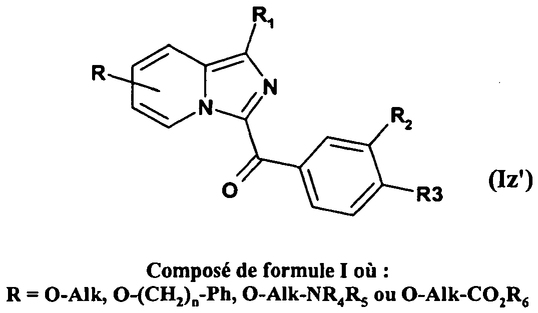 dans laquelle R₁, R₂, R₃ sont tels que définis pour les composés de formule Iz ,
   et lorsque R est un radical -O-Alk-COOR₆, où R₆ représente un radical Alk tel que défini pour les composés de formule I, ces composés peuvent être saponifiés pour obtenir les composés de formule Iz' où R est un radical -O-Alk-COOR₆, où R₆ représente un atome d'hydrogène et R₁, R₂, R₃ sont tels que définis précédemment.

L'Homme du métier saura utiliser les différentes réactions telles que décrites précédemment et illustrées dans les schémas 1 à 6 qui suivent, pour obtenir le composé de formule I en tenant compte des différents radicaux situés sur la molécule et susceptibles de réagir.

Dans les schémas 1 à 6, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les différentes alternatives A, B, C, D, E ou F précédemment décrites sont représentées respectivement par les schémas 1, 2, 3, 4, 5 et 6 qui suivent.

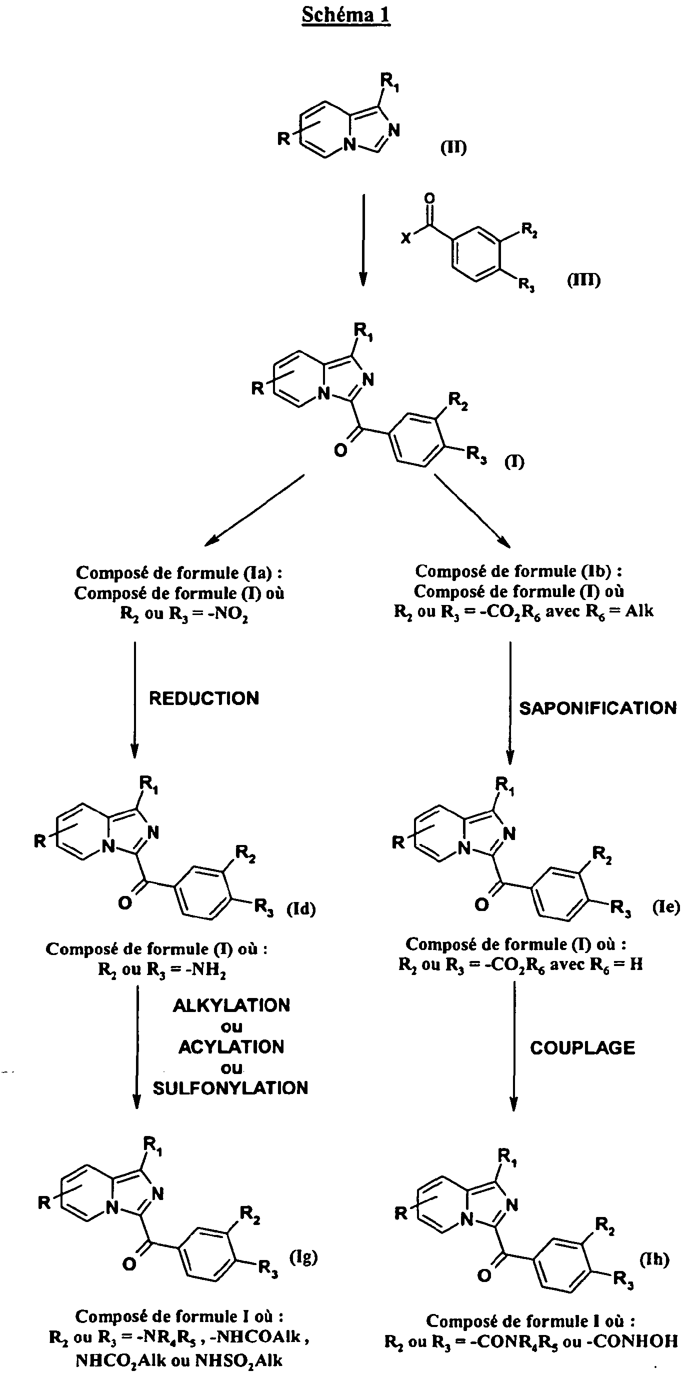

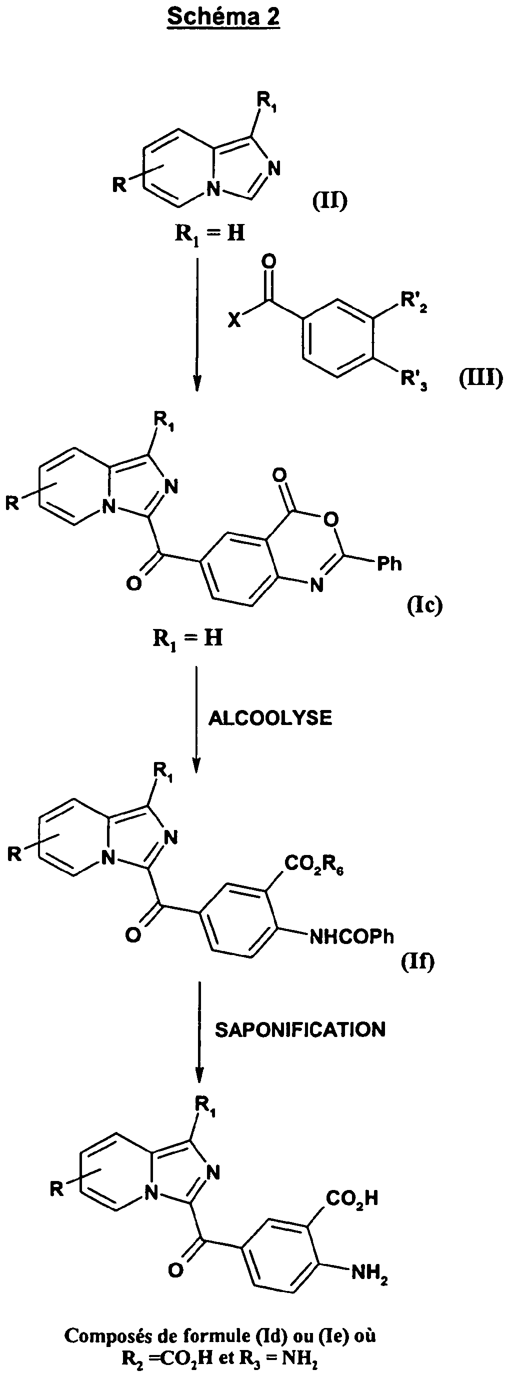

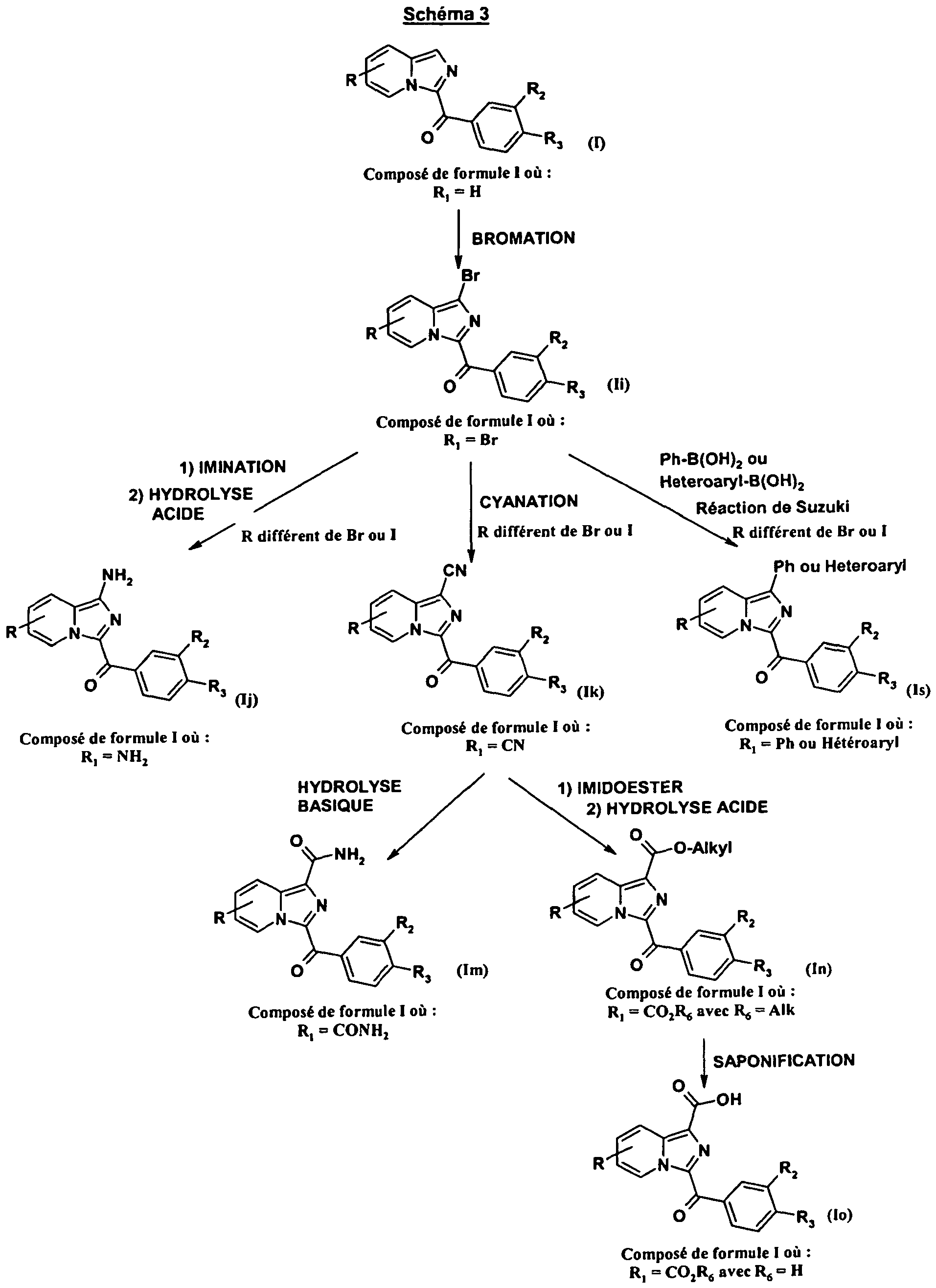

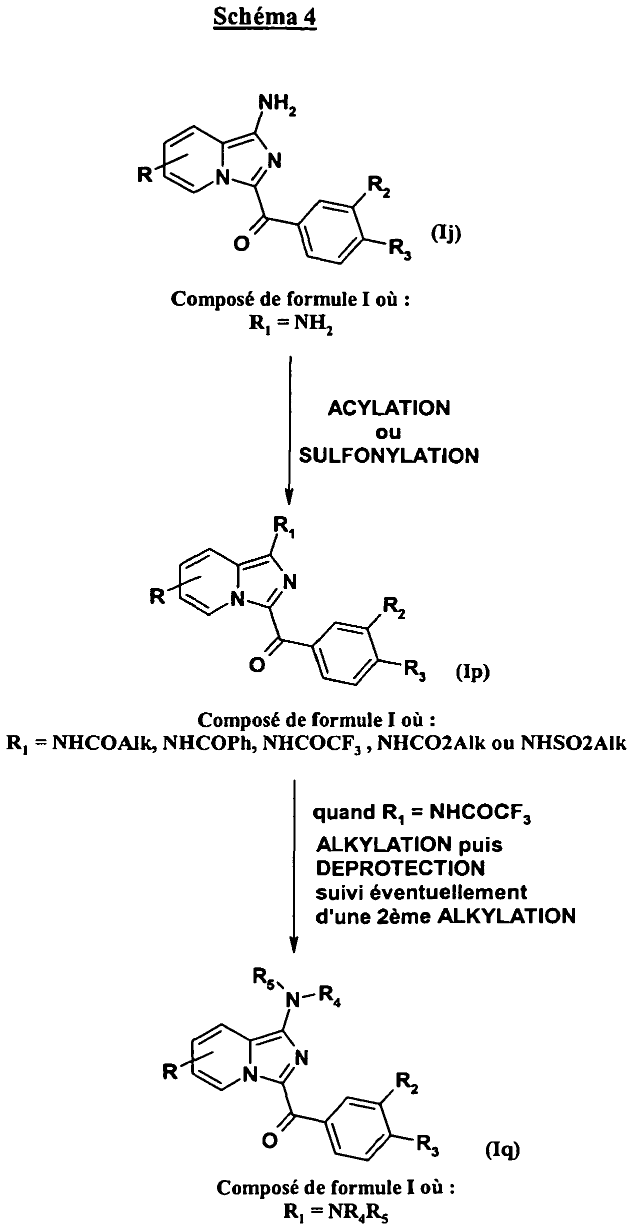

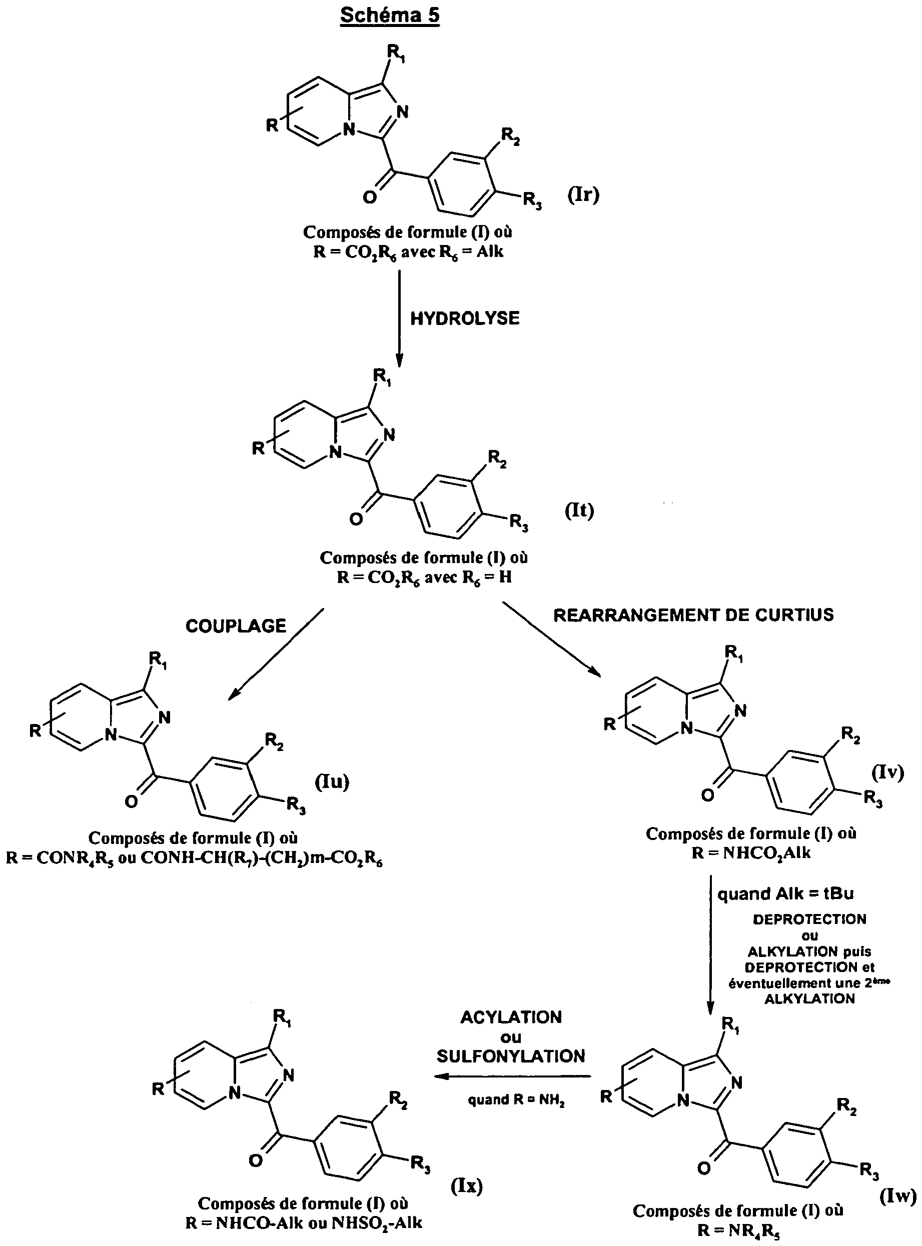

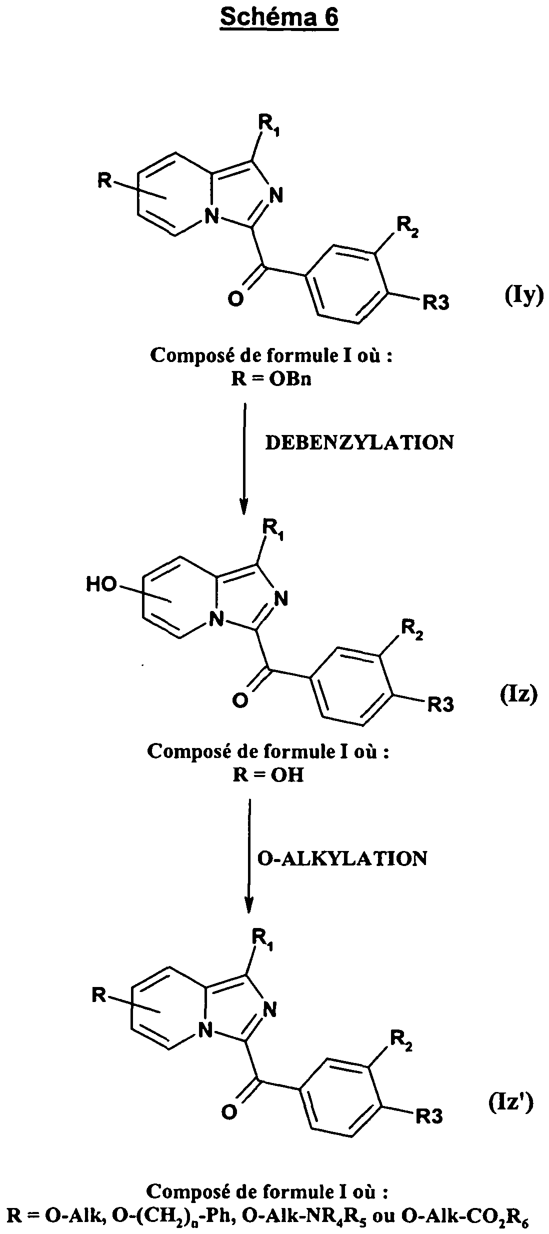

Les composés de formule II, notamment lorsque R₁ = H, sont obtenus par des méthodes connues dans la littérature à partir des 2-aminométhylpyridines convenablement substituées, selon le schéma réactionnel suivant, décrit dans J. Chem. Soc. ; (1955), 2834-2836 :

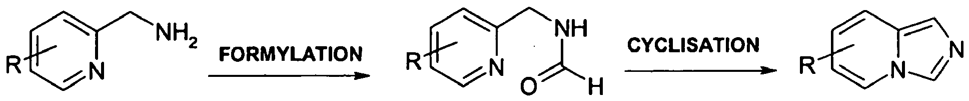

On peut aussi citer trois demandes de brevets décrivant la synthèse d'imidazo[1,5-a]pyridines : WO 03/070732, WO 04/064836 et WO 04/046133

Les composés de formule III dans laquelle R₂ et R₃, identiques ou différents, ont les mêmes définitions que pour les composés de formule Ia ou Ib et X représente un atome de chlore sont obtenus par action du chlorure de thionyle sur les acides benzoïques correspondants, qui sont commerciaux ou décrits dans la littérature.

Les composés de formule III' dans laquelle R'₂ et R'₃ forment ensemble, avec les atomes de carbone du noyau phényle auquel ils sont rattachés, un cycle carboné à 6 chaînons comportant un atome d'azote et un autre hétéroatome tel que l'oxygène et X représente un atome de chlore, peuvent être obtenus par action du chlorure de thionyle sur les acides correspondants décrits dans la littérature. On peut citer par exemple l'acide 4-oxo-2-phényl-4H-3,1-benzoxazine-6-carboxylique, préparé selon la méthode décrite dans le brevet français FR2333511, qui par traitement avec le chlorure de thionyle conduit au chlorure d'acide correspondant, lequel est utilisé pour acyler les composés de formule II et donner les composés de formule Ic.

Les composés de la formule I selon la présente invention sont des antagonistes puissants de FGF-1 et -2. Leurs capacités d'inhiber à la fois la formation de nouveaux vaisseaux à partir de cellules endothéliales différenciées et de bloquer la différentiation de cellules de moelle osseuse humaine adulte CD34+ CD 133+ en cellules endothéliales ont été démontrées *in vitro.* De plus, leur capacité à inhiber l'angiogénèse pathologique a été démontrée *in vivo.* Par ailleurs, il a été démontré que les composés de formule I sont des antagonistes puissants du récepteur FGF-1.

De manière générale, les récepteurs FGFs sont impliqués de façon importante par l'intermédiaire de sécrétions autocrines, paracrines ou juxtacrines dans les phénomènes de dérégulation de la stimulation de la croissance des cellules cancéreuses. De plus, les récepteurs FGFs affectent l'angiogénèse tumorale qui joue un rôle prépondérant à la fois sur la croissance de la tumeur mais aussi sur les phénomènes de métastasisation.

L'angiogénèse est un processus de génération de nouveaux vaisseaux capillaires à partir de vaisseaux préexistants ou par mobilisation et différentiation de cellules de la moelle osseuse. Ainsi, à la fois une prolifération incontrôlée des cellules endothéliales et une mobilisation d'angioblastes à partir de la moelle osseuse sont observées dans les processus de néo-vascularisation des tumeurs. Il a été montré *in vitro* et *in vivo* que plusieurs facteurs de croissance stimulent la prolifération endothéliale, et notamment le récepteur FGF-1 ou a-FGF et le récepteur FGF-2 ou b-FGF. Ces deux facteurs induisent la prolifération, la migration et la production de protéases par les cellules endothéliales en culture et la néo-vascularisation *in vivo.* Les récepteurs a-FGF et b-FGF interagissent avec les cellules endothéliales par l'intermédiaire de deux classes de récepteurs, les récepteurs de haute affinité à activité tyrosine kinase (FGFs) et les récepteurs de basse affinité de type héparane sulfate protéoglycane (HSPGs) situés à la surface des cellules et dans les matrices extracellulaires. Alors que le rôle paracrine de ces deux facteurs sur les cellules endothéliales est largement décrit, les a-FGF et b-FGF pourraient également intervenir sur ces cellules à travers un processus autocrine. Ainsi, les a-FGF et b-FGF et leurs récepteurs représentent des cibles très pertinentes pour les thérapies visant à inhiber les processus d'angiogénèse (Keshet E, Ben-Sasson SA.,. J. Clin. Invest, (1999), vol. 501, pp104-1497 ; Presta M, Rusnati M, Dell'Era P, Tanghetti E, Urbinati C, Giuliani R et al, *New York: Plenum Publishers,* (2000), pp7-34, Billottet C, Janji B, Thiery J.P., Jouanneau J, Oncogene, (2002) vol. 21, pp8128-8139).

Par ailleurs, des études systématiques visant à déterminer l'expression due aux a-FGF et b-FGF et de leurs récepteurs (FGFs) sur différents types de cellules tumorales mettent en évidence qu'une réponse cellulaire à ces deux facteurs est fonctionnelle dans une grande majorité de lignées tumorales humaines étudiées. Ces résultats supportent l'hypothèse qu'un antagoniste des a-FGF et b-FGF pourrait également inhiber la prolifération des cellules tumorales (Chandler LA, Sosnowski BA, Greenlees L, Aukerman SL, Baird A, Pierce GF., Int.J.Cancer, (1999), vol. 58, pp81-451).

Les a-FGF et b-FGF jouent un rôle important dans la croissance et le maintien des cellules de la prostate. Il a été montré à la fois dans des modèles animaux et chez l'homme qu'une altération de la réponse cellulaire à ces facteurs joue un rôle primordial dans la progression du cancer de la prostate. En effet dans ces pathologies on enregistre à la fois une augmentation de la production des a-FGF et b-FGF par les fibroblastes et les cellules endothéliales présentes au niveau de la tumeur et une augmentation de l'expression des récepteurs FGFs sur les cellules tumorales. Ainsi une stimulation paracrine des cellules cancéreuses de la prostate s'opère, et ce processus serait un composant majeur de cette pathologie. Un composé possédant une activité antagoniste des récepteurs FGFs tels que les composés de la présente invention peut représenter une thérapie de choix dans ces pathologies (Giri D, Ropiquet F., Clin.Cancer Res., (1999), vol. 71, pp5-1063 ; Doll JA, Reiher FK, Crawford SE, Pins MR, Campbell SC, Bouck NP., Prostate, (2001), vol. 305, pp 49-293).

Plusieurs travaux montrent la présence de a-FGF et b-FGF et de leurs récepteurs FGFRs à la fois dans les lignées tumorales humaines du sein (notamment MCF7) et dans des biopsies de tumeurs. Ces facteurs seraient responsables dans cette pathologie de l'apparition de phénotype très agressif et induisant une forte métastasisation. Ainsi un composé possédant une activité antagoniste des récepteurs FGFRs, comme les composés de la formule I, peut représenter une thérapie de choix dans ces pathologies (Vercoutter-Edouart A-S, Czeszak X, Crépin M, Lemoine J, Boilly B, Le Bourhis X et al., Exp.Cell Res., (2001), vol. 262, pp59-68).

Les mélanomes cancéreux sont des tumeurs qui induisent avec une fréquence importante des métastases et qui sont très résistantes aux différents traitements de chimiothérapie. Les processus d'angiogénèse joue un rôle prépondérant dans la progression d'un mélanome cancéreux. De plus, il a été montré que la probabilité d'apparition de métastases augmente très fortement avec l'augmentation de la vascularisation de la tumeur primaire. Les cellules de mélanomes produisent et sécrètent différents facteurs angiogéniques dont le a-FGF et le b-FGF. Par ailleurs, il a été montré qu'une inhibition de l'effet cellulaire de ces deux facteurs par le RÉCEPTEUR FGF-1 soluble bloque *in vitro* la prolifération et la survie des cellules tumorales de mélanome et bloque *in vivo* la progression tumorale. Ainsi un composé possédant une activité antagoniste des récepteurs FGFs comme les composés de la présente invention peut représenter une thérapie de choix dans ces pathologies (Rofstad EK, Halsor EF., Cancer Res., (2000) ; Yayon A, Ma Y-S, Safran M, Klagsbrun M, Halaban R., Oncogene, (1997), vol. 14, pp 2999-3009).

Les cellules de gliome produisent *in vitro* et *in vivo* du a-FGF et du b-FGF et possèdent à leur surface, différents récepteurs FGFs. Cela suggère donc que ces deux facteurs par un effet autocrine et paracrine jouent un rôle pivotal dans la progression de ce type de tumeur. De plus, comme la plupart des tumeurs solides, la progression des gliomes et leur capacité à induire des métastases, est très dépendante des processus angiogéniques dans la tumeur primaire. Il a également été montré que des antisens du récepteur FGF-1 bloquent la prolifération d'astrocytomes humains. De plus, des dérivés des naphthalenesulfonates sont décrites pour inhiber les effets cellulaires des a-FGF et b-FGF *in vitro* et l'angiogénèse induite par ces facteurs de croissance *in vivo.* Une injection intracérébrale de ces composés induit une augmentation très significative de l'apoptose et une diminution importante de l'angiogénèse se traduisant par une régression considérable de gliomes chez le rat. Ainsi un composé possédant une activité antagoniste des a-FGF et / ou b-FGF et / ou des récepteurs FGFs, comme les composés de la présente invention, peut représenter une thérapie de choix dans ces pathologies (Yamada SM, Yamaguchi F, Brown R, Berger MS, Morrison RS, Glia, (1999), vol. 76, pp28-66 ; Auguste P, Gürsel DB, Lemière S, Reimers D, Cuevas P, Carceller F et al., Cancer Res., (2001), vol. 26, pp 61-1717).

Plus récemment le rôle potentiel d'agents pro angiogéniques dans les leucémies et lymphomes a été documenté. En effet de manière générale il a été rapporté que des clones cellulaires dans ces pathologies peuvent être soit détruits naturellement par le système immunitaire soit basculer dans un phénotype angiogénique qui favorise leur survie puis leur prolifération. Ce changement de phénotype est induit par une sur expression de facteurs angiogéniques notamment par les macrophages et / ou une mobilisation de ces facteurs à partir de la matrice extracellulaire (Thomas DA, Giles FJ, Cortes J, Albitar M, Kantarjian HM., Acta Haematol, (2001), vol. 207, pp106-190). Parmi les facteurs angiogéniques, le b-FGF a été détecté dans de nombreuses lignées cellulaires tumorales lymphoblastiques et hématopoiétiques. Les récepteurs FGFs sont également présents sur la majorité de ces lignées suggérant un possible effet cellulaire autocrine des a-FGF et b-FGF induisant la prolifération de ces cellules. Par ailleurs il a été rapporté que l'angiogénèse de la moelle osseuse par des effets paracrines était corrélée à la progression de certaines de ces pathologies.

De manière plus particulière il a été montré dans les cellules CLL (chronic lymphocytic leukemia) que le b-FGF induit une augmentation de l'expression de protéine anti apoptotique (Bcl2) conduisant à une augmentation de la survie de ces cellules et participe donc de manière importante à leur cancérisation. De plus, les taux de b-FGF mesurés dans ces cellules sont très bien corrélés avec le stade d'avancement clinique de la maladie et la résistance à la chimiothérapie appliquée dans cette pathologie (fludarabine). Ainsi, un composé possédant une activité antagoniste des récepteurs FGFs, comme les composés de la présente invention, peut représenter une thérapie de choix soit seul soit en association avec la fludarabine ou d'autres produits actifs dans cette pathologie (Thomas

DA, Giles FJ, Cortes J, Albitar M, Kantarjian HM., Acta Haematol, (2001), vol. 207, pp106-190 ; Gabrilove JL, Oncologist, (2001), vol. 6, pp4-7).

Il existe une corrélation entre le processus d'angiogénèse de la moelle osseuse et les "extramedullar disease" dans les CML (chronic myelomonocytic leukemia). Différentes études démontrent que l'inhibition de l'angiogénèse, en particulier par un composé possédant une activité antagoniste des récepteurs FGFs, pourrait représenter une thérapeutique de choix dans cette pathologie.

La prolifération et la migration de cellules musculaires lisses vasculaires contribuent à l'hypertrophie intimale des artères et joue ainsi un rôle prépondérant dans l'athérosclérose et dans la resténose après angioplastie et endoarterectomie.

Des études *in vivo* montrent, après lésion de la carotide par "balloon injury", une production locale de a-FGF et de b-FGF. Dans ce même modèle un anticorps neutralisant anti FGF2 inhibe la prolifération des cellules musculaires lisses vasculaires et diminue ainsi l'hypertrophie intimale.

Une protéine chimérique FGF2 liée à une molécule telle que la saporine inhibe la prolifération des cellules musculaires lisses vasculaires *in vitro* et l'hypertrophie intimale *in vivo* (Epstein CE, Siegall CB, Biro S, Fu YM, FitzGerald D., Circulation, (1991), vol. 87, pp84-778 ; Waltenberger J., Circulation, (1997), pp96-4083).

Ainsi, les antagonistes des récepteurs FGFs, tels que les composés de la présente invention représentent une thérapie de choix, soit seul, soit en association avec des composés antagonistes d'autres facteurs de croissance impliqués dans ces pathologies comme le PDGF, dans le traitement des pathologies liées à la prolifération des cellules musculaires lisses vasculaires telles que l'athérosclérose, la resténose post-angioplastie ou suite à la pose de prothèses endovasculaires (stents) ou lors de pontages aorto-coronariens.

L'hypertrophie cardiaque intervient en réponse à un stress de la paroi ventriculaire induit par une surcharge en terme de pression ou de volume. Cette surcharge peut être la conséquence de nombreux états physio pathologiques comme l'hypertension, l'AC (aortic coarctation), l'infarctus du myocarde, et différents troubles vasculaires. Les conséquences de cette pathologie sont des changements morphologiques, moléculaires et fonctionnels comme l'hypertrophie des myocytes cardiaques, l'accumulation de protéines matricielles et la ré-expression de gènes foetaux. Le b-FGF est impliqué dans cette pathologie. En effet l'addition de b-FGF à des cultures de cardiomyocytes de rat nouveau-né modifie le profil des gènes correspondants aux protéines contractiles conduisant à un profil de gènes de type foetaux. De manière complémentaire des myocytes de rat adulte montrent une réponse hypertrophique sous l'effet du b-FGF, cette réponse etant bloquée par des anticorps neutralisants anti b-FGF. Des expériences réalisées *in vivo* sur des souris transgéniques "knock-out" pour le b-FGF, montrent que le b-FGF est le facteur stimulant majeur de l'hypertrophie des myocyte cardiaque dans cette pathologie (Schultz JeJ, Witt SA, Nieman ML, Reiser PJ, Engle SJ, Zhou M et al., J.Clin. Invest., (1999), vol. 19, pp104-709).

Ainsi un composé, comme les composés de la présente invention, possédant une activité antagoniste des récepteurs FGFs représente une thérapie de choix dans le traitement de l'insuffisance cardiaque et toute autre pathologie associée à une dégénérescence du tissu cardiaque. Ce traitement pourrait être réalisé seul ou en association avec les traitements courants (beta-bloquants, diurétiques, antagonistes d'angiotensine, antiarrythmiques, anti-calciques, anti-thrombotiques etc...)

Les troubles vasculaires dus au diabète se caractérisent par une altération de la réactivité vasculaire et du flux sanguin, une hyperperméabilité, une réponse proliférative exacerbée et une augmentation des dépôts de protéines matricielles. De manière plus précise le a-FGF et le b-FGF sont présents dans les membranes pré rétiniennes de patients ayant des rétinopathies diabétiques, dans les membranes des capillaires sous jacents et dans l'humeur vitrée de malades souffrants de rétinopathies prolifératives. Un récepteur du FGF soluble capable de lier à la fois le a-FGF et le b-FGF est développé dans les troubles vasculaires liés au diabète (Tilton RG, Dixon RAF, Brock TA., Exp. Opin. Invest. Drugs, (1997), vol. 84, pp6-1671). Ainsi un composé comme les composés de formule I possédant une activité antagoniste des récepteurs FGFs représente une thérapie de choix soit seul soit en association avec des composés antagonistes d'autres facteurs de croissance impliqués dans ces pathologies comme le VEGF.

L'arthrite rhumatoïde (RA) est une maladie chronique avec une étiologie inconnue. Alors qu'elle affecte de nombreux organes, la forme la plus sévère de RA est une inflammation synoviale des articulations progressive aboutissant à la destruction. L'angiogénèse semble affecter de manière importante la progression de cette pathologie. Ainsi le a-FGF et le b-FGF ont été détectés dans le tissu synovial et dans le fluide articulaire de patients atteints de RA, indiquant que ce facteur de croissance intervient dans l'initiation et / ou la progression de cette pathologie. Dans des modèles de AIA (adjuvant-induced model of arthritis) chez le rat, il a été montré que la sur-expression de b-FGF augmente la sévérité de la maladie alors qu'un anticorps neutralisant anti b-FGF bloque la progression de la RA (Yamashita A, Yonemitsu Y, Okano S, Nakagawa K, Nakashima Y, Irisa T et al., J.Immunol., (2002), vol. 57, pp 168-450 ; Manabe N, Oda H, Nakamura K, Kuga Y, Uchida S, Kawaguchi H, Rheumatol, (1999), vol. 20, pp38-714). Ainsi les composés selon l'invention représentent une thérapie de choix dans cette pathologie.

Il a été également décrit que les taux de facteurs de croissance ayant une activité pro angiogénique tels les FGF1 et 2 étaient fortement augmentés dans le liquide synovial de patients atteint d'ostéoarthrite. Dans ce type de pathologie on enregistre une modification importante de la balance entre les facteurs pro et anti angiogènes induisant la formation de nouveaux vaisseaux. Et par voie de conséquence la vascularisation de structures non vascularisée tels que les cartilages articulaires ou les disques inter vertébraux. Ainsi, l'angiogenèse représente un facteur clef dans la formation osseuse (ostéophytes) contribuant ainsi à la progression de la maladie. De manière complémentaire, l'innervation des nouveaux vaisseaux peut également contribuer aux douleurs chroniques associées à cette pathologie (Walsh DA., Curr Opin Rheumatol. 2004 Sep;16(5):609-15) Ainsi les composés selon l'invention représentent une thérapie de choix dans cette pathologie

Les IBD (inflammatory bowel disease) comprennent deux formes de maladies inflammatoires chroniques de l'intestin : les UC (ulcerative colitis) et la maladie de Crohn's (CD). Les IBD sont caractérisées par une dysfonction immunitaire se traduisant par une production inappropriée de cytokines inflammatoires induisant l'établissement d'un système micro-vasculaire local. Cette angiogénèse d'origine inflammatoire a pour conséquence une ischémie intestinale induite par vasoconstriction. Des taux circulants et locaux de b-FGF importants ont été mesurés chez des patients atteints de ces pathologies (Kanazawa S, Tsunoda T, Onuma E, Majima T, Kagiyama M, Kkuchi K., American Journal of Gastroenterology, (2001), vol. 28, pp 96-822 ; Thorn M, Raab Y, Larsson A, Gerdin B, Hallgren R., Scandinavian Journal of Gastroenterology, (2000), vol. 12, pp35-408). Les composés de l'invention présentant une activité anti angiogénique importante dans un modèle d'angiogénèse inflammatoire représentent une thérapie de choix dans ces pathologies.

Les récepteurs FGF-1, -2 et -3 sont impliqués dans les processus de chronogénèse et osteogénèse. Des mutations conduisant à l'expression de FGFRs toujours activés ont été reliées à un grand nombre de maladies génétiques humaines se traduisant par des malformations du squelette comme les syndromes de Pfeiffer, Crouzon, Apert, Jackson-Weiss et Bear-Stevenson cutis gyrata. Certaines de ces mutations affectant plus particulièrement le récepteur FGF-3 conduisent notamment à des achondroplasies (ACH), des hypochondroplasies (HCH) et des TD (Thanatophoric dysplasia); ACH étant la forme la plus courante de nanisme. D'un point de vue biochimique l'activation soutenue de ces récepteurs s'effectue par une dimérisation du récepteur en absence de ligand (Chen L., Adar R. , Yang X. Monsonego E.O., LI C., Hauschka P.V, Yagon A. and Deng C.X., (1999), The Journ. Of Clin. Invest., vol. 104, n° 11, pp 1517-1525). Ainsi les composés de l'invention présentant une activité antagoniste de la liaison du b-FGF au récepteur FGF et inhibant ainsi la dimérisation du récepteur représentent une thérapie de choix dans ces pathologies.

Par ailleurs on connaît que le tissu adipeux est un des rares tissus qui chez l'adulte peut se développer ou régresser. Ce tissu est très vascularisé et un réseau très dense de micro vaisseaux entoure chaque adipocyte. Ces observations ont conduit à tester l'effet d'agent anti angiogéniques sur le développement du tissu adipeux chez l'adulte. Ainsi il apparaît que dans des modèles pharmacologiques chez la souris ob/ob, l'inhibition de l'angiogenèse se traduit par une perte significative de poids des souris (Rupnick MA et al, (2002), PNAS, vol. 99, n°16, pp 10730-10735). Ainsi un composé antagoniste des récepteurs FGFs possédant une activité anti angiogénique puissante peuvent représenter une thérapie de choix dans les pathologies liées à l'obésité.

Grâce à leur faible toxicité et leurs propriétés pharmacologiques et biologiques, les composés de la présente invention trouvent leur application dans le traitement de tout carcinome ayant un degré de vascularisation important (poumon, sein, prostate, oesophage) ou induisant des métastases (colon, estomac, mélanome) ou étant sensibles au a-FGF ou au b-FGF de manière autocrine ou enfin dans des pathologies de type lymphomes et leucémies. Ces composés représentent une thérapie de choix soit seul soit en association avec une chimiothérapie adaptée. Les composés selon l'invention trouvent également leur application dans le traitement de maladies cardiovasculaires comme l'athérosclérose, la resténose post angioplastie dans le traitement des maladies liés aux complications apparaissant suite à la pose de prothèses endovasculaires et/ou de pontages aorto-coronariens ou d'autres greffes vasculaires et l'hypertrophie cardiaque ou de complications vasculaires du diabète comme les rétinopathies diabétiques. Les composés selon l'invention trouvent également leur application dans le traitement de maladies inflammatoires chroniques comme l'arthrite rhumatoïde ou les IBD. Enfin les composés selon l'invention peuvent être utilisés dans le traitement des achondroplasies (ACH), des hypochondroplasies (HCH) et des TD (Thanatophoric dysplasia), comme également dans le traitement de l'obésité.

Les produits selon l'invention trouvent également leur application dans le traitement de la dégénérescence maculaire, notamment la dégénérescence maculaire liée à l'âge (ou DMLA). Un caractère majeur de la perte de la vision chez l'adulte est la néo-vascularisation et les hémorragies consécutives qui causent des désordres fonctionnels importants au niveau de l'oeil et qui se traduisent par une cécité précoce. Récemment, l'étude des mécanismes impliqués dans les phénomènes de néo-vascularisation oculaire a permis de mettre en évidence l'implication de facteur pro-angiogéniques dans ces pathologies. En mettant en oeuvre un modèle de néoangiogenèse choroidienne induite par laser il a été possible de confirmer que les produits selon l'invention permettent également de moduler la néo-vascularisation de la choroïde.

Par ailleurs, les produits de l'invention peuvent être utilisés dans le traitement ou la prévention des thrombopénies dues notamment à une chimiothérapie anti-cancéreuse. Il a été en effet démontré que les produits de l'invention peuvent améliorer les taux des plaquettes circulantes lors d'une chimiothérapie.

Ainsi, selon un autre de ses aspects, l'invention a pour objet des médicaments qui comprennent un composé de formule I, ou un sel d'addition de ce dernier à un acide ou à une base pharmaceutiquement acceptable, ou encore un hydrate ou un solvat du composé de formule I.

Selon un autre de ses aspects, la présente invention concerne des compositions pharmaceutiques comprenant, en tant que principe actif, un composé de formule I selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité (par exemple les voies orale, sublinguale, sous-cutanée, intramusculaire, intraveineuse, transdermique, transmuqueux, locale ou rectale), parmi les excipients habituels qui sont connus de l'Homme du métier.

Les compositions pharmaceutiques selon la présente invention sont administrées de préférence par voie orale.

Dans les compositions pharmaceutiques de la présente invention pour l'administration orale, les principes actifs peuvent être administrés sous forme unitaire d'administration, en mélange avec des supports pharmaceutiques classiques. Les formes unitaires d'administration appropriées comprennent par exemple les comprimés éventuellement sécables, les gélules, les poudres, les granules et les solutions ou suspensions orales.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention concerne également une composition pharmaceutique telle que définie plus haut, en tant que médicament.

La présente invention a également pour objet l'utilisation d'un composé de formule I, tel que défini plus haut, pour la préparation d'un médicament utile dans le traitement des maladies nécessitant une modulation des FGF's.

La présente invention a également pour objet l'utilisation d'un composé de formule I, tel que défini plus haut, pour la préparation d'un médicament utile dans le traitement des cancers, notamment des carcinomes ayant un degré de vascularisation important tels que les carcinomes de poumon, sein, prostate et oesophage, des cancers induisant des métastases tels que le cancer du colon et le cancer de l'estomac, des mélanomes, des gliomes, des lymphomes, et des leucémies.

Un composé de formule I selon la présente invention peut être administré seul ou en association avec un ou plusieurs composé(s) possédant une activité anti-angiogène ou avec un ou plusieurs composé(s) cytotoxique(s) (chimiothérapie), ou encore en association avec un traitement par des radiations. Ainsi, la présente invention a également pour objet l'utilisation d'un composé de formule I, tel que défini plus haut, en association avec un ou plusieurs principe(s) actif(s) anticancéreux et/ou avec une radiothérapie.

La présente invention a également pour objet l'utilisation d'un composé de formule I, tel que défini plus haut, pour la préparation d'un médicament utile dans le traitement de maladies cardiovasculaires telles que l'athérosclérose, la resténose post angioplastie, des maladies liés aux complications apparaissant suite à la pose de prothèses endovasculaires et/ou de pontages aorto-coronariens ou d'autres greffes vasculaires de l'hypertrophie cardiaque, ou des complications vasculaires du diabète comme les rétinopathies diabétiques.

La présente invention a également pour objet l'utilisation d'un composé de formule I, tel que défini plus haut, pour la préparation d'un médicament utile dans le traitement de maladies inflammatoires chroniques comme l'arthrite rhumatoïde ou les IBD.

La présente invention a également pour objet l'utilisation d'un composé de formule I, tel que défini plus haut, pour la préparation d'un médicament utile dans le traitement de l'ostéoarthrite, des achondroplasies (ACH), des hypochondroplasies (HCH) et des TD (Thanatophoric dysplasia).

La présente invention a également pour objet l'utilisation d'un composé de formule I, tel que défini plus haut pour la préparation d'un médicament utile dans le traitement de l'obésité.

La présente invention a également pour objet l'utilisation d'un composé de formule I, tel que défini plus haut, pour la préparation d'un médicament utile dans le traitement de la dégénérescence maculaire, tel que la dégénérescence maculaire liée à l'âge (DMLA).

Les compositions selon l'invention, pour une administration orale, contiennent des doses recommandées de 0,01 à 700 mg. Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, l'âge, le poids et la réponse du patient, ainsi que selon le degré de progression de la maladie.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

Les exemples suivants décrivent la préparation de certains composés conformes à l'invention. Ces exemples ne sont pas limitatifs et ne font qu'illustrer la présente invention.

Les produits et intermédiaires, lorsque leur préparation n'est pas explicitée, sont connus dans la littérature ou commerciaux. Certains intermédiaires utiles pour la préparation des composés de formule I peuvent également servir en tant que produits finaux de formule I, ainsi qu'il apparaîtra dans les exemples donnés ci-après. De façon similaire, certains composés de formule I de l'invention peuvent servir en tant qu'intermédiaires utiles pour la préparation d'autres composés de formule I selon l'invention.

Dans ce qui suit :
- BOC : *tert*-butyloxycarbonyle.
- BOP: benzotriazol-1-yloxy-tris(diméthylamino)-phosphonium hexafluorophosphate.
- DMSO : diméthylsulfoxyde.
- Les RMN ont été mesurées sur des appareils de BRUKER Avance 250MHz, 300MHz et 400MHz.
- Les points de fusion ont été mesurés sur un appareil BUCHI type B-540.
- M.S. : Spectrométrie de Masse, mesurée sur un appareil AGILENT MSD1.

### PREPARATIONS D'INTERMEDIAIRES DE SYNTHESE

### Préparation I

### Synthèse d'imidazo[1,5-a]pyridine-6-carboxylate de tert-butyle

A 570mg (3,52 mmoles) d'acide imidazo[1,5-a]pyridine-6-carboxylique [décrit dans Bioorg. Med. Chem. Lett. ; (2002), 12(3), 465-470] dans un mélange de 5ml de diméthylformamide et de 5ml de toluène, on ajoute 3.37ml (14,06 mmoles) de N,N-diméthylformamide di tert-butylacétate et chauffe à 90°C pendant 6 heures. On ajoute à nouveau au milieu réactionnel 3,37ml (14,06 mmoles) de N,N-diméthylformamide di *tert-*butylacétate et chauffe à 90°C 4 heures de plus. Le milieu réactionnel est versé sur de l'eau et extrait à l'acétate d'éthyle. La phase organique est décantée, lavée par une solution aqueuse saturée de chlorure de sodium, séchée sur sulfate de sodium et concentrée sous pression réduite. Le produit est purifié par filtration sur lit de silice en éluant avec un mélange de dichlorométhane et de méthanol (98-2). Après évaporation, on obtient 580mg d'une poudre beige. Point de fusion : 77°C; RMN-¹H (DMSO-*d6*) : 1,59(9H,s); 7,71(1H,d); 7,36(1H,s); 7,58(1H,d); 8,56(1H,s); 9,03(1H,s)

### Préparation II

### Synthèse de 7-(benzyloxy)imidazo[1,5-a]pyridine

### Etape A

### 4-(benzyloxy)-2-(chlorométhyl)pyridine

A 2g (9.29 mmoles) de [4-(benzyloxy)pyridin-2-yl]méthanol [décrit dans J. Org. Chem. ; (1996), 61(8), 2624] dans 46ml de dichlorométhane, on ajoute 1,76ml (24,16 mmoles) de chlorure de thionyle. Le milieu réactionnel est agité à température ambiante pendant 18 heures puis concentré sous pression réduite. Le résidu obtenu est repris par une solution aqueuse saturée de carbonate de sodium puis extraite au dichlorométhane. La phase organique est séchée sur sulfate de sodium puis concentrée sous pression réduite. On recueille 2,1g d'une huile marron. Spectrométrie de Masse (ModeES+) : MH+=234

### Etape B

### 1-[4-(benzyloxy)pyridin-2-yl]méthanamine

A 2,1g (8,99 mmoles) de 4-(benzyloxy)-2-(chlorométhyl)pyridine (décrit à l'étape A) dans 60ml de dichlométhane, on ajoute 1,5g (10,78 mmoles) d'hexaméthylène tétramine puis 1,3g d'iodure de sodium. Le milieu réactionnel est chauffé au reflux pendant 12 heures puis concentré sous pression réduite. Le résidu obtenu est repris par 45ml de méthanol. On ajoute 7,5ml (89,90 mmoles) d'une solution d'acide chlorhydrique 12N. Le milieu réactionnel est chauffé à reflux pendant 16 heures. Après ajout d'éther éthylique, le précipité obtenu est filtré puis repris par une solution aqueuse saturée de carbonate de sodium. La phase aqueuse est extraite à l'acétate d'éthyle, séchée sur sulfate de sodium puis concentrée sous pression réduite. On recueille 1,1g d'une huile beige. Spectrométrie de Masse (ModeES+) : MH+=215

### Etape C

0.7g (3,27 mmoles) de 1-[4-(benzyloxy)pyridin-2-yl]méthanamine (décrit à l'étape B) dans 16ml d'acide formique sont chauffés à reflux pendant 5 heures. Le milieu réactionnel est concentré sous pression réduite. Le résidu obtenu est repris dans 7ml de 1,2 dichloroéthane. On ajoute 0.6ml (6.54 mmoles) de chlorure de phosphoryle dissous dans 7ml de 1,2-dichloroéthane. Après 4 heures de chauffage au reflux, le milieu réactionnel est concentré sous pression réduite puis repris dans du dichlorométhane. La phase organique est lavée par une solution aqueuse saturée en hydrogénocarbonate de sodium, séchée sur sulfate de sodium et concentrée sous pression réduite. On recueille 0.97g d'une huile marron.

Spectrométrie de Masse (ModeES+) : MH+=225 ; RMN-¹H (DMSO-*d6*) : 5,092(2H, s); 6,44-6,47(1H, m); 7,07(1H, m); 7,08(1H, s); 7,37-7,49(5H, m); 8,17(1H, s); 8,23-8,27(1H, m)

### Préparation III

### Synthèse de 8-(benzyloxy)imidazo[1,5-a]pyridine

Ce composé est préparé selon le même mode opératoire que dans la préparation II (étape C) à partir de 2.8g (13.25 mmoles) de 1-[3-(benzyloxy)pyridin-2-yl]méthanamine [décrit dans Inorg. Chem. ; (2003), 42(14), 4401] par formylation avec l'acide formique puis cyclisation par réaction du chlorure de phosphoryle. On obtient 1,74g d'une huile marron. Spectrométrie de Masse (ModeES+) : MH+=225 ; RMN-¹H (DMSO-*d6*) : 5.26(2H,s); 6.21-6.28(1H,m); 6.55-6.60(1H,m); 7.28-7.52(6H,m); 7.96-7.99(1H,m); 8.35(1H,s)

### EXEMPLES

### Exemple 1

### (Imidazo[1,5-a]pyridin-3-yl)(3-méthoxy-4-nitrophényl)méthanone

A 3g (0,025 mole) d'imidazo[1,5-a]pyridine [décrit dans J. Chem. Soc. ; (1955), 2834-2836] dissous dans 100ml de 1,2-dichloroéthane, on ajoute 11,5g (0,053 mole) de chlorure de 3-méthoxy-4-nitrobenzoyle et 7,8ml (0,056 mole) de triéthylamine. On agite à température ambiante pendant 2 heures. Le milieu réactionnel est concentré sous pression réduite puis repris dans du dichlorométhane et une solution aqueuse saturée de bicarbonate de sodium. La phase organique est décantée, lavée par une solution aqueuse saturée de chlorure de sodium, séchée sur sulfate de sodium et concentrée sous pression réduite. Le résidu est repris au dichlorométhane et purifié par filtration sur un lit de gel de silice. Après évaporation, on recueille 7,1g d'un solide jaune. Point de fusion : 183°C ; RMN-¹H (DMSO-*d6) :* 4,01(3H, s); 7,35-7,40((1H, m); 7,47-7,54(1H, m); 7,97(1H, s); 8,06-8,11(3H, m); 8,15(1H, s); 9,77(1H, d).

### Exemple 2 à 4

En procédant selon la procédure décrite à l'exemple 1, on synthétise les composés de formule Ia décrits dans le tableau I ci-dessous par acylation des imidazo[1,5-a]pyridines convenablement substituées (décrites dans les demandes de brevet internationales WO 04/046133 et WO 03/070732), avec le chlorure de 3-méthoxy-4-nitrobenzoyle.

**TABLEAU I**

| Ex. | R | R₁ | R₂ | R₃ | Point de fusion (°C) |
|---|---|---|---|---|---|
| 2 | 8-CO₂Et | H | OMe | NO₂ | 210 |
| 3 | 7- CO₂Et | H | OMe | NO₂ | 196 |
| 4 | 6-CO₂Me | H | OMe | NO₂ | 218 |
| 5 | 8-Me | H | OMe | NO₂ | 130 |
| 6 | 7-OBn | H | OMe | NO₂ | 220 |
| 7 | 8-OBn | H | OMe | NO₂ | 211 |
| 8 | 7-Me | H | OMe | NO₂ | 176 |

Les RMN des exemples 2 à 8 du TABLEAU I sont présentées dans le TABLEAU I' ci-dessous :

**TABLEAU I'**

| Ex. | RMN-¹H (DMSO-*d6*) |
|---|---|
| 2 | 1,43(3H, t);3,99(3H, s); 4,47(2H, q); 7,46(1H, t); 7,66-8,27(5H, m); 9,93(1H, d) |
| 3 | 1,40(3H, t); 4,02(3H, s); 4,40(2H, q); 7,68(1H, d); 8,05(2H, s); 8,15 -8,20(2H, m); 8,0(1H,s); 9,72(1H, d) |
| 4 | 3,98(3H, s); 4,03(3H, s); 7,79(1H, d); 8,04(1H, s); 8,08-8,17(4H,m);10,31(1H, s) |
| 5 | 4,01(3H, s); 7,23-7,30(2H, m); 7,99(1H, s); 8,04-8,04(1H, m); 8,04-9,62(2H, m); 8,14(1H, s) |
| 6 | 4,01(3H, s); 5,28(2H, s); 7,10-7,44(2H, m); 7,30-7,45(4H, m); 7,60-9,70(2H, m); 7,74(1H, s); 8,03(1H, m); 8,12(1H, s) |
| 7 | 4,02(3H, s); 5,42(2H, s); 7,03-7,59(2H, m); 7,24-7,30(1H,t); 7,38-7,58(4H, m); 7,97(1H, s); 8,06(2H, m); 8,10(1H, s); 9,35-9,38(1H, m) |
| 8 | 2,47(3H, s); 4,02(3H, s); 7,21-9,68(2H, m); 7,83-7,84(2H, m); 8,04-8,05(2h, m); 8,14(1H, s) |

### Exemple 9

### 5-[(imidazo[1,5-a]pyridin-3-yl)carbonyl]-2-nitro-benzoate de méthyle

A 1,3g (0,011 mole) d'imidazo[1,5-a]pyridine [décrit dans J. Chem. Soc. ; (1955), 2834-2836] dissous dans 100ml de 1,2-dichloroéthane, on ajoute 5,6g (0,023 mole) de 5-(chlorocarbonyl)-2-nitrobenzoate de méthyle et 3,4ml (0,024 mole) de triéthylamine. On agite à température ambiante pendant 4 heures. Le milieu réactionnel est concentré sous pression réduite puis repris dans du dichlorométhane et une solution aqueuse saturée de bicarbonate de sodium. La phase organique est décantée, lavée par une solution aqueuse saturée de chlorure de sodium, séchée sur sulfate de sodium et concentrée sous pression réduite. Le produit est purifié par chromatographie colonne sur gel de silice en éluant au dichlorométhane. Après évaporation, on recueille 3,1g d'un solide jaune. Point de fusion : 151°C ; RMN-¹H (DMSO-*d6*) : 3,92(3H, s); 7,39-7,42(1H, m); 7,50-7,54(1H, m); 8,00(1H, s); 8,10(1H, d); 8,25(1H, d); 8,69(1H, d); 8,76(1H, s); 9,78(1H, d)

### Exemple 10

### 3-[3-(méthoxycarbonyl)-4-nitrobenzoyl]imidazo[1,5-a]pyridine-6-carboxylate de tert-butyle

Ce composé est obtenu selon le même procédé que celui décrit dans l'exemple 9 par benzoylation de l'imidazo[1,5-a]pyridine-6-carboxylate de *tert*-butyle par le 5-(chlorocarbonyl)-2-nitrobenzoate de méthyle en présence de triéthylamine. On obtient un solide jaune. Point de fusion : 170°C ; RMN-¹H (DMSO-*d6*) : 1,63(9H,s); 3,89(3H,s); 7,76(1H,d); 8,05(1H,s); 8,12(1H,d); 8,26(1H,d); 8,70(1H,d); 8,77(1H,s); 10,25(1H,s)

### Exemple 11

### Acide 3-[3-(méthoxycarbonyl)-4-nitrobenzoyl]imidazo[1,5-a]pyridine-6-carboxylique

A 610mg (1.43 mmole) de 3-[3-(méthoxycarbonyl)-4-nitrobenzoyl]imidazo[1,5-a]pyridine-6-carboxylate de *tert*-butyle dans 2ml de dichlorométhane on ajoute 2.13 ml (28.68 mmoles) d'acide trifluoroacétique et agite à température ambiante pendant 5 heures. Le milieu réactionnel est concentré sous pression réduite, puis le résidu obtenu est repris à l'acétone. Le précipité formé est filtré, lavé à l'acétone et séché. On obtient 450mg d'une poudre jaune. Point de fusion : 290°C ; RMN-¹H (DMSO-*d6*) : 3,90(3H,s); 7,78(1H,d); 8,04(1H,s); 8,12(1H,d); 8,26(1H,d); 8,68 (1H,d); 8,75(1H,s); 10,26(1H,s)

### Exemple 12

### 5-({6-[(tert-butoxycarbonyl)amino]imidazo[1,5-a]pyridin-3-yl}carbonyl)-2-nitrobenzoate de méthyle

A 430mg (1.16 mmole) d'acide 3-[3-(méthoxycarbonyl)-4-nitrobenzoyl]imidazo[1,5-a]pyridine-6-carboxylique dans 20ml de toluène, on ajoute 0.55ml (3.96 mmoles) de triéthylamine et 1.07ml de tert.butanol puis 0.31ml (1.40 mmole) de diphénylphosphoryl azide. Le milieu réactionnel est chauffé à 110°C pendant 3 heures puis refroidi à température ambiante. On ajoute de l'eau et une solution aqueuse saturée en bicarbonate de sodium puis extrait à l'acétate d'éthyle. La phase organique est décantée, lavée avec une solution aqueuse saturée en chlorure de sodium puis séchée sur sulfate de sodium et concentrée sous pression réduite. Le produit est purifié par chromatographie colonne sur gel de silice en éluant avec un mélange de dichlorométhane et de méthanol (98-2). On obtient 480mg d'un solide orangé. Point de fusion : 182°C ; RMN-¹H (DMSO-*d6*) : 1,55(9H,s); 3,92(3H,s); 7,45(1H,d); 7,90(1H,s); 7,99(1H,d); 8,24(1H,d); 8,67(1H,d); 8,76(1H,s); 9,89(1H,s)

### Exemple 13

### [6-(tert-butoxycarbonylamino)imidazo[1,5-a]pyridin-3-yl](3-méthoxy-4-nitrophényl) méthanone

Ce composé est obtenu selon le même procédé que celui décrit dans l'exemple 12 précédent par réarrangement de Curtius à partir de l'acide 3-(3-méthoxy-4-nitrobenzoyl)imidazo[1,5-a] pyridine-6-carboxylique avec le diphénylphosphoryl azide en présence de *tert*.butanol. On obtient un solide jaune. Point de fusion : 200°C ; RMN-¹H *(DMSO-d6)* : 1,54(9H,s); 4,02(3H,s); 7,42(1H,d); 7,87(1H,s); 8,11-7,87(3H,m); 8,15(1H,s); 9,87(1H,s)

### Exemple 14

### 5-({6-[(tert-butoxycarbonyl)(méthyl)amino]imidazo[1,5-a]pyridin-3-yl}carbonyl)-2-nitrobenzoate de méthyle

A 53.9mg (1.35 mmole) d'hydrure de sodium (à 60% en dispersion dans l'huile) dans 2ml de diméthylformamide on ajoute 540mg (1.23 mmole) de 5-({6-[(ter-butoxycarbonyl)amino]imidazo[1,5-a]pyridin-3-yl}carbonyl)-2-nitrobenzoate de méthyle dans 10ml de diméthylformamide et agite 30 minutes à température ambiante, puis ajoute 84µl (1.35 mmole) d'iodure de méthyle et laisse agiter à température ambiante une nuit. On acidifie à pH = 4 avec une solution aqueuse d'hydrogénosulfate de potassium et extrait à l'acétate d'éthyle. La phase organique est lavée avec une solution aqueuse saturée en chlorure de sodium puis séchée sur sulfate de sodium et concentrée sous pression réduite. Le produit est purifié par filtration sur lit de silice en éluant au dichlorométhane. On obtient 475mg d'une poudre orangée. Point de fusion : 55°C ; RMN-¹H (DMSO-*d6*) : 1,46(9H,s); 3,33(3H,s); 3,92(3H,s); 7,57(1H,d); 7,98(1H,s); 8,06 (1H,d); 8,25(1H,d); 8,68(1H,d); 8,76(1H,s); 9,77(1H,s)

### Exemple 15

### 5-([6-(méthylamino)imidazo[1,5-a]pyridin-3-yl]carbonyl)-2-nitrobenzoate de méthyle

A 460mg (1.01 mmole) de 5-({6-[(*tert*-butoxycarbonyl)(méthyl)amino]imidazo[1,5-a]pyridin-3-yl}carbonyl)-2-nitrobenzoate de méthyle dans 5ml de dichlorométhane on ajoute 1.3ml d'acide trifluoroacétique et agite à température ambiante une nuit. Le milieu réactionnel est concentré sous pression réduite. Le résidu est repris à l'eau et basifié avec une solution aqueuse saturée en bicarbonate de sodium puis extrait au dichlorométhane. La phase organique est lavée avec une solution aqueuse saturée en chlorure de sodium puis séchée sur sulfate de sodium et concentrée sous pression réduite. On obtient 350mg d'une poudre rouge. Point de fusion : 183°C ; RMN-¹H (DMSO-*d6*) : 2,79(3H,d); 3,90(3H,s); 7,11(1H,d); 7,77(1H,s); 7,82(1H,d); 8,31(1H,d); 8,66(1H,d); 8,75(1H,s); 9,01(1H,s)

### Exemple 16

### (6-aminoimidazo[1,5-a]pyridin-3-yl)(3-méthoxy-4-nitrophényl)méthanone

Ce composé est obtenu selon le même procédé que celui décrit dans l'exemple 15 précédent par déprotection de l'amine du composé [6*-(tert-*butoxycarbonylamino)imidazo[1,5-a]pyridin-3-yl](3-méthoxy-4-nitrophényl) méthanone avec l'acide trifluoroacétique. On obtient un solide jaune qui est salifié sous forme de chlorhydrate. Point de fusion : 252°C ; RMN-¹H (DMSO-*d6*) : 4,01(3H,s); 7,14(1H,d); 7,79(1H,s); 7,87(1H,d) ; 7,97-8,01(2H,m); 8,13(1H,s); 9,41(1H,s)

### Exemple 17

### N-[3-(3-méthoxy-4-nitrobenzoyl)imidazo[1,5-a]pyridin-6-yl]méthane sulfonamide

A 0.40g (1.15 mmole) de chlorhydrate de (6-aminoimidazo[1,5-a]pyridin-3-yl)(3-méthoxy-4-nitrophényl)méthanone dans 10ml de pyridine refroidi à 5°C, on ajoute 0.107ml (1.38 mmole) de chlorure de mésyle puis laisse revenir à température ambiante et agite 18 heures. Le milieu est repris par 130ml d'acide chlorhydrique 1N et extrait à l'acétate d'éthyle. La phase organique est lavée avec une solution aqueuse saturée en chlorure de sodium puis séchée sur sulfate de sodium et concentrée sous pression réduite. Le résidu est repris à l'éther isopropylique, filtré, lavé à l'éther isopropylique puis séché. On obtient 411mg d'un solide jaune. Point de fusion : 249°C ; RMN-¹H (DMSO-*d6*) : 3,14(3H,s); 4,02(3H,s); 7,39(1H,d); 7,94(1H,s); 8,02-8,11(3H,m); 8,13(1H,s); 9,88(1H,s)

### Exemple 18

### N-[3-(3-méthoxy-4-nitrobenzoyl)imidazo[1,5-a]pyridin-6-yl]acétamide

A 0.50g (1.43 mmole) de chlorhydrate de (6-aminoimidazo[1,5-a]pyridin-3-yl)(3-méthoxy-4-nitrophényl)méthanone dans 25ml de 1.2-dichloroéthane refroidi à 5°C, on ajoute 1.29ml (9.29 mmoles) de triéthylamine puis 0.51ml (7.15 mmoles) de chlorure d'acétyle puis laisse revenir à température ambiante et agite 18 heures. Le milieu est repris par de l'eau, basifié avec une solution de bicarbonate de sodium et extrait au dichlorométhane. La phase organique est lavée avec une solution aqueuse saturée en chlorure de sodium puis séchée sur sulfate de sodium et concentrée sous pression réduite. Le produit est purifié par chromatographie colonne sur gel de silice en éluant avec un mélange de dichlorométhane et d'acétone(90-10). On obtient 316mg d'un solide jaune. Point de fusion : 257°C ; RMN-¹H (DMSO-*d6*) : 2,15(3H,s); 4,01(3H,s); 7,45(1H,d); 7,92(1H,s); 8,00-8,06(3H,m); 8,13(1H,s); 10,62(1H,s)

### Exemple 19

### 2-(benzoylamino)-5-[(imidazo[1,5-a]pyridine-3-yl)carbonyl]benzoate de méthyle

A 1,97g (0,017 mole) d'imidazo[1,5-a]pyridine [décrit dans J. Chem. Soc. ; (1955), 2834-2836] dans 100ml d'acétonitrile, on ajoute 5,2ml (0,037 mole) de triéthylamine puis sous atmosphère d'azote à 0°C 10g (0,035 mole) de chlorure de 4-oxo-2-phényl-4H-3,1-benzoxazine-6-carbonyle. Après 22 heures d'agitation à température ambiante, le milieu réactionnel est filtré. Le résidu obtenu est lavé à l'acétate d'éthyle, à l'eau, à l'acétone puis séché. A 9,75g (0,026 mole) du solide jaune obtenu précédemment, dans 50ml de méthanol et 50ml de *N,N*-diméthylformamide, on ajoute 0,32g (2,65 mmoles) de *N,N-*diméthylpyridine-4-amine. Après 22 heures de chauffage au reflux, le milieu réactionnel est filtré. Le résidu est lavé à l'eau puis séché. Le produit est purifié par chromatographie colonne sur gel de silice en éluant avec un mélange de dichlorométhane et de méthanol (99,9-0,1). On obtient 3,67g d'un solide jaune. Point de fusion : 218°C; RMN-¹H (CDCl3) : 4,05(3H, s); 7,08-7,09(1H, m); 7,27-7,29(1H, m); 7,57-7,60(3H, m); 7,76-7,81(2H, m); 8,12(2H, d); 8,78(1H,d); 9,15(1H,d); 9,28(1H,s); 9,88(1H,d)

### Exemple 20

### (1-bromoimidazoil,5-alpyridin-3-yl)(3-méthoxy-4-nitrophényl)méthanone

A 9,8g (0,033 mole) de (imidazo[1,5-a]pyridin-3-yl)(3-méthoxy-4-nitrophényl)méthanone obtenu à l'exemple 1, dans 170ml de chloroforme, on ajoute 3,51g (0,043 mole) d'acétate de sodium puis goutte à goutte une solution de 1,85ml (0,036 mole) de brome dans 15ml de chloroforme en maintenant le milieu à température ambiante. L'introduction terminée, on agite 1 heure de plus à la même température. Le milieu réactionnel est versé sur une solution aqueuse saturée de bicarbonate de sodium et extrait au dichlorométhane. La phase organique est décantée, lavée avec une solution aqueuse de chlorure de sodium, séchée sur sulfate de sodium et concentrée sous pression réduite. Le résidu est repris dans un mélange toluène-dichlorométhane puis purifié par filtration sur un lit de gel de silice en éluant avec du toluène. Après évaporation, on recueille 7,71 g d'un solide jaune. Point de fusion : 189°C ; RMN-¹H (CDCl3) : 4,10(3H, s) ; 7,23-7,29(1H, m); 7,41-7,46(1H, m); 7,78(1H, d); 7,96 (1H, d) ; 8,14-8,20(2H, m) ; 9,90(1H, d)

### Exemples 21 à 24

En procédant comme la préparation décrite à l'exemple 20, on synthétise les composés de formule Ii décrits dans le tableau II ci dessous par bromation des composés de formule I (avec R1 = H) en présence de brome et d'acétate de sodium.

**TABLEAU II**

| Ex. | R | R₁ | R₂ | R₃ | Point de Fusion (°C) |
|---|---|---|---|---|---|
| 21 | H | Br | CO₂Me | NO₂ | 172 |
| 22 | H | Br | CO₂Me | NHCO-Ph | 209 |
| 23 | 7-CONHMe | Br | OMe | NO₂ | 281 |
| 24 | 7-CO₂Et | Br | OMe | NO₂ | 204 |

Les RMN des exemples 21 à 24 du TABLEAU II sont présentées dans le TABLEAU II' ci-dessous :

**TABLEAU II'**

| Ex. | RMN-¹H |
|---|---|
| 21 | (DMSO-*d6*): 3,92(3H,s); 7,48-7,67(2 H, m); 7,92(1H, d); 8,27(1H, d); 8,62(2H,m); 9.75(1H, d) |
| 22 | (CDCl3) : 4,08(3H, s); 7,13-7,16(1H, m); 7,30-7,31(1H, m); 7,58-7,62(3H, m); 7,76(1H, d); 8,13(2H, d); 8,82(1H, d); 9,15(1H, d); 9,28(1H, s); 9,89(1H, d) |
| 23 | (DMSO-*d6*) : 2,86(3H, d); 4,01(3H, s); 7,76-7,96(2H, m); 7,96-9,72(2H,m); 8,03-8,36(1H, m); 8,36(1H, s); 8,96(1H, m) |
| 24 | (CDCl3) : 1,43-1,71(3H, t); 4,11(3H, s); 4,47-4,57(2H, q); 7,71-7,98(2H, m); 8,18-9,85(2H, m); 8,22(1H, s); 8,47(1H, s) |

### Exemple 25

### (3-méthoxy-4-nitrophényl)[1-(4-méthoxyphényl)imidazo[1,5-a]pyridin-3-yl]méthanone

A 0,850g (0,023 mole) de (1-bromoimidazo[1,5-a]pyridin-3-yl)(3-methoxy-4-nitrophenyl)méthanone obtenu à l'exemple 20, dans 30 ml de dioxanne, sous atmosphère d'argon, on ajoute 0,447g (0,003 mole) d'acide 4-méthoxyphénylboronique, 2,24g (0,009 mole) de K₃PO₄.H₂O puis 0,131g (0,011 mole) de tétrakis(triphénylphosphine)palladium(0). On chauffe à reflux pendant 1 heure. Le milieu réactionnel est versé sur de l'eau et extrait à l'acétate d'éthyle. La phase organique est décantée, lavée avec une solution aqueuse saturée de chlorure de sodium, séchée sur sulfate de sodium et concentrée sous pression réduite. Le produit est purifié par filtration sur gel de silice en éluant avec un mélange dichlorométhane-cyclohexane(2-1) puis au dichlorométhane. Après évaporation, on recueille 0,850g d'un solide orange. Point de fusion : 185°C ; RMN-¹H (DMSO-*d6*) : 3,85(3H, s); 4,06(3H, s); 7,12(2H, d); 7,41-7,44(1H,m); 7,54-7,58 (1H,m); 7,95(2H, d); 8,08-8,10(2H, m); 8,34(1H, d); 8,36(1H, s); 9,81(1H, d)

### Exemple 26 à 58

En procédant selon la préparation décrite à l'exemple 25, on synthétise les composés de formules Is, décrits dans le tableau III ci dessous, par couplage de type Suzuki des composés bromés de formule générale Ii, avec des dérivés phénylboroniques ou hétéroarylboroniques en faisant varier les conditions expérimentales (catalyseurs, ligands, bases) selon les composés à obtenir.

**TABLEAU III**

| Ex. | R | R₁ | R₂ | R₃ | Catalyseur/Ligand / Base | PF (°C) ou M.S. |
|---|---|---|---|---|---|---|
| 26 | H | 2-thiényl | OMe | NO₂ | Pd(tBu₃)₂/Pd₂dba₃ Na₂CO₃ | 206 |
| 27 | H | 4-pyridinyl | OMe | NO₂ | PdCl₂dppf Na₂CO₃ | 230 |
| 28 | H | 3-furyl | OMe | NO₂ | Pd(PPh₃)₄ Na₂CO₃ | 225 |
| 29* | H | 3-pyridinyl | OMe | NO₂ | PdCl₂dppf Na₂CO₃ | 238 |
| 30 | H | 2-(5-carboxythiényl) | OMe | NO₂ | Pd(tBu₃)₂/Pd₂dba₃ Na₂CO₃ | 232 |
| 31 | H | phényl | OMe | NO₂ | Pd(PPh₃)₄ Na₂CO₃ | 167 |
| 32 | H | 4-fluorophényl | OMe | NO₂ | Pd(PPh₃)₄ Na₂CO₃ | 240 |
| 33 | H | 2-méthoxyphényl | OMe | NO₂ | Pd(PPh₃)₄ Na₂CO₃ | 186 |
| 34 | H | 3-méthoxyphényl | OMe | NO₂ | Pd(PPh₃)₄ Na₂CO₃ | 132 |
| 35 | H | 3-carboxyphényl | OMe | NO₂ | Pd(PPh₃)₄ Na₂CO₃ | 303 |
| 36 | H | 4-chlorophényl | OMe | NO₂ | Pd(PPh₃)₄ Na₂CO₃ | 233 |
| 37 | H | 3-chlorophényl | OMe | NO₂ | Pd(PPh₃)₄ Na₂CO₃ | 233 |
| 38 | H | N-BOC-2-pyrrolyl | OMe | NO₂ | Pd(PPh₃)₄ Na₂CO₃ | MH+= 463 |
| 39 | H | 3-thiényl | OMe | NO₂ | Pd(PPh₃)₄ Na₂CO₃ | 194 |
| 40 | H | 4-méthoxyphényl | CO₂Me | NHCOP h | Pd(PPh₃)₄ K₃PO₄.H₂O | 243 |
| 41 | H | 2-méthoxyphényl | CO₂Me | NO₂ | Pd(PPh₃)₄ K₃PO₄.H₂O | 145 |
| 42 | H | 3-thiényl | CO₂Me | NO₂ | Pd(tBu₃)₂/Pd₂dba₃ K₃PO₄.H₂O | 172 |
| 43 | H | 3-méthoxyphényl | CO₂Me | NO₂ | Pd(PPh₃)₄ K₃PO₄.H₂O | 98 |
| 44 | H | 2-thiényl | CO₂Me | NO₂ | Pd(tBu₃)₂/Pd₂dba₃ K₃PO₄.H₂O | 135 |
| 45* | H | 3-pyridinyl | CO₂Me | NO₂ | PdCl₂dppf K₃PO₄.H₂O | 195 |
| 46 | H | 4-méthoxypyridin-3-yl | OMe | NO₂ | PdCl₂dppf K₃PO₄.H₂O | 246 |
| 47 | H | 3-furyl | CO₂Me | NO₂ | Pd(PPh₃)₄ K₃PO₄.H₂O | 151 |
| 48 | 7-CONHMe | 3-carboxyphényl | OMe | NO₂ | Pd(PPh₃)₄ Na₂CO₃ | 328 |
| 49 | 7-CO₂Et | 3-méthoxyphényl | OMe | NO₂ | Pd(PPh₃)₄ K₃PO₄.H₂O | 212 |
| 50 | 7-CO₂Et | 4-méthoxypyridin-3-yl | OMe | NO₂ | PdCl₂dppf K₃PO₄.H₂O | 208 |
| 51 | H | 4-carboxyphényl | OMe | NO₂ | Pd(PPh₃)₄ Na₂CO₃ | 356 |
| 52 | H | 4-fluorophényl | CO₂Me | NO₂ | Pd(PPh₃)₄ K₃PO₄.H₂ | 180 |
| 53 | H | 4-méthoxypyridin-3-yl | CO₂Me | NO₂ | PdCl₂dppf K₃PO₄.H₂O | 192 |
| 54 | H | 3-fluoro-4-méthoxyphényl | OMe | NO₂ | Pd(PPh₃)₄Na₂CO₃ | 211 |
| 55 | H | 3-fluorophényl | OMe | NO₂ | Pd(PPh₃)₄ Na₂CO₃ | 214 |
| 56 | H | 4-chlorophényl | CO₂Me | NO₂ | Pd(PPh₃)₄ K₃PO₄.H₂O | MH+= 436 |
| 57 | H | 3-carboxyphényl | OMe | NHSO₂ Me | Pd(PPh₃)₄ Na₂CO₃ | 145 |
| 58 | H | -Ph-3-CO₂Me | OMe | NO₂ | Pd(PPh₃)₄ K₃PO₄.H₂O | 227 |

| | | | | | | |
|---|---|---|---|---|---|---|
| *on utilise les dérivés pinacol boronates au lieu des acides boroniques correspondants. BOC = tert-butoxycarbonyle | | | | | | |

Les RMN des exemples 26 à 58 du TABLEAU III sont présentées dans le TABLEAU III' ci-dessous :

**TABLEAU III'**

| EX | RMN-¹H (DMSO-d6) ou M.S. |
|---|---|
| 26 | 4,02(3H, s); 7,24(1H, t); 7,47-7,65(4H, m); 7,80-7,82(1H, m); 7,82-7,99(2H, m); 8,52(1H, d); 8,55(1H, s); 9,76(1H, d) |
| 27 | 4,06(3H, s); 7,45-7,71(2H, m); 8,02(2H, m); 8,04(2H, m); 8,30(1H, s); 8,55(1H, d); 8,69(2H, m); 9,84(1H, d) |
| 28 | MH+ = 364 |
| 29 | 4,12(3H, s); 7,42-7,51(3H, m); 7,97(1H, d); 8,15(1H, d); 8,29(2H, m); 8,39(1H, s); 8,68(1H, d); 9,25(1H, s); 10,00(1H, d) |
| 30 | 4,08(3H, s); 7,47-7,69(2H, m); 7,76-7,83(2H, m); 8,03-8,13(2H, m); 8,39(1H, s); 8,47(1H, d); 9,84(1H, d) |
| 31 | 3,92(3H, s); 7,32-7,80(9H, m); 8,34(1H, s); 8,40(1H, d); 9,85(1H, d) |
| 32 | 4,03(3H, s); 7,35-7,62(4H, m); 8,03-8,81(4H, m); 8,30(1H, s); 8,38 (1H, d); 9,84(1H, d) |
| 33 | 3,85(3H, s); 4,04(3H, s); 7,11-7,51(5H, m); 7,64(1H, d); 7,88(1H, d) 8,05-8,30(2H, m) ; 8,30(1H, s) ; 9,81(1H, d) |
| 34 | 3,86(3H, s); 4,05(3H, s); 7,02(1H, m); 7,42-7,60(5H, m); 8,08(2H, m); 8,35-8,39(2H, m); 9,84(1H, d) |
| 35 | 4,08(3H, s) ; 7,34-8,69(10H, m) ; 9,79(1H, m) |
| 36 | 4,03(3H, s); 7,42-7,65(4H, m); 8,04-8,08(4H, m); 8,31(1H, s); 8,40 (1H, d); 9,84(1H, d) |
| 37 | 4,07(3H, s); 7,45-7,64(4H, m); 8,0-8,03(4H, m); 8,35(1H, s); 8,43 (1H, d); 9,84(1H, d) |
| 38 | 1,18(9H, s); 4,02(3H, s); 6,43(1H, m); 6,63(1H, m); 7,43-7,56(3H, m); 7,89(1H, d); 8,03(1H, d); 8,12-8,15(2H, m); 9,83(1H, d) |
| 39 | 4,09(3H, s); 7,23-7,26(1H, m); 7,45-7,65(3H, m); 7,81(1H, d); 7,99-8,11(2H, m); 8,44(1H, m); 8,52(1H, s); 9,84(1H, d) |
| 40 | (CDCl3) : 3,94(3H, s); 4,09(3H, s); 7,09-7,13(3H, m); 7,30-7,36(1H, m); 7,59-7,62(3H, m); 7,98(2H, d); 8,09-8,16(3H, m); 8,99(1H, d); 9,16(1H, d); 9,61(1H,s); 9,98(1H, d) |
| 41 | 3,86(3H, s); 3,91(3H, s); 7,12-7,63(5H, m); 7,63(1H, d); 7,92(1H, d); 8,24(1H, d) ; 8,74-8,80(2H, m) ; 9.81(1H, d) |
| 42 | 3,93(3H, s); 7,43-7,77(2H, m); 7,78-7,79(2H, m); 8,18(1H, s); 8,24(1H, d); 8,45(1H, d), 8,78(1H, d); 8,90(1H, s); 9,85(1H, d) |
| 43 | 3,95(3H, s); 4,00(3H, s); 7,02(1H, d); 7,22(1H, t); 7,45-7,56(4, m); 8,01(1H, d); 8,18(1H, d); 8,06(1H, d); 9,98(1H, d) |
| 44 | 4,02(3H, s); 7,13-7,44(3H, m); 7,63(1H, d); 8,03(1H, d); 8,17(1H, d); 8,78(1H, m); 8,90(1H, d); 9,05(1H, s); 9,96(1H, d) |
| 45 | 3,92(3H, s); 7,44-7,60(3H, m); 8,25(1H, d); 8,44(1H, d); 8,45(1H, d); 8,61(1H, d); 8,81(1H, d); 8,85(1H, s); 9,24(1H, s); 9,83(1H, d) |
| 46 | 3,93(3H, s); 4,04(3H, s); 7,00(1H, d); 7,41-7,61(2H, m); 8,05-8,13 (2H, m); 8,29-8,41(3H, m); 8,82(1H, s); 9,84(1H, d) |
| 47 | 3,93(3H, s); 7,11(1H, d); 7,44-7,62(2H, m); 7,89(1H, d); 8,25(1H, d); 8,35(1H, d); 8,51(1H, s); 8,77(1H, d); 8,89(1H, s); 9,85(1H, d) |
| 48 | 2,85-2,89(3H,d); 4,09(3H, s); 7,70-8,78(2H, m); 7,73-7,78(2H, m); 7,96-8,1(1H, m); 8,06-9,54(2H, m); 8,32(1H, s); 8,34(1H, m), 8,35(1H, s); 8,95(1H, m), 13,2(1H, m) |
| 49 | 1,35-1,40(3H, t); 3,86(3H, s); 4,05(3H, s); 4,37-4,45(2H, q); 7,04-7,54(2H, m); 7,46-7,54(2H, m); 7,68-9,77(2H, m); 8,08-8,09(2H, m); 8,35(1H, s); 8,65(1H, s) |
| 50 | 1,37-1,43(3H, t); 3,97(3H, s); 4,05(3H, s); 7,15-7,75(2H, m); 7,97(1H, m); 8,10(1H, m); 8,11-9,80(2H, m); 8,30(1H, m); 8,30-8,75(3H, m)8,70(1H, m) |
| 51 | 4,07(3H, s); 7,40-7,63(2H, m); 7,57-8,14(6H, m); 8,38(1H, m); 8,40-9,88(2H, m) |
| 52 | 3,94(3H, s); 7,36-7,45(3H, m); 7,62-8,09(2H, m); 8,24-8,40(2H, m); 8,75-9,84(2H, m); 8,85(1H, m) |
| 53 | 3,92(3H, s); 3,94(3H, s); 6,98-8,25(2H, m); 7,39-7,61(2H, m); 8,28-8,83(2H, m); 8,37-9,83(2H, m); 8,80(1H, m) |
| 54 | 3,92(3H, s); 4,05(3H, s); 7,32-7,41-(2H, m); 7,58-8,09(2H, m); 7,82-8,09(2H, m); 8,32(1H, m); 8,36-9,84(2H, m) |
| 55 | 4,05(3H, s); 7,24-7,45(2H, m); 7,57-7,62(2H, m); 7,86(1H, m); 7,89(1H, m); 8,08-8,09(2H, m); 8,30(1H, s); 8,41-9,84(2H, m) |
| 56 | 3, 92(3H, s); 7,38-7,61(2H, m); 7,57-8,06(4H,m ); 7,68-8, 36(2H, m); 8,53(1H, m); 9,83(1H, m) |
| 57 | 3,13(3H, s); 4,00(3H, s); 7,35-7,67(4H, m); 7,94-8,07(2H, m); 8,26-8,44(2H, m); 8,44(1H, s); 8,64(1H, s);9,3(1H, m); 9,81-9,84(1H, m); 13(1H, m) |
| 58 | (CDCl₃) : 4,01(3H, s); 4,16(3H, s); 7,20-7,66(2H, m); 7,29-7,49(1H, m); 7,98-8,22(2H, m); 8,01-8,18(2H, m); 8,21-10,00(2H, m); 8,55(1H, s); 8,65(1H, s) |

### Exemple 59

### 3-(3-méthoxy-4-nitrobenzoyl)imidazo[1,5-a]pyridine-6-carboxamide

A 0,346g (1,01 mmole) de l'acide 3-(3-méthoxy-4-nitrobenzoyl)imidazo[1,5-a]pyridine-6-carboxylique obtenu à l'exemple 182 dans 10ml de N,N-diméthylformamide, on ajoute 0,16ml (1,12mmole) de triéthylamine puis 0,49g (1,12 mmole) de BOP. Le milieu réactionnel est agité pendant 30 minutes à température ambiante puis on ajoute 1,35ml d'une solution d'ammoniac 1N dans le tétrahydrofurane et agite à température ambiante 18 heures. Le précipité formé est filtré puis lavé à l'eau. On recueille 0,25g d'un solide jaune. Point de fusion : 289°C ; RMN-¹H (DMSO-*d6*) : 4,02(3H, s); 7,82(1H, d); 7,98(1H, s); 8,06-8,10(3H, m); 8,15(1H,s); 10,21(1H,s).

### Exemples 60 à 69

En procédant selon la préparation décrite à l'exemple 59, on synthétise les composés de formule générale **Iu** décrits dans le tableau IV ci-dessous par couplage peptidique de l'acide 3-(3-méthoxy-4-nitrobenzoyl)imidazo[1,5-a]pyridine-6-carboxylique obtenu à l'exemple 182 ou de l'acide 3-(3-méthoxy-4-nitrobenzoyl)imidazo[1,5-a]pyridine-7-carboxylique obtenu à l'exemple 184, avec des amines ou des esters d'aminoacides en présence de BOP comme réactif de couplage.

**TABLEAU IV**

| Ex. | R | R₁ | R₂ | R₃ | Point de Fusion (°C) |
|---|---|---|---|---|---|
| 60 | 7-CONHCH₂CO₂Et | H | OMe | NO₂ | 196 |
| 61 | (R)-7-CONHCH(CH₃)CO₂Me | H | OMe | NO₂ | 208 |
| 62 | (S)-7-CONHCH(CH₃)CO₂Me | H | OMe | NO₂ | 207 |
| 63 | (S)-7-CONHCH(CH₂OH)CO₂CH₃ | H | OMe | NO₂ | 204 |
| 64 | 6-CONHCH₂CO₂Me | H | OMe | NO₂ | 221 |
| 65 | (S)-6-CONHCH(Bn)CO₂Me | H | OMe | NO₂ | 230 |
| 66 | 6-CONHCH₂CH₂CO₂Me | H | OMe | NO₂ | 190 |
| 67 | (S)-6-CONHCH(CH₂OH)CO₂CH | H | OMe | NO₂ | 202 |
| 68 | (S)-6-CONHCH(CH₃)CO₂Me | H | OMe | NO₂ | 225 |
| 69 | 6-CONHMe | H | OMe | NO₂ | 237 |

Les RMN des exemples 60 à 69 du TABLEAU IV sont présentées dans le tableau IV' ci-dessous :

**TABLEAU IV'**

| Ex. | RMN-¹H (DMSO-*d6*) |
|---|---|
| 60 | 1,21-1,25(3H, t); 4,02(3H, s); 4,07-4,09(2H, d); 4,12-4,19(2H, q); 7,67-7,70(1H, m); 8,05-8,16(4H, m); 8,56(1H, s); 9,30-9,34(1H, t); 9,72-9,74(1H, m) |
| 61 | 1,44-1,47(3H, d); 3,68(3H, s); 4,52-4,57(2H, m); 7,69-7,72(2H, m); 8,05-8,06(2H, m); 8,15-8,17(2H, m); 8,59-8,60(1H, m); 9,16-9,18(1H, d); 9,70-9,73(1H, m) |
| 62 | 1,44-1,47(3H, d); 3,68(3H, s); 4,02(3H, s); 4,50-4,59(1H, m); 7,69-9,73(2H, m); 8,05-8,17(2H, m); 8,06-8,16(2H, m); 8,59(1H, s); 9,16-9,18(1H, d) |
| 63 | 3,68(3H, s); 3,82-3,86(2H, m); 4,02(3H, s); 4,57-4,63(1H, m); 5,11-5,15(1H, t); 7,70-9,74(2H, m); 8,06(2H, m); 8,16(2H, m); 9,01-9,03(1H, d) |
| 64 | 3,71(3H,s); 4,03(3H,s); 4,12(2H,d); 7,82(1H,d); 8,02-8,17(5H,m);10,20(1H,s) |
| 65 | 3,69(3H,s); 4,03(3H,s); 4,74-4,79(1H,m); 7,22-7,39(5H,m); 7,74(1H,d); 8,0(1H,s); 8,07-8,17(4H,m); 10,15(1H,s) |
| 66 | 2,67(2H,t); 3,57(2H,m); 3,65(3H,s); 4,03(3H,s); 7,77(1H,d); 8,00(1H,s); 8,07-8,17(4H,m); 10,20(1H,s) |
| 67 | 3,69(3H,s); 3,84-3,89(2H,m); 4,02(3H,s); 4,60-4,65(1H,m); 7,86(1H,d); 8,01(1H,s); 8,05-8,16(4H,m); 10,25(1H,s) |
| 68 | 1,47(3H,d); 3,70(3H,s); 4,03(3H,s); 7,85(1H,d); 8,01(1H,s); 8,04-8,17(4H,m); 10,22(1H,s) |
| 69 | 2,86(3H,d); 4,02(3H,s); 7,83(1H,d); 7,98(1H,s); 8,04-8,11(2H,m); 8,15(1H,s); 10,18(1H,s) |

### Exemple 70

### 3-(3-méthoxy-4-nitrobenzoyl)-N,N-diméthylimidazo[1,5-a]pyridine-8-carboxamide

A 0,318g (0,88 mmole) de l'acide 3-(3-méthoxy-4-nitrobenzoyl)imidazo[1,5-a]pyridine-8-carboxylique obtenu à l'exemple 183, dans 10ml de dichlorométhane, on ajoute 0,17ml (2,36 mmoles) de chlorure de thionyle puis 30µl de N,N-diméthylformamide. On chauffe à reflux 2 heures. Le milieu réactionnel est concentré sous pression réduite. Le résidu obtenu est ajouté à 5ml d'une solution de diméthylamine 2N dans le tétrahydrofurane. Après agitation 18h à température ambiante, le milieu réactionnel est concentré sous pression réduite. Le résidu est repris dans le dichlorométhane. La phase organique est lavée avec une solution aqueuse 1N d'acide chlorhydrique puis une solution aqueuse saturée de chlorure de sodium, séchée sur sulfate de sodium et concentrée sous pression réduite. Le résidu est purifié par chromatographie colonne sur gel de silice en éluant avec du dichlorométhane puis un mélange de dichlorométhane-méthanol (99-1). On recueille 0,19g d'un solide jaune. Point de fusion : 176°C ; RMN-¹H (DMSO-*d6*) : 4,02(3H, s); 7,39(1H, t); 7,53(1H, d); 7,83(1H, s); 8,0-8,12(3H, m); 9.75(1H, d)

### Exemples 71 à 74

En procédant selon la préparation décrite à l'exemple 70, on synthétise les composés de formule générale Iu décrits dans le tableau V ci-dessous, par couplage de la fonction acide des composés de formule It avec l'amine correspondante.

**TABLEAU V**

| Ex. | R | R₁ | R₂ | R₃ | PF(°C) |
|---|---|---|---|---|---|
| 71 | 7-CONHMe | H | OMe | NO₂ | 255°C |
| 72 | 7-CONH₂ | H | OMe | NO₂ | 279°C |
| 73 | 7-CONMe₂ | H | OMe | NO₂ | 184°C |
| 74 | 8-CONHMe | H | OMe | NO₂ | 258 |

Les RMN des exemples 71 à 74 du tableau V sont présentées dans le tableau V'ci-dessous :

**TABLEAU V'**

| Ex. | RMN-¹H (DMSO-*d6*) |
|---|---|
| 71 | 2,35(3H, s); 2,85(3H, d); 4,02(3H, s); 7,70-8,06(2H, m); 7,74(2H, m); 7,85(1H, s); 8,12-9,73(2H, m); 8,55(1H, s); 8,93(1H, m) |
| 72 | 4,99(3H, s); 7,55-8,14(2H, m); 8,03(1H, s); ,8,04(1H, s); 8,14-9,70(2H, m); 8,37(1H, s); 8,58(1H, s) |
| 73 | 3,04(6H, m); 4,02(3H, s); 7,34-8,16(2H, m); 8,02(1H, s); 8,06(1H, s); 8,15-9,73(2H, m) |
| 74 | 2,87-2,89(3H, d); 4,02(3H, s); 7,39-7,45(1H, t); 7,82-9,85(2H, m); 8,05(2H, m); 8,08(1H, m); 8,22(1H, m); 8,79-8,81(1H, m) |

### Exemple 75

### 3-(3-méthoxy4-nitrobenzoyl) imidazo[1,5-a]pyridine-1-carbonitrile

A 6,94g (18,45 mmoles) de (1-bromoimidazo[1,5-a]pyridin-3-yl)(3-méthoxy-4-nitrophényl)méthanone obtenu à l'exemple 20, dans 160ml de N,N-diméthylformamide, on ajoute sous atmosphère d'azote 2,17g (18,48 mmoles) de cyanure de zinc puis 1,07g (0,93 mmole) de tétrakis(triphénylphosphine)palladium(0). Le milieu réactionnel est chauffé à 90°C pendant 17h. Le précipité obtenu est filtré, lavé à l'eau puis avec une solution aqueuse saturée de bicarbonate de sodium et à l'eau. Après séchage on recueille 5,9g d'un solide jaune. Point de fusion : 219°C ; RMN-¹H (DMSO-*d6*): 4,01(3H, s); 7,53-7,55(1H, m); 7,76-7,81(1H, m); 7,95-8,01(2H, m); 8,08(1H, d); 8,19(1H, d); 9,70(1H, d)

### Exemple 76

### (1-aminoimidazo[1,5-a]pyridin-3-yl)(3-méthoxy-4-nitrophényl)méthanone

A 5g (13,29 mmoles) de (1-bromoimidazo[1,5-a]pyridin-3-yl)(3-méthoxy-4-nitrophényl)méthanone obtenu à l'exemple 20, dans 66ml de N,N-diméthylformamide, on ajoute sous atmosphère d'azote 8,67g (26,61 mmoles) de carbonate de césium puis 4,5ml (26,82 mmoles) de benzophénone imine. Après agitation pendant 30 minutes on ajoute 1,66g (2,67 mmoles) de 2,2'-Bis(diphénylphosphino)-1,1'-binaphtyl puis 1,22g (1,33 mmole) de tris(dibenzelidèneacétone)dipalladium(0). Le milieu réactionnel est chauffé pendant 3 heures puis concentré sous pression réduite. Le résidu est repris par un mélange de dichlorométhane et d'eau. La phase organique est décantée, séchée sur sulfate de sodium, concentrée sous pression réduite. Le résidu est repris dans 250ml de tétrahydrofurane et ajoute 135ml d'une solution aqueuse d'acide chlorhydrique 2N. Après une heure d'agitation à température ambiante, le milieu réactionnel est concentré sous pression réduite. Le résidu solide obtenu est repris à l'acétone, filtré, lavé à l'acétone puis à l'éther éthylique et séché. On recueille 3,43g d'un solide marron. Point de fusion : 214°C ; RMN-¹H (DMSO-*d6*) 4,02(3H, s) ; 7,29-7,36(2H, m); 7,96-8,04(2H, m); 8,11(1H, d); 8,16(1H, s); 9,78(1H, d)

### Exemple 77

### 3-méthoxy-N-[3-(3-métboxy-4-nitrobenzoyl)imidazo[1,5-a]pyridin-1-yl]benzamide

A 0,8g (2,29 mmoles) d'acide 3-méthoxybenzoïque dans 20ml d'acétonitrile, on ajoute, sous atmosphère d'azote, 0,78ml (5,05 mmoles) de triéthylamine puis 1,17g (2,65 mmoles) de BOP. Après agitation pendant 30 minutes à température ambiante on ajoute 0,8g (2,29 mmoles) de (1-aminoimidazo[1,5-a]pyridin-3-yl)(3-méthoxy-4-nitrophényl)méthanone obtenu à l'exemple 76, puis chauffe à 80°C pendant 20 heures. Le milieu réactionnel est repris par un mélange d'eau et d'acétate d'éthyle. La phase organique est décantée, lavée par une solution aqueuse saturée de chlorure de sodium, séchée sur sulfate de sodium et concentrée sous pression réduite. Le résidu est purifié par chromatographie colonne sur gel de silice en éluant avec un mélange de dichlorométhane-acétone (99-1). On recueille 0,618g d'un solide orangé. Point de fusion : 167°C ; RMN-¹H (DMSO-*d6*) : 3,87(3H, s) ; 4,02(3H, s); 7,21(1H, d); 7,41-7,49(3H, m); 7,63-7,66(2H, m); 7,95-8,10(3H, m); 8,11(1H, s); 9,80(1H, d)

### Exemple 78

### N-[3-(3-méthoxy-4-nitrobenzoyl)imidazo[1,5-a]pyridin-1-yl]acétamide

A 0,8g (2,56 mmoles) de (1-aminoimidazo[1,5-a]pyridin-3-yl)(3-méthoxy-4-nitrophényl)méthanone obtenu à l'exemple 76 dans 20ml de 1,2-dichloroéthane, on ajoute sous atmosphère d'azote 0,54ml (3,84 mmoles) de triéthylamine puis 0,21ml (2,95 mmoles) de chlorure d'acétyle. Le milieu réactionnel est agité à température ambiante pendant 16 heures puis repris par un mélange de dichlorométhane et d'eau. La phase organique est décantée, séchée sur sulfate de sodium et concentrée sous pression réduite. Le résidu obtenu est purifié par chromatographie colonne sur gel de silice en éluant avec un mélange de dichlorométhane-acétone (94-6). On recueille 0,523g d'un solide orange. Point de fusion : 256°C ; RMN-¹H (DMSO-*d6*) : 2,14(3H, s); 4,02(3H, s); 7,34-7,44(2H, m); 7,94-8,08(3H, m); 8,09(1H, s); 9,75(1H, d)

### Exemple 79

### (3-méthoxy-4-nitrophényl)[1-(métbylamino)imidazo[1,5-a]pyridin-3-yl]méthanone

### ETAPE A

### 2,2,2-trifluoro-N-[3-(3-méthoxy-4-nitrobenzoyl)imidazo[1,5-a]pyridin-1-yl]acétamide

Ce composé est préparé selon le même procédé que celui décrit dans l'exemple 78 par acylation de 1.2g (3.44 mmoles) de chlorhydrate de (1-aminoimidazo[1,5-a]pyridin-3-yl)(3-méthoxy-4-nitrophényl)méthanone par l'anhydride trifluoroacétique dans le 1,2-dichloroéthane en présence de triéthylamine. On obtient 1.08g d'un solide jaune. Point de fusion : 228°C ; RMN-¹H (DMSO*-d6*) : 4,03(3H,s); 7,43-7,47(1H,m); 7,517,55(1H,m); 7,93-7,99(2H,m); 8,04-8,11(2H,m); 9,76(1H,d)

### ETAPE B

A une solution de 1.03g (2.52 mmoles) de 2,2,2-trifluoro-*N*-[3-(3-méthoxy-4-nitrobenzoyl)imidazo[1,5-a]pyridin-1-yl]acétamide dans 35ml de diméthylformamide on ajoute à 0°C 0.121g (3.03 mmoles) d'hydrure de sodium (en dispersion à 60% dans l'huile). Le milieu réactionnel est agité 1 heure à cette température puis on ajoute 0.189ml (3.03 mmoles) d'iodure de méthyle. L'introduction terminée on laisse revenir à température ambiante et agite 20 heures. On ajoute 20ml de méthanol puis 0.523g (3.78 mmoles) de carbonate de potassium et agite 2 heures à température ambiante. Le milieu réactionnel est versé sur de l'eau et extrait au dichlorométhane. La phase organique est décantée, lavée à l'eau, séchée sur sulfate de sodium et concentrée sous pression réduite. On obtient 0.86g d'un solide rouge qui est salifié sous forme de chlorhydrate. On obtient 625mg d'un solide rouge. Point de fusion : 208°C ; RMN-¹H (DMSO-d6) : 3,00(3H,s); 4,04(3H,s); 7,33-7,36(2H,m); 7,99-8,09(3H,m); 8,59(1H,s); 9,89(1H,d)

### Exemple 80

### N-[3-(3-méthoxy-4-nitrobenzoyl)imidazo[1,5-a]pyridin-1-yl]méthanesulfonamide

A 0,473g (1.36 mmole) de chlorhydrate de (1-aminoimidazo[1,5-a]pyridin-3-yl)(3-méthoxy-4-nitrophényl)méthanone obtenu à l'exemple 76 dans 14ml de pyridine, on ajoute sous atmosphère d'azote à une température de 5°C 116µl (1.49 mmole) de chlorure de mésyle. L'introduction terminée, on laisse revenir à température ambiante et agite trente minutes. Le milieu réactrionnel est versé sur 95ml d'acide chlorhydrique 2N et extrait à l'acétate d'éthyle. La phase organique est lavée à l'eau, décantée, séchée sur sulfate de sodium et concentrée sous pression réduite. Le résidu solide obtenu est repris à l'éther isopropylique, filtré lavé à l'éther isopropylique puis séché. On recueille 0,40g d'un solide orange. Point de fusion : 231°C ; RMN-¹H (DMSO-*d6*) : 3,24(3H, s); 4,02(3H, s); 7,40-7,44(1H, m); 7,49-7,53(1H, m); 7,88-7,92(1H,d);8,00-8,07(2H,m); 8,26(1H,s); 9,75(1H,d)

### Exemple 81

### (4-amino-3-méthoxyphényl)[1-(4-méthoxyphényl)imidazo[1,5-a]pyridin-3-yl]méthanone

A 0,835g (2,07 mmoles) de (3-méthoxy-4-nitrophényl)[1-(4-méthoxyphényl)imidazo[1,5-a]pyridin-3-yl]méthanone obtenu à l'exemple 25, dans 30ml de dioxanne et 10ml d'éthanol, on ajoute 0,167g de Pd/C à 10% puis 2,1ml (21 mmoles) de cyclohexène et chauffe à reflux 7 heures. Le milieu réactionnel est refroidi, filtré sur talc. Le filtrat est concentré sous pression réduite. Le produit est purifié par chromatographie colonne sur gel de silice en éluant avec un mélange de toluène-acétate d'éthyle (97-3). On obtient 0,760g d'un solide jaune. Le produit est salifié par dissolution de la poudre précédemment obtenue dans l'acétone puis ajout de 3,8ml (2,6 équivalents) d'acide chlorhydrique 1N dans l'éther éthylique. Après addition d'éther éthylique, le précipité obtenu est filtré, lavé à l'éther éthylique puis séché. On recueille 0,553 g d'un solide jaune sous forme de chlorhydrate. Point de fusion : 232°C; RMN-¹H (DMSO-*d6*) : 3,85(3H, s); 3,94(3H, s); 6,95(1H, d); 7,10-7,26(3H, m); 7,36-7,40(1H, m); 7,96(2H, d); 7,98-8,25(3H, m); 9,76(1H, d)

### Exemples 82 à 95

En procédant selon la préparation décrite à l'exemple 81, on synthétise les composés de formule générale Id décrits dans le tableau VI ci dessous, par réduction de la fonction nitro des composés de formule Ia avec le cyclohexène en présence de Pd/C à 10% comme catalyseur.

**TABLEAU VI**

| Ex. | R | R₁ | R₂ | R₃ | Sel | Point de Fusion (°C) |
|---|---|---|---|---|---|---|
| 82 | H | H | CO₂Me | NH₂ | - | 195 |
| 83 | 6-CO₂M | H | OMe | NH₂ | - | 179 |
| 84 | 6-CONH₂ | H | OMe | NH₂ | HCl, 0.4H₂O | 224 |
| 85 | H | CN | OMe | NH₂ | HCl | 224 |
| 86 | H | NHCOCH₃ | OMe | NH₂ | HCl, 0.4H₂O | 237 |
| 87 | 7-CONHCH₂CO₂Et | H | OMe | NH₂ | HCl | 219 |
| 88 | (R)-7-CONHCH(CH₃)CO₂Me | H | OMe | NH₂ | HCl, 1.42H₂O | 192 |
| 89 | (S)-7-CONHCH(CH₃)CO₂Me | H | OMe | NH₂ | HCl, 0.65H₂O | 190 |
| 90 | 6-CONHCH₂CO₂Me | H | OMe | NH₂ | HCl, 0.3H₂O | 190 |
| 91 | (S)-6-CONHCH(Bn)CO₂Me | H | OMe | NH₂ | HCl, H₂O | 230 |
| 92 | 6-CONHCH₂CH₂CO₂Me | H | OMe | NH₂ | HCl, H₂O | 174 |
| 93 | (S)-6-CONHCH(Me)CO₂Me | H | OMe | NH₂ | HCl | 124 |
| 94 | 6-NHMe | H | CO₂Me | NH₂ | - | 122 |
| 95 | 6-CONHMe | H | OMe | NH₂ | HCl, 1.05H₂O | 256 |

Les RMN des exemples 82 à 95 du TABLEAU VI sont présentées dans le TABLEAU VI' ci-dessous :

**TABLEAU VI'**

| Ex. | RMN-¹H (DMSO*-d6*) |
|---|---|
| 82 | 3,87(3H, s); 6,91(1H, d); 7,19-7,22(1H, m); 7,30-7,36(1H, m); 7,85(1H, s); 7,96(1H, d); 8,43(1H, d); 9,12(1H, s); 9,69(1H, d) |
| 83 | 3,87(3H, s); 3,94(3H, s); 6,71(1H, d); 7,61(1H,d); 7,89(1H, s); 7,94-7,98(2H, m) ; 8,25(1H, d); 10,27(1H, s) |
| 84 | 3,89(3H, s); 6,82(1H, d); 7,65(1H, d); 7,85(1H, s); 7,94-7,98(2H, m); 8,21(1H, d); 10,15(1H, s) |
| 85 | 3,88(3H, s); 6,82(1H, d); 7,33-7,39(1H, m); 7,61-7,68(1H, m); 7,79(1H, s); 8,08(2H, d); 9,59(1H, d) |
| 86 | 2,15(3H, s); 3,90(3H, s); 6,90(1H, d); 7,15-7,28(2H, m); 7,85-7,91(2H, m); 8,15(1H, d); 9,67(1H, d) |
| 87 | 1,21-1,27(3H, s); 3,89(3H, s); 4,05-4,08(2H, d); 4,12-4,20(2H, q); 6,76-9,65 (2H, m); 7,49-8,24(2H, m); 7,96(1H, s); 8,04(1H, s); 8,47(1H, s); 9,25(1H, t) |
| 88 | 1,44-1,47(3H, d); 3,68(3H, s); 3,98(3H, s); 4,51-4,57(1H, m); 6,75-9,65(2H, m); 7,51-8,24(2H, m); 7,97(1H, s); 8,04(1H, s); 8,51(1H, s); 9,08-9,10(1H, d) |
| 89 | 1,44-1,45(3H, d), 3,73(3H, s); 3,92(3H, s); 4,53-4,56(1H, m); 6,74-9,61(2H, m); 7,50-8,22(2H, m); 7,96(1H, s); 8,03(1H, s); 8,49(1H, s); 9,05-9,08(1H, d) |
| 90 | 3,70(3H,s); 3,89(3H,s); 4,09(2H,d); 6,83(1H,d); 7,64(1H,d); 7,88(1H,s); 7,98-8,02(2H,m); 8,22(1H,d); 10,18(1H,s) |
| 91 | 3,09-3,27(2H,m); 3,68(3H,s); 3,89(3H,s); 4,69-4,78(1H,m); 6,82(1H,d), 7,19-7,37(5H,m); 7,56(1H,d); 7,86(1H,s); 7,95-7,99(2H,m); 8,21(1H,d); 10,15(1H,s); 10,12(1H,s) |
| 92 | 2,64(2H,t); 3,55(2H,m); 3,63(3H,s); 3,88(3H,s); 6,84(1H,d); 7,61(1H,d); 7,85 (1H,s); 7,93-7,98(2H,m); 8,22(1H,d); 10,12(1H,s) |
| 93 | 1,44(3H,d); 3,68(3H,s); 3,88(3H,s); 4,49(1H,q); 6,81(1H,d); 7,64(1H,d); 7,76 (1H,s); 7,85-7,98(2H,m); 8,22(1H,d); 10,12(1H,s) |
| 94 | 2,75(3H,d); 3,86(3H,s); 6,88(1H,d); 6,97(1H,d); 7,66(1H,s); 7,72(1H,d); 8,45(1H,d); 8,97(1H,s); 9,11(1H,s) |
| 95 | 2,84(3H,d); 3,89(3H,s); 6,87(1H,d); 7,64(1H,d); 7,86(1H,s); 7,96-8,00(2H,m); 8,21(1H,d 10,15(1H,s |

### Exemple 96

### (4-amino-3-méthoxyphényl)[1-(1H-pyrrol-2-yl)imidazo[1,5-a]pyridin-3-yl]méthanone

A 0,480g (0,001 mole) de 2-[3-(3-méthoxy-4-nitrobenzoyl)imidazo[1,5-a]pyridin-1-yl]pyrrole-1-carboxylate de tert-butyl obtenu à l'exemple 38, en solution dans un mélange de 13mil d'eau et 7ml d'éthanol, on ajoute 1,78ml d'acide acétique et 0,209g de fer. On chauffe à 70°C pendant 7 heures puis on laisse revenir à température ambiante et verse le milieu réactionnel sur une solution aqueuse de soude 1N et extrait au dichlorométhane. La phase organique est décantée, séchée sur sulfate de sodium puis concentrée sous pression réduite. Le produit est purifié par chromatographie colonne sur gel de silice en éluant avec du toluène puis un mélange de toluène-acétate d'éthyle (90-10). On obtient 200mg d'une huile marron qui est salifiée sous forme de chlorhydrate. On obtient 60mg d'un solide rouge. Point de fusion : 136°C ; RMN-¹H (DMSO-d6): 3,91(3H, s); 6,21(1H, m); 6,68(1H, m); 6,81(1H, d); 6,90(1H, m); 7,17-7,33(2H, m); 7,98(1H, s); 8,15(1H, d); 8,38(1H, d); 9,73(1H, d)

### Exemples 97 à 119

En procédant selon la préparation décrite à l'exemple 96, on synthétise les composés de formule générale Id décrits dans le tableau VII ci dessous, par réduction de la fonction nitro des composés de formule Ia avec le fer et l'acide acétique.

**TABLEAU VII**

| Ex. | R | R₁ | R₂ | R₃ | Sels | Point de Fusion (°C) |
|---|---|---|---|---|---|---|
| 97 | H | 3-chlorophényl | OMe | NH₂ | 0.8HCl | 230 |
| 98 | H | 4-chlorophényl | OMe | NH₂ | 0.7HCl | 230 |
| 99 | H | 4-fluorophényl | OMe | NH₂ | 1HCl, 0.02H₂O | 214 |
| 100 | H | 3-pyridinyl | OMe | NH₂ | 1HCl, 1.97H₂O | 248 |
| 101 | H | 4-pyridinyl | OMe | NH₂ | 1HCl, 0.89 H₂O | 237 |
| 102 | H | Br | OMe | NH₂ | 0.3HCl, 0.15 H₂O | 204 |
| 103 | 8-CO₂Et | H | OMe | NH₂ | 1HCl, 0.6 H₂O | 189 |
| 104 | 7- CO₂Et | H | OMe | NH₂ | HCl | 220 |
| 105 | H | NHCOPh-3-OMe | OMe | NH₂ | 0.7HCl, 0.15 H₂O | 199 |
| 106 | H | -NHSO₂CH₃ | OMe | NH₂ | 1HCl, 0.95H₂O | 226 |
| 107 | H | NH-Me | OMe | NH₂ | 1.9HCl | 210 |
| 108 | (S)-6-CONHCH(CH₂OH)CO₂Me | H | OMe | NH₂ | 1HCl, 1.2 H₂O | 179 |
| 109 | 6-NHSO₂CH3 | H | OMe | NH₂ | 0.4HCl | 125 |
| 110 | 6-NHCOCH3 | H | OMe | NH₂ | HCl | 228 |
| 111 | H | -Ph-3-CO₂Me | OMe | NH₂ | - | 166 |
| 112 | 7-CO₂Et | 4-methoxypyridin-3-yl | OMe | NH₂ | - | MH+=447 |
| 113 | H | 4-fluorophényl | CO₂Me | NH₂ | - | 234 |
| 114 | H | 3-fluoro-4-méthoxyphényl | OMe | NH₂ | HCl, 0.14 H₂O | 226 |
| 115 | 7-OBn | H | OMe | NH₂ | 1.2HCl, 0.2 H₂O | 210 |
| 116 | (S)-7-CONHCH(CH₂OH)CO₂Me | H | OMe | NH₂ | 1.55HCl | 209 |
| 117 | H | 3-fluorophényl | OMe | NH₂ | HCl, 1.25 H₂O | 228 |
| 118 | H | 4-chlorophényl | CO₂Me | NH₂ | - | 268 |
| 119 | 8-OBn | H | OMe | NH₂ | 1.5HCl, 0.1 H₂O | 174 |

Les RMN des exemples 97 à 119 du TABLEAU VII sont présentées dans le TABLEAU VII' ci-dessous :

**TABLEAU VII'**

| Ex. | RMN-¹H (DMSO-*d6*) |
|---|---|
| 97 | 3,92(3H, s); 6,81(1H, d); 7,22-7;58(4H, m); 7,99-8,31(5H, m); 9,73(1H, d) |
| 98 | 6.81(1H, d); 7,20-7,45(2H, m) ; 7,57-7,61(2H, m) ; 8,04-8,29(5H, m) ; 9,73(1H, d) |
| 99 | 3,92(3H, s); 6,86(1H, d); 7,23-7,42(4H, m); 8,03-8,27(5H, m); 9,75(1H, d) |
| 100 | 3,92(3H,s); 6,80(1H, d); 7,28-7,56(2H,m); 8,04-8,18(2H, m); 8,21 (1H, d); 8,44(1H, d); 8,81(1H, d); 9,05(1H, d); 9,41(1H, s); 9,74(1H, d) |
| 101 | 3,91(3H, s); 6,82(1H, d); 7,37-7,75(2H, m); 7,97(1H, s); 8,16(1H, d); 8,57-8,62(3H, m); 8,87(2H, d); 9,71(1H, d) |
| 102 | 3,88(3H, s); 6,80(1H, d); 7,25-7,28(1H, m); 7,38-7,45(1H, m); 7,76(1H, d); 7,84(1H, s); 8,12(1H, d); 9,67(1H, d) |
| 103 | 1,42(3H, t), 3,88(3H, s); 4,45(2H, q); 6,78(1H, d); 7,25(1H, t); 7,92 (1H, s); 7,99(1H, d); 8,14-8,20(2H, m); 9,82(1H, d) |
| 104 | 1,36(3H, t); 3,86(3H, s); 4,36(2H, s); 6,70(1H, d); 7,45(1H, d); 7,92 (1H, s); 8,20(1H, d); 8,57(1H, s); 9,59(1H, d) |
| 105 | 3,86(3H, s); 3,88(3H, s); 6,80(1H, d); 7,18-7,31(3H, m); 7,44-7,50(1H, m); 7,65-7,69(2H, m); 7,81(1H, d); 7,89(1H, s); 8,22(1H, d); 9,71(1H, d) |
| 106 - | 3,24(3H,s); 3,89(3H,s); 6,84(1H,d); 7,18-7,7,23(1H,m); 7,30-7,36(1H, m); 7,89 (1H,d); 7,99(1H,d); 8,06(H,s); 9,66(1H,d) |
| 107 | 3,29(3H,s); 4,19(3H,s); 7,41-7,57(3H,m); 8,08-8,35(2H,m); 8,36(1H,s); 9,98 (1H,d) |
| 108 | 3,69(3H,s); 3,84(2H,m); 3,90(3H,s); 4,56-4,63(1H,m); 6,87(1H,d); 7,73(1H,d); 7,88(1H,s); 7,97-8,02(2H,m); 8,21(1H,d 10,18(1H,s) |
| 109 | 3,09(3H,s); 3,87(3H,s); 6,78(1H,d); 7,22(1H,d); 7,81(1H,s 7,92-7,94(2H,m); 8,21(1H 9,80(1H,s) |
| 110 | 2,13(3H,s); 3,89(3H,s); 6,88(1H,d); 6,91(1H,d); 7,77(1H,s); 7,85(1H,d); 7,95 (1H,s); 8,11(1H,d); 10,32(1H,s) |
| 111 | (CDCl₃) 4,00(3H, s); 4,05(3H, s); 6,82-7,06(2H, m); 7,01-7,04(1H, m); 7,29-7,63(2h, m); 7,58-8,05(2H, m); 8,07-9,40(2H, m); 8,33(1H, s) |
| 112 | 1,35-1,39(3H, t); 3,89(3H, s); 3,96(3H, s); 4,38-4,40(2H, q); 5,93(2H, m); 6,72-7,06(2H, m); 7,46-8,29(2H, m); 8,06(1H, s); 8,21-9,62(2H, m); 8,53(1H, m); 8,76(1H, m) |
| 113 | 3,89(3H, s); 6,89-7,42(2H m); 7,21-8,10(2H, m); 7,35-8,27(2H, m); 7,35-7,43(4H, m); 8,39-9,84(2H, m); 9,49(1H, s) |
| 114 | 3,93(6H, m); 6,82-7,37(2H, m); 7,21-7,40(2H,m); 7,78-8,19(2H, m); 8,15(1H, m); 8,24-9,75(2H, m) |
| 115 | 3,88(3H, s); 5,02(2H, m); 5,25(2H, s); 6,78-9,63(2H, m); 6,95-8,20(2H, m); 7,39-7,55(6H, m); 7,60(1H, s); 7,96(1H, s) |
| 116 | 3,68(3H, s); 3,83-83,85(2H, d); 3,88(3H, s); 4,55-4,62(1H, m); 5,20-5,41(2H, m); 6,77-9,65(2H, m); 7,54-8,24(2H, m); 8,04(1H, s); 8,19(1H, s); 8,56(1H, s); 8,96-8,99(1H, d) |
| 117 | 3,94(3H, s); 5.35(2H, m); 6,90-9,76(2H, m); 7,22-7,28(2H, m); 7,41-7,60(2H, m); 7,87-7,88(2H, m); 7,88-8,34(2H, m); 8,14-8,19(2H, m) |
| 118 | 6,88-8,30(2H, m); 7,22-7,45(2H, m); 7,55-8,01(4H, m); 8,27-8,30(2H, m); 9,29(1H, s); 9,73(1H, m) |
| 119 | 3,88(3H, s); 6,76-9,29(2H, m); 7,03-7,09(1H, t); 6,80-8,23(2H, m); 7,82(1H, s); 7,92(1H, s) |

### Exemple 120

### (4-amino-3-méthoxyphényl)[1-(3-méthoxyphényl)imidazo[1,5-a]pyridin-3-yl]méthanone

A 0,441g (1 mmole) de (3-méthoxy-4-nitrophényl)[1-(3-méthoxyphényl)imidazo[1,5-a]pyridin-3-yl]méthanone obtenu à l'exemple 34, dans 10ml de méthanol, on ajoute 0,117g de Pd/C à 10% puis 0,27ml (5,47 mmoles) d'hydrate d'hydrazine. On chauffe à 70°C 3 heures. Le milieu réactionnel est filtré sur talc et le catalyseur est lavé au méthanol. Le filtrat est concentré sous pression réduite. Le résidu est repris au dichlorométhane et lave avec une solution aqueuse saturée de chlorure de sodium puis séche sur sulfate de sodium. Après concentration sous pression réduite, le résidu est purifié par chromatographie colonne sur gel de silice en éluant au dichlorométhane. On recueille 0,354g d'une mousse jaune. Le produit est salifié par ajout d'acide chlorhydrique 1N dans l'éther éthylique. Après addition d'éther éthylique, le précipité est filtré, lavé à l'éther éthylique puis séché. On recueille un solide jaune sous forme de chlorhydrate. Point de fusion : 210°C ; RMN-¹H (DMSO-*d6*) : 3,86(3H, s); 3,92(3H, s); 6,88(1H, d); 6,99(1H, d); 7,22(1H, t)-7,40-7,60(5H, m); 8,14-8,27(3H, m); 9,75(1H, d)

### Exemples 121 à 148

En procédant selon la préparation décrite à l'exemple 120, on synthétise les composés de formule générale Id décrits dans le tableau VIII ci dessous, par réduction de la fonction nitro des composés de formule la avec l'hydrate d'hydrazine en présence de Pd/C à 10% comme catalyseur.

**TABLEAU VIII**

| Ex. | R | R₁ | R₂ | R₃ | Sels | Point de Fusion (°C) ou M.S. |
|---|---|---|---|---|---|---|
| 121 | H | 2-méthoxyphényl | OMe | NH₂ | 1HCl, 0.4H₂O | 211 |
| 122 | H | phényl | OMe | NH₂ | 1.4HCl, 0.4 H₂O | 195 |
| 123 | H | 2-(5-carboxythiényl) | OMe | NH₂ | Na | 275 |
| 124 | H | 3-furyl | OMe | NH₂ | 1HCl, 0.5 H₂O | 187 |
| 125 | H | 3-thiényl | OMe | NH₂ | 1HCl, 1.1 H₂O | 200 |
| 126 | H | 2-thiényl | OMe | NH₂ | 1.65HCl | 221 |
| 127 | H | 3-carboxyphényl | OMe | NH₂ | 1Na, 2.5 H₂O | 239 |
| 128 | H | 3-pyridinyl | CO₂Me | NH₂ | - | 234 |
| 129 | H | 2-thiényl | CO₂Me | NH₂ | - | 143 |
| 130 | H | 3-méthoxyphényl | CO₂Me | NH₂ | - | 179 |
| 131 * | H | 3-thiényl | CO₂H | NH₂ | 1.8Na, 2.6H₂O | 284 |
| 132 | H | 2-méthoxyphényl | CO₂Me | NH₂ | - | MH+ =402 |
| 133 | H | H | OMe | NH₂ | HCl, 0.2 H₂O | 218 |
| 134 | H | 4-méthoxypyridin-3-yl | OMe | NH₂ | HCl, 0.66 H₂O | 205 |
| 135 | 8-CONMe₂ | H | OMe | NH₂ | HCl, 1.7 H₂O | 125 |
| 136 | H | 3-furyl | CO₂Me | NH₂ | - | MH+=362 |
| 137 | 7-CONHMe | H | OMe | NH₂ | HCl | 249 |
| 138 | 7-CONH₂ | H | OMe | NH₂ | HCl | 252 |
| 139 | 7-CONMe₂ | H | OMe | NH₂ | HCl | déc.360 |
| 140 | 7-CONHMe | 3-carboxyphényl | OMe | NH₂ | Na | déc.360 |
| 141 | 7-CO₂Et | 3-méthoxyphényl | OMe | NH₂ | - | MH+=432 |
| 142 | H | 4-carboxyphényl | OMe | NH₂ | HCl | 257 |
| 143 | H | 4-methoxypyridin-3-yl | CO₂Me | NH₂ | - | 204 |
| 144 | 8-Me | H | OMe | NH₂ | HCl | 221 |
| 145 | 7-OH | H | OMe | NH₂ | HCl | 238 |
| 146 | 8-CONHMe | H | OMe | NH₂ | HCl, 1.1 H₂O | 243 |
| 147 | 8-OH | H | OMe | NH₂ | 1HCl, 1.35H₂O | 254 |
| 148 | 7-Me | H | OMe | NH₂ | HCl | 235 |

| | | | | | | |
|---|---|---|---|---|---|---|
| *ester méthylique en R₃ saponifié lors de l'hydrogénation | | | | | | |

Les RMN des exemples 121 à 148 du TABLEAU VIII sont présentées dans le TABLEAU VIII' ci-dessous :

**TABLEAU VIII'**

| Ex. | RMN-¹H (DMSO-*d6*) ou M.S. |
|---|---|
| 121 | 3.85(3H, s), 3.90(3H, s), 6.88(1H, d), 7.09-7.44(5H, m), 7.66(1H, d), 7.77(1H, d), 8.11(1H, s), 8.15(1H, d), 9.72(1H, d) |
| 122 | 3.93(3H, s), 6.85(1H, d), 7.23-7.58(5H, m), 8.03(2H, d), 8.16-8.30 (3H, m), 9.75(1H, d) |
| 123 | 3,94(3H,s); 6,84(1H, d); 7,25-7,53(2H,m); 7,75-7,80(2H, m); 8,07 (1H, d); 8,21(1H, s); 8,33 (1H, d); 9,72(1H, d) |
| 124 | 3,93(3H,s); 6,88(1H, d), 7,12(2H, m); 7,22-7,39(2H, m); 7,85(1H, s); 8,14-8,22(3H, m); 8,44(1H, s); 9,74(1H, d) |
| 125 | 3,92(3H, s); 6,81(1H, d); 7,22-7,41(2H, m); 7,72-7,81(2H, m); 8,09 (1H, m); 8,17(2H, m); 8,30(1H, d); 9,75(1H, d) |
| 126 | 6,80(1H,d) ; 7,22-7,44(3H, m) ; 7,60(1H, d) ; 7,72(1H, d) ; 8,06(1H, m) ; 8,28-8,33(2H, m) ; 9,74(1H, d) |
| 127 | 3,94(3H, s), 6,75(1H, d); 7,20-7,47(3H, m); 7,87-7,95(2H, m); 8,16-8,22(2H, m); 8,27(1H, s); 8,58(1H, s); 9,75(1H, d) |
| 128 | 3,90(3H, s); 6,92(1H, d); 7,26-7,46(4H, m); 7,46-7,59(1H, m); 8,33-8,58(3H, m); 8,60(1H, d); 9,29(1H, s); 9,51(1H, s); 9,76(1H, d) |
| 129 | 3,89(3H, s); 6,81-7,60(6H, m); 8,20-8,43(2H, m); 9,13(1H, s); 9,75 (1H, m) |
| 130 | 3,87(3H, s); 3,89(3H, s); 6,91-6,94(2H, m); 7,24-7,62(5H, m); 8,27-8,40(2H, d); 9,55(1H, d); 9,77(1H, d) |
| 131 | 6.59(1H, d), 7.12-7.35(2H, m), 7.70-7.73(1H, m), 7.82(1H, d), 8.06 (1H, d), 8.24-8.32(2H, m), 9.09(1H, s), 9.69(1H, d) |
| 132 | MH+ = 402 |
| 133 | 3,90(3H, s); 6,93(1H, d); 7,16-7,21(1H, m); 7,29-7,35(1H, m); 7,84(1H, s); 7,96(1H, d); 7,98(1H, s); 8,18(1H, d); 9,68(1H, d) |
| 134 | 3,93(3H, s); 3,95(3H, s); 7,03(1H, d); 7,22-7,45(4H, m); 8,13-8,35 (4H, m); 8,80(1H, s); 9,76(1H, d) |
| 135 | 3,91(3H, s); 6,89(1H, d); 7,21(1H, t); 7,32(1H, d); 7,70(1H, s); 7,95 (1H, s); 8,18(1H, d); 9,66(1H, d) |
| 136 | 3,89(3H, s);7,12-7,64(5H, m); 7,86-7,89(1H, m); 8,15-8,25(1H, m); 8,43-8,46(1H, m); 9,39(1H, s); 9,75(1H, d) |
| 137 | 2,84(3H, d); 5,96(2H, m); 6,79-7,53(2H, m); 7,96(1H, s); 8,00(1H, s); 8,19-9,63(2H, m); 8,41(1H, s); 8,76(1H, m) |
| 138 | 5,80(2H, m); 6,73-7,54(2H, m); 7,64(1H, m); 7,96(1H, s); 7,64(1H, s); 8,20-9,26(2H, m); 8,45(1H, s) |
| 139 | 3,88(3H, s); 5,51(2H, m); 6,76-7,17(2H, m); 7,90(1H, s); 7,94(1H, s); 8,02(1H, s); 8,19-p,64(2H, m) |
| 140 | 2,86(3H, d); 3,94(3H, s); 5,89(2H, s); 6,74-7,47(2H,m); 7,52-8,20(2H, m); 7,90-7,99(1H, m); 8,27(1H, s); 8,58-9,71(2H, m); 8,91(1H, m) |
| 141 | CDCl₃ :1,27-1,50(3H, t); 3,95(3H, s); 4,02(3H, s); 4,44-4,52(2H, q); 6,79-7,62(2H, m); 6,99-7,52(2H, m); 7,29-7,53(2H, m); 8,23(1H, s); 8,40-9,80(2H, m); 8,78(1H, s) |
| 142 | 3,94(3H, s); 6,88-8,20(2H, m); 7,26-7,48(2H, m); 8,08-8,20(5H, m); 8,34-9,77(2H, m) |
| 143 | CDCl3 : 3,98(3H, s); 4,05(3H, s); 6,30(2H, m); 6,75-6,95(2H, m); 7,05-7,29(2H, m); 7,99-9,92(2H, m); 8,26-8,64(2H, m); 8,79(1H, s); 9,51(1H, s) |
| 144 | 2,59(3H, s); 3,89(3H, s); 6,83-8,20(2H, m); 7,06-9,55(2H, m); 7,10(1H, m); 7,86(1H, s); 7,95(1H, s) |
| 145 | 3,88(3H, s); 6,82-6,88(2H, m); 7,05(1H, m); 7,51(1H, s); 7,95(1H, s); 8,12-9,65(2H, m) |
| 146 | 2,86-2,87(3H, d); 3,89(3H, s); 5,23-5,25(2H, m); 6,83-9,75(2H, m); 7,20-7,26(1H, t); 7,67-8,21(2H, m); 7,93(1H, s); 8,10(1H, s); 8,74-8,75(1H, m) |
| 147 | 3,91(3H, m); 6,42(2H, m); 6,43-8,18(2H, m); 6,98-7,07(2H, m); 7,85(1H, s); 7,96(1H, s); 9,24-9,27(1H, m) |
| 148 | 2,41(3H, s); 3,89(3H, s); 6,00(2H, m); 6,82-8,21(2H, m); 7,02-9,62(2H, m); 7,69-7,71(2H, m); 7,96(1H, m) |

### Exemple 149

### 3-(4-amino-3-méthoxybenzoyl)imidazo[1,5-a]pyridine-1-carboxamide

A 0,5g (1,7 mmole) de 3-(4-amino-3-méthoxybenzoyl)imidazo[1,5-a]pyridine-1-carbonitrile obtenu à l'exemple 85 dans 50ml d'éthanol, sous atmosphère d'azote, on ajoute 4,4ml (26,4 mmoles) d'une solution aqueuse de soude 6N. Après 2 heures de chauffage au reflux, le milieu réactionnel est concentré sous pression réduite. Le résidu obtenu est lavé à l'eau, à l'acétone à l'éther éthylique puis séché. On recueille 0,447g d'un solide jaune. Le produit est salifié par ajout d'une solution d'acide chlorhydrique 1N dans l'éther éthylique. On obtient 0,310g d'un solide jaune. Point de fusion : 241°C ; RMN-¹H (DMSO-*d6*) *:* 3,89(3H, s) ; 6,74(1H, d); 7,24-7,27(1H, m); 7,45-7,49(1H, m); 7,88(1H, s); 8,33(1H, d); 8,42(1H, d); 9,65(1H, d)

### Exemple 150

### 3-(4-amino-3-méthoxybenzoyl)imidazo[1,5-a]pyridine-1-carboxylate de méthyle

A 20ml d'une solution de méthanol saturée en acide chlorhydrique à une température de 5°C on ajoute 0,61g (2,09 mmoles) de 3-(4-arnino-3-méthoxybenzoyl)imidazo[1,5-a]pyridine-1-carbonitrile obtenu à l'exemple 85, puis laisse revenir à température ambiante et agite 17 heures. Le milieu réactionnel est concentré sous pression réduite. Le résidu est repris dans 14ml d'une solution aqueuse d'acide chlorhydrique 1N puis chauffé à 70°C pendant 5h. Le milieu réactionnel est basifié avec du bicarbonate de sodium et extrait avec un mélange d'acétate d'éthyle et de tétrahydrofurane. La phase organique est lavée à l'eau, séchée sur sulfate de sodium, concentrée sous pression réduite. Le résidu obtenu est repris au dichlorométhane puis filtré sur un lit de gel de silice en éluant avec un mélange de dichlorométhane-méthanol (99,8-0,2). On recueille 0,3g d'une mousse jaune. Point de fusion : 63°C ; RMN-¹H (DMSO-*d6*): 3,87(3H, s) ; 3,92(3H, s); 6,73(1H, d); 7,27-7,32(1H, m); 7,57-7,62(1H, m); 7,91(1H, s); 8,14(1H, d); 8,30(1H, d); 9,62(1H, d)

### Exemple 151

### Acide 2-amino-5-{[1-(3-méthoxyphényl)imidazo[1,5-a]pyridin-3-yl]carbonyl}benzoïque

A 0,650g (1,62mmoles) de 2-amino-5-{[1-(3-methoxyphényl)imidazo[1,5-a]pyridin-3-yl]carbonyl}benzoate de méthyle obtenu à l'exemple 130, en solution dans 30ml de dioxanne on ajoute 4,05ml (8,1 mmoles) d'une solution aqueuse de soude 2N. On chauffe le milieu réactionnel à 60°C pendant 2 heures puis on laisse revenir à température ambiante. On concentre sous pression réduite. Le résidu est repris dans le dichlorométhane. La phase organique est lavée avec une solution aqueuse d'acide chlorhydrique1N, séchée sur sulfate de sodium puis concentrée sous pression réduite. Le résidu est purifié par chromatographie colonne sur gel de silice en éluant avec un mélange dichlorométhane-méthanol (99-1). Les 167mg de solide orange obtenu sont salifiés sous forme de sel de sodium, 0,98 H₂O. Point de fusion : 257°C ; RMN-¹H (DMSO-*d6*) : 3,89(3H, s); 6,64(1H, d); 6,95(1H, d); 7,14-7,40(3H, m); 7,44-7,60 (2H, m); 8,22(1H, d); 8,32(1H, d); 9,12(1H, s); 9,69(1H, d)

### Exemples 152 à 161

En procédant selon la préparation décrite à l'exemple 151, on synthétise les composés de formule générale Ie décrits dans le tableau IX ci dessous, par saponification de la fonction ester présente sur les substituants R₂ ou R₃ des composés de formule Ib

**TABLEAU IX**

| Ex. | R | R₁ | R₂ | R₃ | Sel | Point de Fusion (°C) ou M.S. |
|---|---|---|---|---|---|---|
| 152 | H | 2-méthoxyphényl | CO₂H | NH₂ | Na, 1.15H₂O | 297 |
| 153 | H | 2-thiényl | CO₂H | NH₂ | Na, 0.8 H₂O | 265 |
| 154 | H | 3-pyridinyl | CO₂H | NH₂ | Na, 2.5 H₂O | 300 |
| 155 | H | 4-méthoxyphényl | CO₂H | NHCOPh | - | MH+ = 492 |
| 156 | H | H | CO₂H | NH₂ | Na, 0.55 H₂O | 366 |
| 157 | H | 3-furyl | CO₂H | NH₂ | Na, 1.14H₂O | 296 |
| 158 | H | 4-fluorophényl | CO₂H | NH₂ | Na, 2,4 H₂O | 305 |
| 159 | H | 4-méthoxypyridin-3-yl | CO₂H | NH₂ | Na, 1.47 H₂O | 292 |
| 160 | H | 4-chlorophényl | CO₂H | NH₂ | Na, 1.47 H₂O | 338 |
| 161 | 6-NHMe | H | CO₂H | NH₂ | Na, 1.2 H₂O | 270 |

Les RMN des exemples 152 à 161 du TABLEAU IX sont présentées dans le tableau IX' ci-dessous :

**TABLEAU IX'**

| Ex. | RMN-¹H (DMSO-d6) |
|---|---|
| 152 | 3,85(3H, s); 6,65(1H, d); 7,10-7,25(4H, m); 7,3(1H,t); 7,65-7,75(2H, m); 8,30(1H, d); 8,92(1H, s); 9,63(1H, d) |
| 153 | 6,76(1H, d); 7,09-7,35(3, m); 7,45(1H, d); 7,66(1H, d); 8,16(1H, d); 8,43(1H, d); 9,13(1H, s); 9,72(1H, d) |
| 154 | 6,79(1H, d); 7,23-7,56(2H, m) ; 7,56-7,58(1H, m) ; 8,31-8,42(3H, m) ; 8,58-8,60 (1H, m) ; 9,23-9,26(2H, m) ; 9,73(1H, d) |
| 155 | 3,85(3H, s); 7,12(2H, d); 7,15-7,30(1H, m); 7,43-7,49(1H, m); 7,60-7,65(3H, m); 8,00(2H, d); 8,11(2H, d); 8,28(1H, d); 8,62(1H, d); 8,87(1H, d); 9,26(1H, s);9,82(1H,d) |
| 156 | 6,59(1H, d); 7,08-7,10(1H, m); 7,11-7,25(1H, m); 7,76(1H, s); 7,88(1H, d); 8,24 (1H, d); 8,97(1H, s); 9,63(1H, d) |
| 157 | 6,68(1H, d); 7,13-7,37(3H, m); 7,85(1H, s); 8,17(1H, d); 8,36(1H, d); 8,41(1H, s); 9,19(1H, s); 9,77(1H, d) |
| 158 | 6,60-7,39(2H, m); 7,16-7,39(2H, m); 7,36(1H, m); 8,03-8,33(2H, m); 8,06(1H, m); 8,19-9,69(2H, m), 9,01(1H, s) |
| 159 | 3,95(3H, s); 6,60-7,01(2H, m); 7,16-7,34(2H, m); 8,20-8,35(2H, m), 8,20-9,70 (2H, m); 8,81(1H, s); 9,08(1H, s) |
| 160 | 6,61-8,04(2H, m); 7,17-7,56(2H, m); 7,56-8,01(4H, m); 8,32-8,33(1H, m); 9,04 (1H, s); 9,66-9,69(1H, m) |
| 161 | 2,74(3H,d); 6,58(1H,d); 6,90(1H,d); 7,61(1H,s); 7,67(1H,d); 8,24(1H,d); 8,98 (1H,d) |

### Exemple 162

### Acide 2-amino-5-{[1-(4-méthoxyphényl)imidazo[1,5-a]pyridin-3-yl]carbonyl}benzoïque

A 0,259g (0,5mmole) de l'acide 2-benzoylamino-5-{[1-(4-méthoxyphényl)imidazo[1,5-a]pyridin-3-yl]carbonyl}benzoïque obtenu à l'exemple 155, en solution dans 25ml de dioxanne, on ajoute 1,2g de soude en pastilles. On chauffe à reflux pendant 48 heures. On laisse le milieu réactionnel revenir à température ambiante. Le milieu est repris avec du dioxanne puis acidifié avec de l'hydrogénosulfate de potassium. Le précipité formé est filtré puis rincé à l'eau et séché. On recueille 0.162g d'un solide jaune qui est salifié sous forme de sel de sodium, 1,15 H₂O. Point de fusion : 296°C ; RMN-¹H (DMSO-*d6*) *:* 3,85(3H, s); 6,63(1H, d); 7,09-7,17(3H, m); 7,28-7,35(1H, m); 7,96(2H, d); 8,17(1H, d); 8,34(1H, d); 9,04(1H, s); 9,69(1H, d)

### Exemple 163

### Acide 3-(4-amino-3-méthoxybenzoyl)imidazo[1,5-a]pyridine-1-carboxylique

A 0,272g (0,84 mmole) de 3-(4-amino-3-méthoxybenzoyl)imidazo[1,5-a]pyridine-1-carboxylate de méthyle obtenu à l'exemple 150, dans 12ml d'un mélange (1/1) de dioxanne et de méthanol, on ajoute 1,67ml (1,67 mmole) d'une solution aqueuse de soude 1N. Après 2 heures de chauffage à reflux, le milieu réactionnel est concentré sous pression réduite. Le résidu est repris dans l'eau puis acidifié avec 0,239g (1,7 mmole) d'hydrogénosulfate de potassium. Le précipité formé est filtré lavé à l'eau, l'éther éthylique, séché. On recueille 0.22g d'un solide orange qui est salifié sous forme d'un sel de sodium, 2,55 H₂O. Point de fusion : 226°C ; RMN-¹H (DMSO-*d6*) : 3,86(3H, s); 6,71(1H, d); 7,06-7,11(1H, m); 7,24-7,30(1H, m); 7,89(1H, s); 8,34(1H, d); 8,59(1H, d); 9,63(1H, d)

### Exemple 164

### Acide 3-(4-amino-3-méthoxybenzoyl)imidazo[1,5-a]pyridine-6-carboxylique

A 300mg (0.92 mmole) de 3-(4-amino-3-méthoxybenzoyl)imidazo[1,5-a]pyridine-6-carboxylate de méthyle obtenu à l'exemple 83, dans 15ml d'un mélange (5/5/5) de dioxanne, dichlorométhane et de méthanol, on ajoute 1,51mil (1,51 mmole) d'une solution aqueuse de soude 1N. Le milieu réactionnel est agité une nuit à température ambiante puis concentré sous pression réduite. Le résidu est dissout dans l'eau, lave à l'acétate d'éthyle puis acidifie la phase aqueuse avec 1,5ml d'acide chlorhydrique 1N. Le précipité formé est filtré, lavé à l'eau puis séché. On recueille 346mg d'un solide jaune qui est salifié sous forme de sel de sodium, 0,7H₂O. Point de fusion : 306°C ; RMN-¹H (DMSO-*d6*) : 3,87(3H, s); 6,72(1H, d); 7,70-7,78(3H, m); 7,96(1H, s); 8,18(1H, d); 10,09(1H, s)

### Exemples 165 à 181

En procédant selon la préparation décrite à l'exemple 164, on synthétise les composés de formule générale Id décrits dans le tableau X ci dessous par saponification de la fonction ester présente sur le substituant R.

**TABLEAUX**

| Ex. | R | R₁ | R₂ | R₃ | Sel | Point de Fusion (°C) |
|---|---|---|---|---|---|---|
| 165 | 8-CO₂H | H | OMe | NH₂ | Na | 259 |
| 166 | 7- CO₂H | H | OMe | NH₂ | 1.2HCl 0.95 H₂O | 268 |
| 167 | 7- CO₂H | 3-OMe-Ph | OMe | NH₂ | Na, 2.7 H₂O | 307 |
| 168 | 7- CO₂H | 4-méthoxy pyridin-3-yl | OMe | NH₂ | Na | 295 |
| 169 | 7-CONHCH₂CO₂H | H | OMe | NH₂ | Na, 1,9H₂O | 287 |
| 170 | 7-OCH₂CO₂H | H | OMe | NH₂ | Na, 095 H₂O | 302 |
| 171 | (R)-7-CONHCH(CH₃)CO₂H | H | OMe | NH₂ | Na, 1.5 H₂O | 77 |
| 172 | (S)-7-CONHCH(CH₃)CO₂H | H | OMe | NH₂ | Na, 2.7 H₂O | 261 |
| 173 | 8-OCH₂CO₂H | H | OMe | NH₂ | Na, 2 H₂O | 313 |
| 174 | 7-CO₂H | H | OMe | NHSO₂Me | Na, 2 H₂O | 321 |
| 175 | 8-O(CH₂)₃-CO₂H | H | OMe | NH₂ | Na, 1.6 H₂O | 199 |
| 176 | (S)-7-CONHCH(CH₂OH)CO₂H | H | OMe | NH₂ | Na, 2.2 H₂O | 244 |
| 177 | 6-CONHCH₂CO₂H | H | OMe | NH₂ | Na, 1.5 H₂O | 202 |
| 178 | (S)-6-CONHCH(Bn)CO₂H | H | OMe | NH₂ | Na, 1.2 H₂O | 268 |
| 179 | 6-CONHCH₂CH₂CO₂H | H | OMe | NH₂ | Na, 2.5 H₂O | 271 |
| 180 | (S)-6-CONHCH(Me)CO₂H | H | OMe | NH₂ | Na, 2.35 H₂O | 221 |
| 181 | (S)-6-CONHCH(CH₂OH)CO₂H | H | OMe | NH₂ | Na, 1 H₂O | 244 |

Les RMN des exemples 165 à 181 du TABLEAU X sont présentées dans le tableau X' ci-dessous :

**TABLEAU X'**

| Ex. | RMN-¹H (DMSO-*d6*) |
|---|---|
| 165 | 3,88(3H, s); 6,70(1H, d); 7,12(1H, t); 7,65(1H, d); 7,91(1H, s); 8,20 (1H, d); 8,33(1H, s); 9,70(1H, d) |
| 166 | 3,87(3H, s); 6,72(1H, d); 7,46(1H, d); 7,93-8,06(2H, m); 8,21(1H, d); 8,53 (1H, s); 9,60(1H, d) |
| 167 | 3,87(3H, s); 3,92(3H, s); 5,79(2H, m); 6,74-7,54(2H, m); 6,98-7,65(2H, m) 7,47-7,62(2H, m); 8,14-9,66(2H, m); 8,24(1H, s); 8,51(1H, s) |
| 168 | 3,90(3H, s); 3,95(3H, s); 5,77(2H, m); 6,72-7,05(2H, m); 7,60-8,21(2H, m); 8,13(1H, s); 8,24-9,61(2H, m); 8,41(1H, s); 8,74(1H, s) |
| 169 | 3,52-3,54(2H, d); 3,88(3H, s); 5, 84(2H, m); 6,71-9,62(2H, m); 7,45-8,24(2H, m); 7,96-7,97(2H, m); 8,12-8,16(1H, t); 8,41(1H, s) |
| 170 | 3,85(3H, s); 4,20(2H, s); 5,62-5,64(2H, s); 6,67-9,59(2H, m); 6,82-8,18(2H, m); 6,93(1H, m); 7,49(1H, s); 7,95(1H, s) |
| 171 | 1,42-1,44(3H, d); 3,87(3H, s); 4,40-4,50(1H, m); 5,85(2H, s); 6,70-9,63(2H, m); 7,50-8,23(2H, m); 7,96(1H, s); 8,02(1H, s); 8,48(1H, s); 8,91-8,94(1H, d) |
| 172 | 1,17-1,21(3H, d); 3,87-4,04(4H, m); 5,72(2H, s),; 6,62-9,60(2H, m); 7,42-8,29(2H, m); 7,95-7,97(2H, m); 8,38-8,42(1H, d); 8,84(1H, s) |
| 173 | 3,87(3H, s); 4,36(2H, s); 5,76(2H, s); 6,4051H, m); 6,69-8,26(2H, m); 6,96-7,02(1H, t); 7,77(1H, s); 7,92(1H, m); 9,22(1H, m) |
| 174 | 3,07(3H, s); 3,92(3H, s); 7,44-9,65(2H, m); 7,65-8,13(2H, m); 7,93(1H, s); 8,09(1H, m); 8,31(1H, m) |
| 175 | 1,94-1,98(2H, m); 2,02-2,08(2H, m); 3,85(3H, s); 4,21-4,23(2H, m); 5,76-5,78 (2H, m); 6,87-8,21(2H, m); 6,74(1H, m); 6,99-7,02(1H, t); 7,74(1H, s); 7,89 (1H, s); 8,21(1H, m); 9,23(1H, m) |
| 176 | 3,44-3,51(2H, m); 3,60-3,72(2H, m); 3,87(3H, s); 3,97-4, (2H, m); 5,84(2H, s); 5,90-5,92(1H, m); 6,70-9,63(2H, m); 7,45-8,23(2H, m); 7,96-7,98(2H, m)8,19-8,20(1H, m) |
| 177 | 3,56(2H,d); 3,88(3H,s); 6,73(1H,d); 7,60(1H,d); 7,83(1H,s); 7,92-7,96(2H,m); 8,24(1H,d); 10,10(1H,s) |
| 178 | 3,04-3,27(2H,m); 3,86(3H,s); 4,19-4,22(1H,m); 6,71(1H,d); 7,10-7,19(5H,m); 7,44(1H,d); 7,81(1H,s); 7,89-7,94(4H,m); 8,24(1H,d); 10,05(1H,s) |
| 179 | 2,18(2H,t); 3,42(2H,m); 3,79(3H,s); 6,71(1H,d); 7,56(1H,d); 7,83(1H,s); 7,93-7,95(2H,m); 8,23(1H,d); 10,10(1H,s) |
| 180 | 3,52-3,59(1H,m); 3,68-3,74(1H,m); 3,87(3H,s); 4,04-4,11(1H,m); 6,72(1H,d); 7,60(1H,d); 7,84(1H,s); 7,93-7,97(2H,m); 10,12(1H,s) |
| 181 | 1,31(3H,d); 3,87(3H,s); 3,92(1H,q); 6,71(1H,d); 7,54(1H,d); 7,74(1H,s); 7,92-7,95(2H,m);8,24(1H,d); 10,10(1H,s) |

### Exemple182

### Acide 3-(3-méthoxy-4-nitrobenzoyl)imidazo[1,5-a]pyridine-6-carboxylique

A 530mg (1,49 mmole) de 3-(3-méthoxy-4-nitrobenzoyl)imidazo[1,5-a]pyridine-6-carboxylate de méthyle obtenu à l'exemple 4, dans un mélange de 20ml de dioxane et de 10ml de méthanol, on ajoute 1,64ml (1,64 mmole) d'une solution aqueuse de soude 1N. Le milieu réactionnel est chauffé à 60°C pendant 3 heures puis concentré sous pression réduite. Le résidu est repris à l'eau et la phase aqueuse obtenue est lavée au dichlorométhane puis neutralisée par ajout de 1,64ml d'acide chlorhydrique 1N. Le précipité formé est filtré, lavé à l'eau puis séché. On recueille 405mg d'un solide jaune. Point de fusion : 313°C ; RMN-¹H (DMSO-*d6*) : 4,01(3H, s) ; 7,78(1H, d); 7,98(1H, s); 8,06-8,10(3H, m); 8,15(1H, s); 10,28(1H, s)

### Exemples 183 à 184

En procédant selon la préparation décrite à l'exemple 182, on synthétise les composés de formule générale It décrits dans le tableau XI ci dessous par saponification de la fonction ester présente sur le substituant R.

**TABLEAU XI**

| Ex. | R | R₁ | R₂ | R₃ | Sel | Point de Fusion (°C) |
|---|---|---|---|---|---|---|
| 183 | 8-CO₂H | H | OMe | NO₂ | Na | 332 |
| 184 | 7- CO₂H | H | OMe | NO₂ | - | >350 |

Les RMN des exemples 183 à 184 du TABLEAU XI sont présentées dans le TABLEAU XI' ci-dessous:

**TABLEAU XI'**

| Ex. | RMN-¹H (DMSO-*d6*) |
|---|---|
| 183 | 4,00(3H, s); 7,32(1H, t); 7,82(1H, d); 8,03-8,08(3H, m); 8,47(1H, s); 9,75(1H, d) |
| 184 | 4.02(3H,s); 7.75 (1H,m); 7.78(1H,s); 7.96(2H,m); 8.1(1H,s); 8.30(1H,m); 9.67(1H,m) |

### Exemple 185

### (4-amino-3-méthoxyphényt)(8-méthoxyimidazo[1,5-a]pyridin-3-yl)méthanone

A 0.22g (0.76 mmole) du (4-amino-3-méthoxyphényl)(8-hydroxyimidazo[1,5-a]pyridin-3-yl)méthanone obtenu à l'exemple 147, dans 5 ml de DMF on ajoute 0.79g (2,43mmoles) de carbonate de césium puis 0.05ml (0.84mmole) de iodure de méthyle. Le milieu réactionnel est agité à température ambiante pendant 4 heures. Après ajout d'une solution saturée en hydrogenocarbonate de sodium, le milieu réactionnel est extrait à l'acétate d'éthyle. La phase organique obtenue est séchée sur sulfate de sodium, concentrée sous pression réduite. Le résidu est purifié par chromatographie colonne sur gel de silice en éluant au dichlorométhane. On recueille 0.2g d'un solide jaune orangé. Point de fusion : 229°C ; RMN-¹H (CDCl₃) : 3,86(3H, s); 4,01(3H, s); 5,80(2H, m); 6,69(1H, m); 6,69-6,72(1H, m); 7,03-7,09(1H, t); 7,78(1H, s); 7,92(1H, s); 8,24(1H, m); 9,25-9,28(1H, m)

### Exemples 186 à 189

En procédant selon la préparation décrite à l'exemple 185, on synthétise les composés de formule générale Iz' décrits dans le tableau XII ci-dessous par O-alkylation des composés de formule générale Iz en présence d'un carbonate alcalin et de l'halogénure correspondant:

**TABLEAU XII**

| Ex. | R | R₁ | R₂ | R₃ | Sel | Point de Fusion (°C) |
|---|---|---|---|---|---|---|
| 186 | 7-OCH₂CO₂Et | H | OMe | NH₂ | HCl | 187 |
| 187 | 8-OCH₂CO₂ | H | OMe | NH₂ | HCl | 216 |
| 188 | 8-0(CH₂)₃-CO₂Et | H | OMe | NH₂ | 0.9HCl | 174 |
| 189 | 8-OCH₂CH₂NMe₂ | H | OMe | NH₂ | 2HCl, 3.25H₂O | 142 |

Les RMN des exemples 186 à 189 du TABLEAU XII sont présentées dans le tableau XII' ci-dessous :

**TABLEAU XII'**

| Ex. | RMN-¹H (DMSO-*d6*) |
|---|---|
| 186 | 1,31-1,36(3H, t); 4,28-4,35(2H, q); 4,49(2H, s); 6,74-9,69(4H, m); 7,50(2H, m); 7,94(1H, s); 8,21(1H, m) |
| 187 | 1,21-1,27(3H, t); 3,89(3H, s); 4,17-4,26(2H, q); 5,08-5,11(2H,m); 6,69-8,22(2H, m); 6,86-6,89(1H, m); 7,03-7,09(1H, t); 7,21(2H, m); 7,82(1H, s); 7,93(1H, m); 9,28(1H, m) |
| 188 | 1,10-1,22(3H, t); 2,05-2,16(2H, m); 2,53-2,59(2H, m); 3, 89(3H, s); 4,05-4,13(2H, q); 4,24-4,29(2H, t); 5,44(2H, m); 6,72-8,21(2H, m); 6,85-6,88(1H, m); 7,04-7,10(1H, t); 7,79(1H, s); 7,92(1H, m); 9,25-9,28(1H, m) |
| 189 | 2,85-2,91(6H, d); 3,63-3,64(2H, m); 3,89(3H, s); 4,47(2H, m); 4,61-4,76(2H, m); 6,79-8,20(2H, m); 6,84-6,88(1H, m); 7,08-7,14(1H, t); 7,95(1H, m); 8,05(1H, s); 9,29-9,32(1H, m) |

### Exemple 190

### 3-(3-{3-méthoxy-4-[(propylsulfonyl)aminobenzoyl}imidazo[1,5-a]pyridi-1-yl)benzoate de méthyle

### A 0.5g (1.24mmole) de 3-[3-(4-amino-3-méthoxybenzoyl)imidazo[1,5-a]pyridin-1-yl]benzoate de méthyle obtenu à l'exemple 111 dans 6ml de pyridine on ajoute 0.17ml (1.5mmole) de chlorure de 1-propanesulfonyle. Le milieu réactionnel est agité à température ambiante pendant 18h puis concentré sous pression réduite. Le résidu obtenu est repris au dichlorométhane. La phase organique obtenue est lavée à l'eau, séchée sur sulfate de sodium, concentrée sous pression réduite. Le résidu obtenu est purifié par chromatographie colonne sur gel de silice en éluant au dichlorométhane. On recueille 0.34g d'une huile jaune. MS+ : 508 ; RMN-¹H (DMSO-d6) : 0,96-1,01(3H, t); 1,74-1,81(2H, m); 3,16-3,19(2H, m); 3,92(3H, s); 4,00(3H, s); 7,33-7,73(2H, m; 7,38-7,51(1H, m); 7,53-7,96(2H, m); 8,03-8,30(2H, m); 8,33-9,85(2H, m); 8,38(1H, s); 8,63(1H, s); 9,25(1H, m)

### Exemples 191 à 194

En procédant selon la préparation décrite à l'exemple 190, on synthétise les composés de formule générale Ig décrits dans le tableau XIII ci-dessous par sulfonylation ou acylation des composés de formule générale Id:

**TABLEAU XIII**

| Ex. | R | R₁ | R₂ | R₃ | Point de Fusion (°C) |
|---|---|---|---|---|---|
| 191 | H | Phényl-3-CO₂Et | OMe | NHCOCH₂CH₃ | MH+=458 |
| 192 | H | H | OMe | NHSO₂Me | 78 |
| 193 | 7-CO₂Et | H | OMe | NHSO₂Me | 187 |
| 194 | H | Br | OMe | NHSO₂Me | 206 |

Les RMN des exemples 191 à 194 du TABLEAU XIII sont présentées dans le tableau XIII' ci-dessous :

**TABLEAU XIII'**

| Ex. | RMN-¹H (DMSO-*d6*) |
|---|---|
| 191 | (CDC13) : 1,24-1,29(3H, t); 2,43-2,50(2H, m); 3,91(3H, s); 3,96(3H, s); 6,28-7,18(2H, m); 7,40-7,46(1H, m); 7,88-8,02(4H, m); 8,20-9,75(2H, m); 8,32(1H, s); 8,45-8,55(2H, m) |
| 192 | 3,12(3H, s); 3,94(3H, s); 7,4-7,42(2H, m); 7,48-8,03(2H, m); 7,90(1H, s); 8,00-8,03(2H, m); 8,14-9,73(2H, m) |
| 193 | 1,35-1,40(3H, t); 3,12(3H, s); 3,93(3H, s); 4,35-4,43(2H, q); 7,49-8,15(2H, m); 7,56-9,66(2H, m); 8,02(1H, s); 8,10(1H, s); 8,64(1H, s); 9,26(1H, s) |
| 194 | 3,12(3H, s); 7,34-7,56(2H, m); 7,53-7,56(1H, m); 7,84-7,85(1H, m); 7,88(1H, s); 7,91-7,92(1H, m); 9,26(1H, s); 9,72-9,73(1H, m) |

### Exemple 195

### Acide 3-(3-{3-méthoxy-4-[(propylsulfonyl)amino]benzoyl}imidazo[1,5-a]pyridin-1-yl)benzoïque

A 0.34g (0.7mmole) de 3-(3-{3-méthoxy-4-[(propylsulfonyl)amino]benzoyl}imidazo [1,5-a]pyridin-1-yl)benzoate de méthyle obtenu à l'exemple 190 dans 20ml de méthanol on ajoute 1.4mil d'une solution aqueuse 1N de soude. Le milieu réactionnel est chauffé à 70°C pendant 3 heures puis concentré sous pression réduite. Le résidu est repris à l'eau et la phase aqueuse obtenue est lavée au dichlorométhane, neutralisée par ajout de 1,4m1 d'acide chlorhydrique 1N puis extraite au dichlorométhane. La phase organique obtenue est séchée sur sulfate de sodium, concentrée sous pression réduite. On recueille 0.19g d'un solide jaune qui est salifié sous forme de sel de sodium, 2,1 H₂O. Point de fusion : 145°C ; RMN-¹H (DMSO-*d6*) : 0,96-1,01(3H, t); 1,71-1,83(2H, m); 3,16-3,19(2H, m); 4,00(3H, s); 7,33-7,70(2H, m); 7,36-7,38(1H, m); 7,52-7,97(2H, m); 8,04-8,07(2H, m); 8,27-9,85(2H, m); 8,44(1H, s); 8,64(1H, s); 9,25(1H, m)

### Exemple 196

### Acide 3-{3-[3-méthoxy-4-[propionylamino]benzoyl]imidazo[1,5-a]pyridin-1-yl} benzoïque

Ce composé est obtenu en procédant selon la préparation décrite dans l'exemple 195 par saponification du 3-{3-[3-méthoxy-4-[propionylamino]benzoyl]imidazo[1,5-a]pyridin-1-yl} benzoate de méthyle obtenu à l'exemple 191 avec de la soude 1N. On recueille un solide jaune qui est salifié sous forme de sel de sodium, 2.4 H₂O. Point de fusion : 145°C ; RMN-¹H (DMSO-*d6*) : 1,08-1,24(3H, t); 2,46-2,51(2H, m); 4,03(3H, s); 7,30-7,55(3H, m); 7,88-8,38(6H, m); 8,43(1H, m); 8, 60(1H, m); 9,40(1H, m); 9,45(1H, m); 9,95(1H, m)

### Exemple 197

### Etude de la liaison ¹²⁵I - b-FGF au Récepteur purifié FGF R α IIIc par méthode de scintillation de proximité

Des plaques NBS (NBS plate 96 well solid white CORNING 3600) sont coatées avec 100 µl de gélatine 0.1 % par puit, 2 heures à 37°C. Au terme de l'incubation, on élimine le coating, on rinçe et on sèche bien les plaques. On distribue 100 µl de tampon de binding (Tampon Bis Tris 40 mM pH 7.0) dans les plaques.

Les dilutions des composés de l'invention sont réparties dans les puits à raison de 10 µl / puit. On distribue ensuite 10 µl / puit de b-FGF (AMERSHAM ARM 35050) et 10 µl / puit de FGF R α III c (R&D Systems 658 FR). On ajoute après 10 µl / puit de ¹²⁵I - bFGF (Dupont NEN NEX 268 - Activité spécifique > 70 µCi) et 50 µl / puit des billes SPA (AMERSHAM RPQN 00019). On agite quelques secondes la plaque et on l'incube 60 minutes à 37°C et à l'abri de la lumière.

Au terme de l'incubation, la plaque est lue dans un compteur de radioactivité MIBROBETA TRILUX (: WALLAC - PERKINELMER)

Les composés de l'invention ont démontré une activité spécifique comprise entre 10⁻⁷M et 10⁻⁹M.

### Exemple 198

### Effets des composés de la formule I sur la prolifération d'HUVECs versus 30 ng/ml de b-FGF ou 10 ng/ml de a-FGF

-Coater les plaques 24 puits (FALCON PRIMARIA) avec 200 µl d'une solution de fibronectine (50 µg / ml préparée dans du PBS) / puits.

Ensemencer à raison de 30000 cellules / ml / puit dans un milieu RPMI 1640 + 10 % SVF + 1% Glutamine + mélange Héparine-ECGF (HE).

Incuber à 37 °C, 5 % CO2 le temps que les cellules adhèrent.

Dissoudre les produits et préparer des solutions dans le DMSO / milieu réactionnel ayant une concentration finale de 1 µM final à 10⁻⁷M.

Après adhésion des cellules pendant 6 heures à 37°C en présence de 5% CO₂, le milieu est remplacé par du RPMI 1640 0.1 % SVF + Glutamine +HE.

Pour la dérivatisation, on utilise comme contrôle négatif 0.1 % SVF, comme contrôle positif 0 % SVF et comme témoin 0.1 % SVF + 30 ng/ml de b-FGF ou 10 ng/ml de a-FGF. On incube ensuite 24 heures à 37 °C en présence de 5%CO₂.

Le deuxième jour les cellules sont rincées par 1 ml de PBS et 200µl de trypsine, puis elles sont récupérées dans l'isoton. On procède à la numération (n> 9µm)

Dans ce test de prolifération des cellules endothéliales induite par le b-FGF ou le a-FGF, les composés de l'invention ont démontré une activité spécifique comprise entre 10⁻⁵M et 10⁻⁹M.

### Exemple 199

### MODELE D'ANGIOGENESE IN-VITRO

Préparer les gels en distribuant dans chaque puits de chamberslide (Biocoat Cellware rat tail collagen, Type I, 8-well culturesides: Becton Dickinson 354630) 160 µl de matrigel dilué au 1/6 (Growth factor reduced Matrigel: Becton Dickinson 356230) dans du collagen (Rat Tail Collagène, type I: Becton Dickinson 354236). Laisser gélifier pendant 1 heure à 37°C.

Ensemencer les cellules endothéliales veineuses humaines (HUVEC ref:C-015-10C - cascade Biologics, INC) ou artérielles de porc (PAEC) à 15.10³ cellules/puits dans 400 µl de milieu EBM (Clonetics C3121) + 2% FBS + hEGF 10 µg/ml pour les HUVEC et DMEM + 3% SVF + Glutamine 2 mM + Sodium pyruvate 1 mM + Acides Aminés Non Essentiels 1% (GIBCO) pour les PAEC.

Stimuler avec le b-FGF (TEBU/Peprotech) 10 ng/ml ou le a-FGF (TEBU/Peprotech) 10 ng/ml en présence ou non des produits de l'invention pendant 24h à 37°C en présence de 5% CO₂.

Après 24 heures, fixer les cellules et colorer la lame au trichrome de Masson avant observation au microscope objectif X4 et analyse d'image (BIOCOM- logiciel Visiolab 2000).

Pour le test d'angiogénèse in vitro induite par le b-FGF ou le a-FGF, les composés de l'invention ont démontré une activité spécifique comprise entre 10⁻⁷M et 10⁻¹¹M.

### Exemple 200

### Modèle d'angiogenèse inflammatoire chez la souris

L'angiogenèse est requise pour le développement des maladies inflammatoires chroniques comme l'arthrite rhumatoïde, les IBD, mais aussi pour le développement des tumeurs solides. La formation de nouveaux vaisseaux permet non seulement la perfusion des tissus pathologiques mais également le transport de cytokines responsables de l'établissement de la chronicité de la maladie.

Le modèle décrit par Colville-Nash P et al (D. JPET., 1995, vol 274 n°3, pp1463-1472) permet d'étudier des agents pharmacologiques susceptibles de moduler l'apparition de l'angiogenèse.

Les animaux, souris blanches non consanguines de 25g environ sont anesthésiés avec du pentobarbital sodique (60 mg/kg ; Sanofi Nutrition Santé animale) par voie intrapéritonéale.

Une poche d'air est créée sur le dos de la souris par injection de 3 ml d'air en sous-cutanée.

Après le réveil, les animaux reçoivent un traitement en général par gavage et reçoivent une injection de 0.5 ml d'adjuvant de Freund (Sigma) avec 0.1 % d'huile de croton (Sigma) dans la poche.

Sept jours après, les souris sont à nouveau anesthésiées et placées sur une plaque chauffante à 40°C. Un ml de rouge carmin (5% dans 10 % de gélatine - Aldrich Chemicals) est injecté à la veine de la queue. Les animaux sont ensuite mis à 4°C pendant 2-3 heures.

Les peaux sont ensuite prélevées et mises à sécher pendant 48 heures dans une étuve à 56°C. Les tissus secs sont pesés et mis dans 1.8 ml de tampon digestif (dithiothreitol 2 mM, Na₂HPO₄ 20mM, EDTA 1 mM, papaïne 12U/ml) pendant 24 heures.

Le colorant est alors dissous dans 0.2 ml de NaOH 5M. Les peaux sont centrifugées à 2000g pendant 10 mn. Les surnageants sont filtrés sur membranes d'acétate de cellulose 0.2 µm. Les filtrats sont lus dans un spectrophotomètre à 492 nm. contre une gamme étalon de rouge carmin.

Deux paramètres sont étudiés : le poids sec du granulome et la quantité de colorant après digestion des tissus.

Les résultats sont exprimés en valeurs moyennes (± sem). Les différences entre les groupes sont testées avec une ANOVA suivie d'un test de Dunnet dont le groupe de référence est le groupe "témoin solvant".

Les composés de l'invention sont actifs par voie orale à des doses de 0.1 à 30 mg/kg.

### Exemple 201

### Modèle d'angiogenèse MATRIGEL chez la souris

Le modèle décrit par Passaniti and al. (Laboratory Investigation (1992) 67 (4) pp519-524) permet d'étudier des agents pharmacologiques susceptibles de moduler l'apparition de l'angiogenèse spécifiquement induite par le bFGF. A du Matrigel (Beckton Dickinson) maintenu sous forme liquide à 4°C est ajouté du FGF2 (Peprotecch) à raison de 300 ng/ml. Après homogénéisation, le mélange (0.5ml) est injecté par voie sous-cutanée dans le bas du dos de souris noires femelles(C57/B16) de 20g environ préalablement anesthésiées avec du pentobarbital sodique (60 mg/kg ; Sanofi Nutrition Santé animale) par voie intrapéritonéale. Les animaux sont traités par gavage. Après 5 jours les souris sont à nouveau anesthésiées et la peau du bas du dos est prélevée, à ce stade, les différences qualitatives de vascularisation du granulome sont évaluées (scorées) et les granulomes sont photographiés. Un dosage de DNA dans les granulomes est ensuite effectué pour quantifier la cellularité de celui-ci. Pour cela, les granulomes isolés sont digérés par de la collagénase (3mg/ml) toute une nuit à 37°C. Après centrifugation à 850 g durant 10 min, le surnageant est écarté et le culot est remis en solution dans 1.2 ml de tampon PBS contenant 1 mM CaCl₂, 1 mM MgCl₂ et 5 mM de glucose. La quantité de DNA présente est mesurée à l'aide d'un kit (Cyquant-GR®, Molecular probe) suivant les instructions du fournisseur.

Les résultats sont exprimés en valeurs moyennes (± sem). Les différences entre les groupes sont testées avec une ANOVA suivie d'un test de Dunnet dont le groupe de référence est le groupe "témoin solvant".

Pour les études histologiques, les granulomes sont prélevés avec le muscle et la peau, fixés une nuit dans une solution de formaldéhyde à 10% et inclus dans de la paraffine (Embedder Leica®). Les granulomes sont ensuite coupés à l'aide d'un microtome (Leica) et colorés avec le colorant Trichrome de Mason. La néo vascularisation des granulomes est alors évaluée. Les niveaux de vascularisation sont compris entre une valeur 0 et 5.

Les composés de l'invention sont actifs par voie orale à des doses de 0.1 à 30 mg/kg.

### Exemple 202

### Modèle d'angiogenèse tumorale chez la souris

Ce modèle permet d'étudier des agents pharmacologiques susceptibles de moduler l'apparition de l'angiogenèse spécifiquement induite par le développement tumoral. Des souris C56/B16 de 20g environ sont anesthésiées avec du pentobarbital sodique (60 mg/kg ; Sanofi Nutrition Santé animale) par voie intrapéritonéale. Les tumeurs sont établies par injection sous cutanée sur le dos de cellules Lewis Lung de souris à raison de 2 10⁵ cellules/souris. Après 5 jours, les souris sont traitées tous les jours par gavage. La taille des tumeurs est mesurée 2 fois par semaine durant 21 jours et le volume tumoral est calculé en utilisant la formule : [π/6(ω₁ x ω₂ x ω₂)], où ω₁ représente le plus grand diamètre et ω₂ représente le plus petit diamètre.

Les résultats sont exprimés en valeurs moyennes (± sem). Les différences entre les groupes sont testées avec une ANOVA suivie d'un test de Dunnet dont le groupe de référence est le groupe "témoin solvant".

Les composés de l'invention sont actifs par voie orale à des doses de 0.1 à 30 mg/kg.

### Exemple 203

### Effet sur la thrombopénie

La thrombopénie reste une pathologie pour laquelle il existe peu de traitements efficaces à part la transfusion de concentrés plaquettaires et la thrombopoïétine (Kaushansky, K. New Eng J Med (1998), 339, pp746-754).

La chimiothérapie anti-cancéreuse constitue une des causes majeures de thrombopénie. Un des agents de chimiothérapie, le carboplatine, a été largement utilisé pour induire une thrombopénie chez la souris et pouvoir ainsi caractériser l'effet de composés capables d'améliorer le taux de plaquettes comme par exemple la thrombopoïétine (Hokom MM et al., Blood (1995), 86, pp4486-4492).

150 mg/kg de carboplatine ont été administrés par voie intrapéritonéale à des souris balbC ayant un poids de 20g. Un prélèvement sanguin est effectué périodiquement par ponction rétro-orbitale et le taux de plaquettes circulantes est déterminé par un automate d'hématologie (MS9™ de Melet-Schloesing Laboratoires, Cergy-Pontoise, France). Dans ces conditions, on observe une thrombopénie réversible avec un nadir situé 9 à 10 jours après l'administration de carboplatine (diminution du taux de plaquettes circulantes de 50-60%).

Les composés selon l'invention ou leur solvant (un blanc - témoin) sont administrés par voie orale pendant 5 jours en commençant le traitement 7 jours avant l'administration de carboplatine. Les expériences sont effectuées sur des lots comprenant 10-12 souris et les résultats sont exprimés en moyenne ± erreur standard. Dans ces conditions, les composés de l'invention augmentent le taux de plaquettes circulantes à des doses de 0.1 à 30 mg/kg.

### Exemple 204

### Modèle de CNV (choroïdal neo vascularization) induite par laser argon chez la souris

Un caractère majeur de la perte de transparence oculaire est la néo-vascularisation et les hémorragies consécutives qui causent des désordres fonctionnels importants au niveau de l'oeil et qui se traduisent par une cécité précoce. Récemment, l'étude des mécanismes impliqués dans les phénomènes de néo-vascularisation oculaire a permis de mettre en évidence l'implication de facteur pro-angiogéniques dans ces pathologies.

Le modèle de néo-angiogenèse choroïdienne induite par laser décrit par Rakic JM et al. dans Invest Ophthalmol Vis Sci. (2003) Ju1;44(7), pp3186-3193) permet d'étudier des agents pharmacologiques susceptibles de moduler la néo-vascularisation de la choroïde.

Les souris sont anesthésiées par injection intrapéritonéale d'Avertin™. Les deux pupilles sont dilatées avec une solution à 1% de tropicamide en application topique, et trois lésions sont réalisées autour du disque optique à l'aide d'un laser argon (532 nm; diamètre "spot size" 50 µm; durée 0.05 sec; 400 mW). Le disque optique est ensuite recouvert d'une lentille.

14 jours après, les souris sont sacrifiées et les yeux énucléés et fixés dans un tampon contenant 3.5% de Formalin™, enrobés dans du Tissue TeK™ (Miles Laboratories, Naperville, Illinois) et congeler dans l'azote liquide de manière à pouvoir réaliser des coupes à l'aide d'un cryostat.

La quantification de la néo-vascularisation choroïdienne a été réalisée par une étude morphométrique quantitative permettant d'évaluer l'épaisseur du réseaux de néo-vaisseaux présents au niveau de la choroïde, à l'aide d'un système d'analyse d'image assisté par un ordinateur (Olympus Micro Image version 3.0 for Windows 95/NT, Olympus Optical CO. Europe GmBH).

La néo-vascularisation est estimée par le rapport (B/C) de l'épaisseur de la couche pigmentée de la choroïde au niveau de la lésion (B) à l'épaisseur de cette même couche pigmentée dans une région adjacente à la lésion (C). Les résultats sont exprimés en valeurs moyennes (± sem). Les différences entre les groupes traités et les groupes contrôle sont testées avec une ANOVA suivi d'un test Dunnet dont le groupe de référence est le groupe "témoin solvant".

Les composés de l'invention sont actifs par voie orale à des doses de 0.1 à 30 mg/kg.

## Revendications

1. Composé de formule **I** : 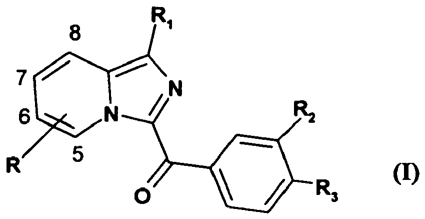 dans laquelle :
• **R**, présent sur les positions 5, 6, 7 ou 8 de l'imidazo[1,5-a]pyridine, représente un atome d'hydrogène, un atome d'halogène, un radical alkyle de 1 à 5 atomes de carbone, un radical hydroxy, un radical alcoxy de 1 à 5 atomes de carbone, un radical -COOR₆ ou un radical de formule :
• -NR₄R₅
• -NH-SO₂-Alk
• -NH-CO-Alk
• -NR₆-CO₂-Alk
• -O-Alk-COOR₆
• -O-Alk-NR₄R₅
• -O-(CH₂)ₙ-Ph
• -CO-NR₄R₅, ou
• -CO-NH-CH(R₇)-(CH₂)ₘ-COOR₆
dans lesquels :
• Alk-représente un radical alkyle ou un radical alkylène de 1 à 5 atomes de carbone,
• n représente un nombre entier de 1 à 5,
• m représente un nombre entier de 0 à 4,
• R₄ et R₅ représentent indépendamment l'un de l'autre un atome d'hydrogène, un radical alkyle de 1 à 5 atomes de carbone ou un radical benzyle,
• R₆ représente un atome d'hydrogène ou un radical alkyle de 1 à 5 atomes de carbone,
• R₇ représente un atome d'hydrogène, un radical alkyle de 1 à 5 atomes de carbone ou un radical de formule :
• -Alk-CONR₄R₅
• -Alk-OR₆
• -Alk-NR₄R₅
• -Ph, ou
• -CH₂Ph, et
• Ph représente un radical phényle éventuellement substitué par un ou plusieurs groupes choisis parmi les atomes d'halogène, les radicaux alcoxy de 1 à 5 atomes de carbone et les radicaux -COOR₆, où R₆ est tel que défini ci-dessus ;
• **R₁** représente un atome d'hydrogène, un atome d'halogène, un radical cyano, un radical -COOR₆ ou un radical de formule :
• -NR₄R₅
• -NH-SO₂-Alk
• -NH-CO-CF₃
• -NH-CO-Ph
• -NH-CO-Alk
• -NH-CO₂-Alk
• -CONR₄R₅
• un radical phényle éventuellement substitué par un ou plusieurs groupes choisis parmi les atomes d'halogène, les radicaux alkyles de 1 à 5 atomes de carbone, les radicaux alcoxy de 1 à 5 atomes de carbone et les radicaux -COOR₆,
• un radical hétéroaryle à 5 chaînons comportant un hétéroatome choisi parmi un atome de soufre, un atome d'oxygène ou un atome d'azote, et comportant éventuellement un second atome d'azote, ledit hétéroaryle étant éventuellement substitué par un ou plusieurs groupes choisis parmi les atomes d'halogène, les radicaux alkyles de 1 à 5 atomes de carbone, les radicaux alcoxy de 1 à 5 atomes de carbone et les radicaux -COOR₆, ou
• un radical hétéroaryle à 6 chaînons comportant 1 ou 2 atomes d'azote et étant éventuellement substitué par un ou plusieurs groupes choisis parmi les atomes d'halogène, les radicaux alkyles de 1 à 5 atomes de carbone, les radicaux alcoxy de 1 à 5 atomes de carbone et les radicaux -COOR₆,
dans lesquels Alk, Ph, R₄, R₅ et R₆ sont tels que définis dans ce qui précède ;
• **R₂** et **R₃** représentent indépendamment l'un de l'autre un radical hydroxy, un radical alcoxy de 1 à 5 atomes de carbone, un radical amino, un radical -COOR₆, un radical nitro ou un radical de formule :
• -NR₄R₅
• -NH-CO-Alk
• -NH-CO-Ph
• -NH-CO₂-Alk
• -NH-SO₂-Alk
• -CO-NR₄R₅, ou
• -CO-NHOH
dans lesquels Alk, Ph, R₄, R₅ et R₆ sont tels que définis dans ce qui précède ;
ou bien **R₂** et **R₃** forment ensemble, avec les atomes de carbone du noyau phényle auquel ils sont rattachés, un cycle carboné à 6 chaînons, comportant un atome d'azote et un autre hétéroatome tel que l'oxygène.
à l'état de base ou de sel, ainsi qu'à l'état d'hydrate ou de solvat.

2. Composé de formule selon la revendication 1, dans laquelle :
• **R**, présent sur les positions 6, 7 ou 8 de l'imidazo[1,5-a]pyridine, représente un atome d'hydrogène, un radical alkyle de 1 à 5 atomes de carbone, un radical alcoxy de 1 à 5 atomes de carbone, un radical hydroxy, un radical -COOR₆ ou un radical de formule :
• -NR₄R₅
• -NH-SO₂-Alk
• -NH-CO-Alk
• -NR₆-CO₂-Alk
• -O-Alk-COOR₆
• -O-Alk-NR₄R₅
• -O-CH₂-Ph
• -CO-NR₄R₅, ou
• -CO-NH-CH(R₇)-(CH₂)ₘ-COOR₆
dans lesquels Alk, Ph, R₄, R₅, R₆, R₇ et m sont tels que définis dans la revendication 1 ;
• **R₁** représente un atome d'hydrogène, un atome d'halogène, un radical cyano, un radical -COOR₆ ou un radical de formule :
• -NR₄R₅
• -NH-SO₂-Alk
• -NH-CO-CF₃
• -NH-CO-Ph
• -NH-CO-Alk
• -CO-NR₄R₅
• un radical phényle éventuellement substitué par un ou deux groupes choisis parmi les atomes d'halogène, les radicaux alcoxy de 1 à 5 atomes de carbone et les radicaux -COOR₆ ;
• un radical hétéroaryle à 5 chaînons comportant un hétéroatome choisi parmi un atome de soufre, un atome d'oxygène ou un atome d'azote, et comportant éventuellement un second atome d'azote, ledit hétéroaryle étant éventuellement substitué par un ou deux groupes choisis parmi les atomes d'halogène, les radicaux alkyles de 1 à 5 atomes de carbone, les radicaux alcoxy de 1 à 5 atomes de carbone et les radicaux -COOR₆, ou
• un radical hétéroaryle à 6 chaînons comportant 1 ou 2 atomes d'azote et étant éventuellement substitué par un ou deux groupes choisis parmi les atomes d'halogène, les radicaux alkyles de 1 à 5 atomes de carbone, les radicaux alcoxy de 1 à 5 atomes de carbone et les radicaux -COOR₆,
où Alk, Ph et R₆ sont tels que définis dans la revendication 1 ;
• **R₂** et **R₃** représentent indépendamment l'un de l'autre un radical alcoxy de 1 à 5 atomes de carbone, un radical -COOR₆, un radical amino, un radical nitro ou un radical de formule :
• - NR₄R₅
• -NH-CO-Alk
• -NH-CO-Ph
• -NH-SO₂-Alk
dans lesquels Alk, Ph, R₄, R₅ et R₆ sont tels que définis dans la revendication 1 ;
à l'état de base ou de sel, ainsi qu'à l'état d'hydrate ou de solvat.

3. Composé de formule 1 selon la revendication 1 ou 2, dans laquelle :
• R, présent sur les positions 6, 7 ou 8 de l'imidazo[1,5-a]pyridine, représente un atome d'hydrogène, un radical alcoxy de 1 à 5 atomes de carbone, un radical hydroxy, un radical -COOR₆ ou un radical de formule :
• -NR₄R₅
• -NH-SO₂-Alk
• -NH-CO-Alk
• - NR₆-CO₂-Alk
• -O-Alk-COOR₆
• -CO-NR₄R₅, ou
• -CO-NH-CH(R₇)-(CH₂)ₘ-COOR₆
dans lesquels m représente 0 ou 1, R₇ représente un atome d'hydrogène, un radical alkyle de 1 à 5 atomes de carbone ou un radical de formule -Alk-OR₆ ou -CH₂-Ph, et Alk, R₄, R₅ et R₆ sont tels que définis dans la revendication 1 ;
• **R₁** représente un atome d'hydrogène, un atome d'halogène, un radical cyano, un radical -COOR₆ ou un radical de formule :
• -NR₄R₅
• -NH-SO₂-Alk
• -NH-CO-Ph
• -NH-CO-Alk
• un radical phényle éventuellement substitué par un ou deux groupes choisis parmi les atomes d'halogène, les radicaux alcoxy de 1 à 5 atomes de carbone et les radicaux -COOR₆,
• un radical hétéroaryle choisi parmi les radicaux thiényle, furyle et pyrrolyle, ledit hétéroaryle étant éventuellement substitué par un ou deux groupes choisis parmi les radicaux alcoxy de 1 à 5 atomes de carbone et les radicaux -COOR₆, ou
• un radical pyridinyle éventuellement substitué par un ou deux groupes choisis
parmi les radicaux alcoxy de 1 à 5 atomes de carbone et les radicaux -COOR₆ ;
dans lesquels Alk, Ph, R₄ et R₆ sont tels que définis dans la revendication 1 ;
• **R₂** et **R₃** représentent indépendamment l'un de l'autre un radical alcoxy de 1 à 5 atomes de carbone, un radical -COOR₆, un radical nitro, un radical amino, ou un radical de formule NH-CO-Alk, -NH-CO-Ph ou -NH-SO₂Alk ;
dans lesquels Alk, Ph et R₆ sont tels que définis dans la revendication 1 ;
à l'état de base ou de sel, ainsi qu'à l'état d'hydrate ou de solvat.

4. Composé de formule 1 selon l'une quelconque des revendication 1 à 3, dans laquelle R₂ représente un radical alcoxy de 1 à 5 atomes de carbone ou un radical -COOR₆, où R₆ est tel que défini dans la revendication 1,
à l'état de base ou de sel, ainsi qu'à l'état d'hydrate ou de solvat.

5. Composé de formule I selon l'une quelconque des revendication 1 à 4, dans laquelle R₃ représente un radical nitro, un radical amino ou un radical de formule -NH-CO-Alk, -NH-CO-Ph ou -NH-SO₂Alk, où Alk et Ph sont tels que définis dans la revendication 1,
à l'état de base ou de sel, ainsi qu'à l'état d'hydrate ou de solvat.

6. Composé de formule I selon l'une quelconque des revendication 1 à 5, dans laquelle :
• **R**, présent sur les positions 6, 7 ou 8 de l'imidazo[1,5-a]pyridine, représente un atome d'hydrogène, un radical hydroxy, un radical -COOR₆ ou un radical de formule :
• -O-Alk-COOR₆
• -CO-NR₄R₅, ou
• -CO-NH-CH(R₇)-COOR₆
dans lesquels R₇ représente un atome d'hydrogène, un radical alkyle de 1 à 5 atomes de carbone ou un radical de formule -Alk-OR₆, et Alk, R₄, R₅ et R₆ sont tels que définis dans la revendication 1 ;
• **R₁** représente un atome d'hydrogène, un atome d'halogène, un radical -COOR₆ ou un radical de formule :
• -NH-CO-Ph
• un radical phényle éventuellement substitué par un ou deux groupes choisis parmi les atomes d'halogène, les radicaux alcoxy de 1 à 5 atomes de carbone et les radicaux -COOR₆, ou
• un radical thiényle éventuellement substitué par un ou deux groupes choisis parmi les radicaux alcoxy de 1 à 5 atomes de carbone et les radicaux -COOR₆, dans lesquels Ph et R₆ sont tels que définis dans la revendication 1 ;
• **R₂** représente un radical alcoxy de 1 à 5 atomes de carbone ou un radical -COOR₆, où R₆ est tel que défini dans la revendication 1 ; et
• **R₃** représente un radical amino,
à l'état de base ou de sel, ainsi qu'à l'état d'hydrate ou de solvat.

7. Procédé de préparation des composés de formule I selon l'une quelconque des revendications précédentes, **caractérisé en ce que** :
A) on condense le composé de formule II : 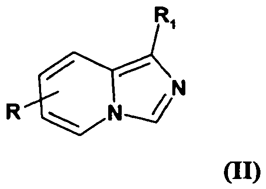 dans laquelle R est tel que défini pour le composé de formule I selon la revendication 1, mais R est différent d'un radical susceptible de réagir avec le composé de formule III, tel qu'un radical hydroxy, un radical carboxy ou un radical -NR₄R₅, et R est différent d'un radical -NH-CO₂R₆ ou d'un radical -CONR₄R₅,
avec le composé de formule III : 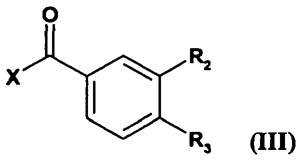 dans laquelle X représente un atome d'halogène et R₂ et R₃ représentent indépendamment l'un de l'autre un radical alcoxy de 1 à 5 atomes de carbone, un radical nitro ou un radical -COOR₆ dans lequel R₆ représente un radical alkyle de 1 à 5 atomes de carbone, pour obtenir :
- les composés de formule Ia qui sont des composés de formule I dans laquelle R₂ ou R₃ représentent un radical nitro, ou
- les composés de formule Ib qui sont des composés de formule I dans laquelle R₂ ou R₃ représentent un radical -COOR₆ dans lequel R₆ représente un radical alkyle de 1 à 5 atomes de carbone,
et ensuite,
a) on soumet les composés de formule Ia à une réaction de réduction pour obtenir les composés de formule Id : 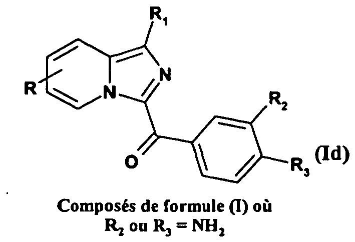 dans laquelle R et R₁ sont tels que définis pour le composé de formule Ia, et R₂ ou R₃ représente un radical amino ;
les composés de formule Id peuvent ensuite être soumis à une réaction d'alkylation, d'acylation ou de sulfonylation pour obtenir les composés de formule Ig : 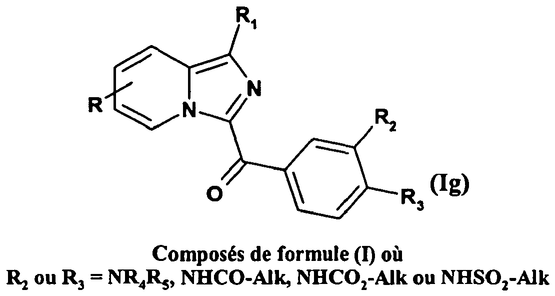 dans laquelle R et R₁ sont tels que définis pour le composé de formule Id, et R₂ ou R₃ représente un radical -NR₄R₅, un radical -NHCOAlk, un radical -NHCO₂Alk ou un radical -NHSO₂Alk ;
b) ou on soumet les composés de formule Ib à une réaction de saponification pour obtenir les composés de formule Ie : 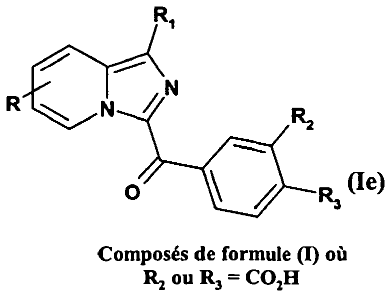 dans laquelle R et R₁ sont tels que définis pour le composé de formule Ib, et R₂ ou R₃ représente un radical carboxy,
les composés de formule Ie peuvent ensuite être soumis à une réaction de couplage après activation de la fonction carboxy, en présence d'une base, puis ajout d'une amine de formule HNR₄R₅ ou de l'hydroxylamine pour obtenir les composés de formule Ih : 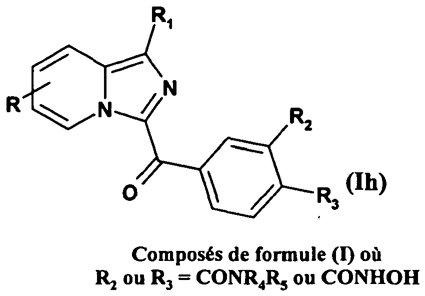 dans laquelle R et R₁ sont tels que définis pour les composés de formule Ie et R₂ ou R₃ représente un radical CONR₄R₅ ou CONHOH ;
**OU**
B) on condense le composé de formule II tel que précédemment défini au point A) avec le composé de formule III' : 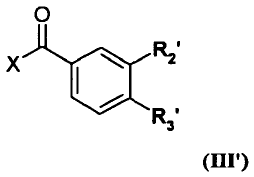 dans laquelle X représente un atome d'halogène et R₂' et R₃' forment ensemble, avec les atomes de carbone du noyau phényle auquel ils sont rattachés, un cycle carboné à 6 chaînons, comportant un atome d'azote et un autre hétéroatome,
pour obtenir les composés de formule Ic : 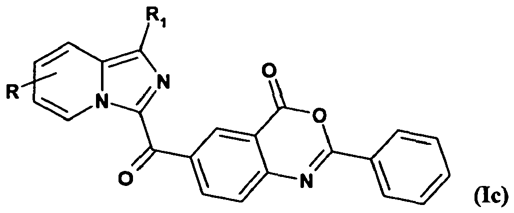 dans laquelle R et R₁ sont tels que définis pour le composé de formule II,
lesdits composés de formule Ic étant ensuite soumis à une réaction d'alcoolyse pour donner les composés de formule If : 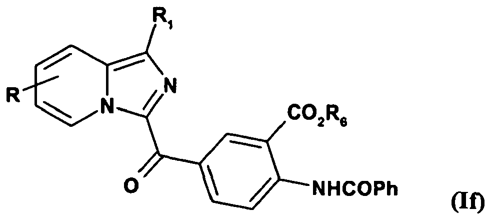 dans laquelle R et R₁ sont tels que définis pour le composé de formule II et R₆ est tel que défini pour le composé de formule I,
les composés If peuvent ensuite être saponifiés pour obtenir les composés de formules Id ou Ie, dans lesquelles R et R₁ sont tels que définis pour le composé de formule II, R₂ représente un radical -COOH et R₃ représente un radical -NH₂ ;
**OU**
C) on soumet le composé de formule I dans lequel R₁ représente un atome d'hydrogène, tel qu'obtenu précédemment au point A), à une réaction de bromation pour obtenir les composés de formule Ii : 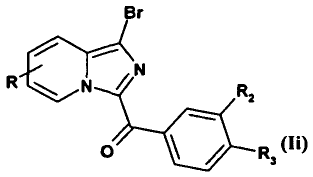 dans laquelle R, R₂ et R₃ sont tels que définis pour le composé de formule I selon la revendication 1, lorsque R₂ et R₃ ne forment pas ensemble un hétéroaryle, et R₁ représente un atome de brome,
les composés de formule Ii, pour lesquels R est différent d'un atome de brome ou d'un atome d'iode, peuvent être soumis en présence d'un catalyseur au palladium, d'un ligand et d'une base :
a) soit à une réaction d'imination avec la benzophénone imine, suivie d'une réaction d'hydrolyse acide, pour obtenir les composés de formule Ij : 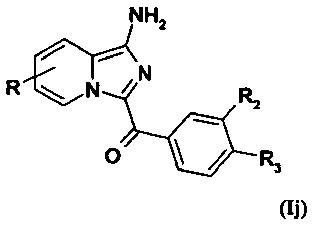 dans laquelle R est tel que défini pour les composés Ii et R₂ et R₃ sont tels que définis pour le composé de formule Ii et R₁ représente un radical -NH₂,
b) soit à une réaction de cyanation avec du cyanure de zinc, pour obtenir les composés de formule Ik: 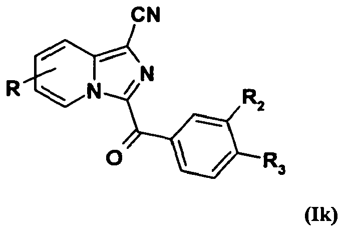 dans laquelle R, R₂ et R₃ sont tels que définis pour les composés Ii et R₁ représente un radical -CN,
- les composés de formule Ik peuvent ensuite être soumis à une réaction d'hydrolyse basique, afin d'obtenir les composés de formule Im : 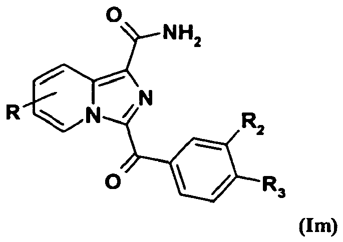
dans laquelle R, R₂ et R₃ sont tels que définis pour les composés de formule Ik et R₁ représente un radical -CONH₂,
- ou bien encore, les composés de formule Ik sont soumis à une réaction de Pinner, avec un alcool primaire en présence de gaz chlorhydrique pour conduire à l'imidoester correspondant, qui par hydrolyse acide conduit aux composés de formule In : 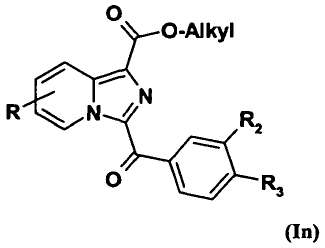
dans laquelle R, R₂ et R₃ sont tels que définis pour les composés de formule Ik et R₁ représente un radical -CO₂Alk où Alk est tel que défini selon la revendication 1,
les composés de formule In pouvant eux-même être soumis à une réaction de saponification pour obtenir les composés de formule Io : 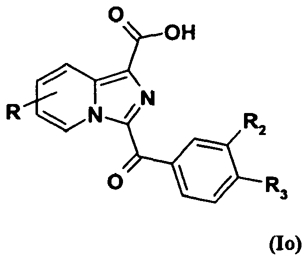 dans laquelle R, R₂ et R₃ sont tels que définis pour les composés de formule Ik et R₁ représente un radical -CO₂H,
c) soit à une réaction de Suzuki, avec des dérivés phénylboroniques ou hétéroarylboroniques pour obtenir les composés de formule Is : 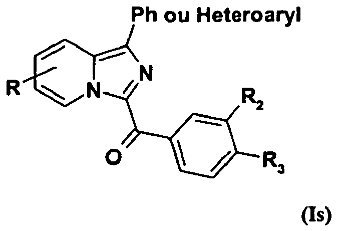 dans laquelle R, R₂ et R₃ sont tels que définis pour les composés Ii et R₁ représente un radical phényle substitué ou un hétéroaryle à 5 ou 6 chaînons éventuellement substitué ;
**OU**
D) on soumet les composés de formule Ij, dans lequel R₁ représente un radical amino, à une réaction d'acylation ou de sulfonylation, pour obtenir les composés de formule Ip : 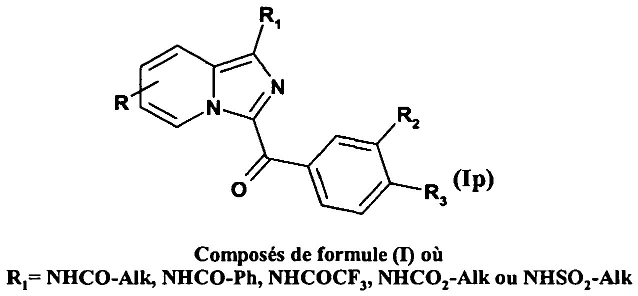 dans laquelle R, R₂ et R₃ sont tels que définis pour les composés de formule Ij et R₁ représente un radical -NHCOAlk,-NHCO₂Alk,-NHSO₂Alk, -NHCOPh ou -NHCOCF₃, dans lesquels Alk et Ph sont tels que définiss pour le composé de formule I selon la revendication 1,
les composés de formule Ip dans laquelle R₁ représente un radical -NHCOCF₃ pouvant eux-mêmes être soumis à une réaction d'alkylation puis de déprotection, éventuellement suivie d'une autre réaction d'alkylation, pour obtenir les composés de formule Iq : 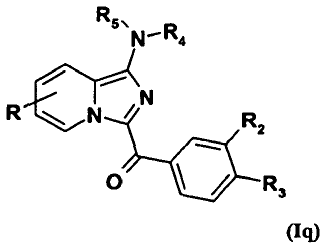 dans laquelle R, R₂, R₃ sont tels que définis pour les composés de formule Ij et R₄ et R₅ sont tels que définis pour le composé de formule I ;
**OU**
E) on soumet les composés de formule Ir, dans lequel R représente un radical -CO₂R₆ où R₆ représente un radical Alk, tel qu'obtenus précédemment au point A), à une réaction d'hydrolyse acide ou basique pour obtenir les composés de formule It : 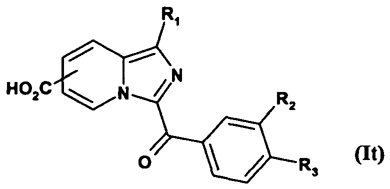 dans laquelle R₁, R₂, R₃ sont tels que définis pour le composé de formule Ir et R représente un radical -COOH,
les composés de formule It peuvent ensuite être soumis :
a) soit à une réaction de couplage après activation de la fonction carboxy, en présence d'une base, puis ajout d'une amine de formule HNR₄R₅ ou H₂N-CH(R₇)-(CH₂)ₘ-COOR₆, où R₆ représente un radical Alk tel que défini selon la revendication 1, pour obtenir les composés de formule Iu : 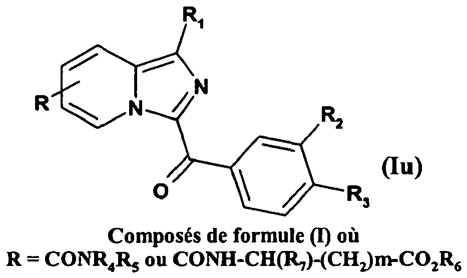 dans laquelle R₁, R₂, R₃ sont tels que définis pour les composés de formule It,
et lorsque R est un radical -CONH-CH(R₇)-(CH₂)ₘ-COOR₆ où R₆ représente un radical Alk tel que défini selon la revendication 1, ces composés peuvent être saponifiés pour obtenir les composés de formule Iu où R est un radical -CONH-CH(R₇)-(CH₂)ₘ-COOR₆ où R₆ représente un atome d'hydrogène et R₁, R₂, R₃ sont tels que définis précédemment,
b) soit être soumis à un réarrangement de Curtius, par action de diphénylphosphorylazide en présence de triéthylamine à reflux dans un solvant inerte puis ajout d'un alcool de formule Alk-OH, pour obtenir les composés de formule Iv : 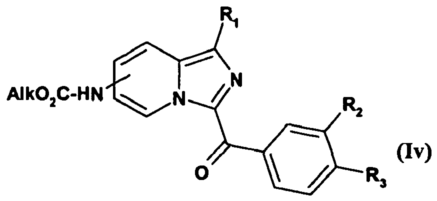 dans laquelle R₁, R₂, R₃ sont tels que définis pour les composés de formule It et R représente un radical -NHCO₂Alk,
les composés de formule Iv dans lesquels R représente un radical -NH-CO₂-Alk, où Alk représente un radical -tBu, peuvent ensuite conduire aux composés de formule Iw, dans laquelle R₁, R₂, R₃, R₄, R₅ sont tels que définis pour le composé de formule I selon la revendication 1 : 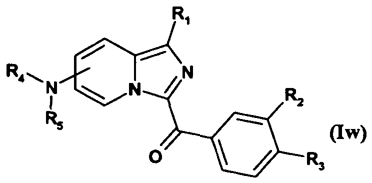
- par déprotection en milieu acide on obtient les composés de formule Iw où R représente un radical -NH₂,
- par alkylation suivi d'une déprotection et d'une éventuelle deuxième alkylation on peut obtenir les composés de formule Iw où R représente un radical -NR₄R₅,
les composés de formule Iw où R représente un radical -NH₂ peuvent être soit acylés, soit sulfonylés pour obtenir les composés de formule Ix : 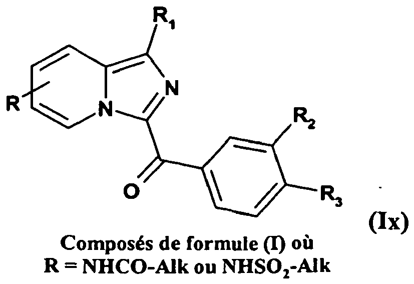 dans laquelle R₁, R₂, R₃ sont tels que définis pour le composé de formule Iw et R représente un radical -NHCOAlk ou -NHSO₂Alk ;
**OU**
F) on soumet les composés de formule Iy : 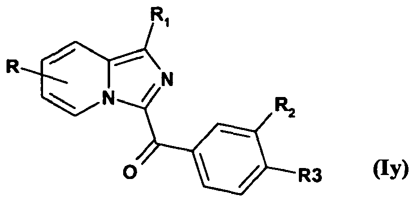 dans laquelle R représente un radical -Obenzyl et R₁, R₂ et R₃ sont tels que définis dans les composés de formule I selon la revendication 1, à une réaction de débenzylation, dans un solvant protique, en présence de palladium sur charbon, pour obtenir les composés de formule Iz : 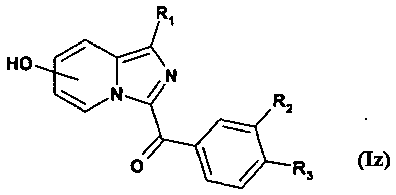 dans laquelle R₁, R₂ et R₃ sont tels que définis pour les composés de formule Iy et R représente un radical hydroxy, et lorsque R₂ ou R₃ représente une fonction nitro on obtient les composés de formule Id dans laquelle R₂ ou R₃ représente un radical NH₂ et R₁ est tel que défini dans les composés de formule I,
les composés de formule Iz peuvent ensuite être soumis à une réaction de O-alkylation sélective par action à température ambiante d'un halogénure d'alkyle dans un solvant polaire, en présence d'un carbonate alcalin, pour obtenir les composés de formule Iz' 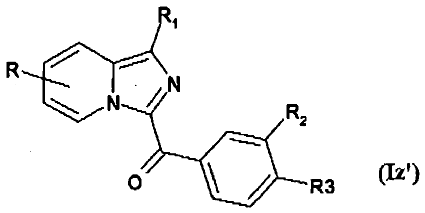 **Composé de formule I où : R = O-Alk, O-(CH₂)ₙ-Ph, O-Alk-NR₄R₅ ou O-Alk-CO₂R₆** dans laquelle R₁, R₂ et R₃. sont tels que définis pour les composés de formule Iz ,
et lorsque R est un radical -O-Alk-COOR₆, où R₆ représente un radical Alk tel que défini pour les composés de formule I, ces composés peuvent être saponifiés pour obtenir les composés de formule Iz' où R est un radical -O-Alk-COOR₆, où R₆ représente un atome d'hydrogène et R₁, R₂, R₃ sont tels que définis précédemment.

8. Médicament, **caractérisé en ce qu'**il comprend un composé de formule I selon l'une quelconque des revendications 1 à 6, ou un sel d'addition de ce composé à un acide ou à une base pharmaceutiquement acceptable, ou encore un hydrate ou un solvat du composé de formule I.

9. Composition pharmaceutique, **caractérisée en ce qu'**elle comprend un composé de formule I selon l'une quelconque des revendications 1 à 6, ou un sel pharmaceutiquement acceptable, un hydrate ou un solvat de ce composé, ainsi qu'au moins un excipient pharmaceutiquement acceptable.

10. Utilisation d'un composé de formule I selon l'une quelconque des revendications 1 à 6 pour la préparation d'un médicament destiné au traitement des cancers, notamment des carcinomes ayant un degré de vascularisation important tels que les carcinomes de poumon, sein, prostate et cesophage, des cancers induisant des métastases tels que le cancer du colon et le cancer de l'estomac, des mélanomes, des gliomes, des lymphomes, et des leucémies.

11. Utilisation selon la revendication 10, **caractérisée en ce que** le composé de formule I est associé avec un ou plusieurs principe(s) actif(s) anticancéreux et/ou avec une radiothérapie.

12. Utilisation selon la revendication 10, pour la préparation d'un médicament destiné au traitement des maladies cardiovasculaires telles que l'athérosclérose, la resténose post angioplastie, des maladies liés aux complications apparaissant suite à la pose de prothèses endovasculaires et/ou de pontages aorto-coronariens ou d'autres greffes vasculaires de l'hypertrophie cardiaque, ou des complications vasculaires du diabète comme les rétinopathies diabétiques.

13. Utilisation selon la revendication 10, pour la préparation d'un médicament destiné au traitement des maladies inflammatoires chroniques comme l'arthrite rhumatoïde ou les IBD.

14. Utilisation selon la revendication 10, pour la préparation d'un médicament destiné au traitement de l'ostéoarthrite, des achondroplasies (ACH), des hypochondroplasies (HCH) et des TD (Thanatophoric dysplasia).

15. Utilisation selon la revendication 10, pour la préparation d'un médicament destiné au traitement de l'obésité.

16. Utilisation selon la revendication 10, pour la préparation d'un médicament destiné au traitement de la dégénérescence maculaire, tel que la dégénérescence maculaire liée à l'âge (DMLA).

## Claims

1. Compound of formula I : 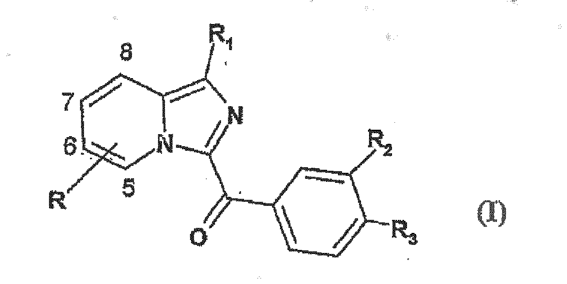 in which:
• R, present on the 5, 6, 7 or 8 positions of the imidazo[1,5-a]pyridine, represents a hydrogen atom, a halogen atom, an alkyl radical of 1 to 5 carbon atoms, a hydroxyl radical, an alkoxy radical of 1 to 5 carbon atoms, a -COOR₆ radical or a radical of formula:
• -NR₄R₅
• -NH-SO₂-Alk
• -NH-CO-Alk
• -NR₆-CO₂-Alk
• -O-Alk-COOR₆
• -O-Alk-NR₄R₅
• -O-(CH₂)ₙ-Ph
• -CO-NR₄R₅, or
• -CO-NH-CH(R₇)-(CH₂)ₘ-COOR₆
in which:
• Alk represents an alkyl radical or an alkylene radical of 1 to 5 carbon atoms,
• n represents an integer from 1 to 5,
• m represents an integer from 0 to 4,
• R₄ and R₅ represent, independently of one another, a hydrogen atom, an alkyl radical of 1 to 5 carbon atoms or a benzyl radical,
• R₆ represents a hydrogen atom or an alkyl radical of 1 to 5 carbon atoms,
• R₇ represents a hydrogen atom, an alkyl radical of 1 to 5 carbon atoms or a radical of formula:
• -Alk-CONR₄R₅
• -Alk-OR₆
• -Alk-NR₄R₅
• -Ph, or
• -CH₂Ph, and
• Ph represents a phenyl radical optionally substituted by one or more groups chosen from halogen atoms, alkoxy radicals of 1 to 5 carbon atoms and -COOR₆ radicals where R₆ is as defined above;
• R₁ represents a hydrogen atom, a halogen atom, a cyano radical, a -COOR₆ radical or a radical of formula:
• -NR₄R₅
• -NH-SO₂-Alk
• -NH-CO-CF₃
• -NH-CO-Ph
• -NH-CO-Alk
• -NH-CO₂-Alk
• -CONR₄R₅
• a phenyl radical optionally substituted by one or more groups chosen from halogen atoms, alkyl radicals of 1 to 5 carbon atoms, alkoxy radicals of 1 to 5 carbon atoms and -COOR₆ radicals,
• a 5-membered heteroaryl radical comprising a heteroatom chosen from a sulphur atom, an oxygen atom or a nitrogen atom and optionally comprising a second nitrogen atom, said heteroaryl optionally being substituted by one or more groups chosen from halogen atoms, alkyl radicals of 1 to 5 carbon atoms, alkoxy radicals of 1 to 5 carbon atoms and -COOR₆ radicals, or
• a 6-membered heteroaryl radical comprising 1 or 2 nitrogen atoms and optionally being substituted by one or more groups chosen from halogen atoms, alkyl radicals of 1 to 5 carbon atoms, alkoxy radicals of 1 to 5 carbon atoms and -COOR₆ radicals,
in which Alk, Ph, R₄, R₅ and R₆ are as defined as above;
• R₂ and R₃ represent, independently of one another, a hydroxyl radical, an alkoxy radical of 1 to 5 carbon atoms, an amino radical, a -COOR₆ radical, a nitro radical or a radical of formula:
• -NR₄R₅
• -NH-CO-Alk
• -NH-CO-Ph
• -NH-CO₂-Alk
• -NH-SO₂-Alk
• -CO-NR₄R₅, or
• -CO-NHOH
in which Alk, Ph, R₄, R₅ and R₆ are as defined as above;
or else R₂ and R₃ together form, with the carbon atoms of the phenyl ring to which they are attached, a 6-membered carbon ring comprising a nitrogen atom and another heteroatom, such as oxygen,
in the base or salt form and in the hydrate or solvate form.

2. Compound of formula I according to Claim 1, in which:
• R, present on the 6, 7 or 8 positions of the imidazo[1,5-a]pyridine, represents a hydrogen atom, an alkyl radical of 1 to 5 carbon atoms, an alkoxy radical of 1 to 5 carbon atoms, a hydroxyl radical, a -COOR₆ radical or a radical of formula:
• -NR₄R₅
• -NH-SO₂-Alk
• -NH-CO-Alk
• -NR₆-CO₂-Alk
• -O-Alk-COOR₆
• -O-Alk-NR₄R₅
• -O-CH₂-Ph
• -CO-NR₄R₅, or
• -CO-NH-CH(R₇)-(CH₂)ₘ-COOR₆
in which Alk, Ph, R₄, R₅, R₆, R₇ and m are as defined in Claim 1;
• R₁ represents a hydrogen atom, a halogen atom, a cyano radical, a -COOR₆ radical or a radical of formula:
• -NR₄R₅
• -NH-SO₂-Alk
• -CO-CF₃
• -NH-CO-Ph
• -NH-CO-Alk
• -CO-NR₄R₅
• a phenyl radical optionally substituted by one or two groups chosen from halogen atoms, alkoxy radicals of 1 to 5 carbon atoms an -COOR₆ radicals;
• a 5-membered heteroaryl radical comprising a heteroatom chosen from a sulphur atom, an oxygen atom or a nitrogen atom and optionally comprising a second nitrogen atom, said heteroaryl optionally being substituted by one or two groups chosen from halogen atoms, alkyl radicals of 1 to 5 carbon atoms, alkoxy radicals of 1 to 5 carbon atoms and -COOR₆ radicals, or
• a 6-membered heteroaryl radical comprising 1 or 2 nitrogen atoms and optionally being substituted by one or two groups chosen from halogen atoms, alkyl radicals of 1 to 5 carbon atoms, alkoxy radicals of 1 to 5 carbon atoms and -COOR₆ radicals, where Alk, Ph and R₆ are as defined in Claim 1;
• R₂ and R₃ represent, independently of on another, an alkoxy radical of 1 to 5 carbon atoms, a -COOR₆ radical, an amino radical, a nitro radical or a radical of formula:
• -NR₄R₅
• -NH-CO-Alk
• -NH-CO-Ph
• -NH-SO₂-Alk
in which Alk, Ph, R₄, R₅ and R₆ are as defined in Claim 1;
in the base or salt form and in the hydrate or solvate form.

3. Compound of formula I according to Claim 1 or 2, in which:
• R, present on the 6, 7 or 8 positions of the imidazo[1,5-a]pyridine, represents a hydrogen atom, an alkoxy radical of 1 to 5 carbon atoms, a hydroxyl radical, a -COOR₆ radical or a radical of formula:
• -NR₄R₅
• -NH-SO₂-Alk
• -NH-CO-Alk
• -NR₆-CO₂-Alk
• -O-Alk-COOR₆
• -CO-NR₄R₅, or
• -CO-NH-CH(R₇)-(CH₂)ₘ-COOR₆
in which m represents 0 or 1, R₇ represents a hydrogen atom, an alkyl radical of 1 to 5 carbon atoms or a radical of formula -Alk-OR₆ or -CH₂-Ph, and Alk, R₄, R₅ and R₆ are as defined in Claim 1;
• R₁ represents a hydrogen atom, a halogen atom, a cyano radical, a -COOR₆ radical or a radical of formula:
• -NR₄R₅
• -NH-SO₂-Alk
• -NH-CO-Ph
• -NH-CO-Alk
• a phenyl radical optionally substituted by one or two groups chosen from halogen atoms, alkoxy radicals of 1 to 5 carbon atoms and -COOR₆ radicals,
• a heteroaryl radical chosen from thienyl, furyl and pyrrolyl radicals, said heteroaryl optionally being substituted by one or two groups chosen from alkoxy radicals of 1 to 5 carbon atoms and -COOR₆ radicals, or
• a pyridinyl radical optionally substituted by one or two groups chosen from alkoxy radicals of 1 to 5 carbon atoms and -COOR₆ radicals,
in which Alk, Ph, R₄ and R₆ are as defined in Claim 1;
• R₂ and R₃ represent, independently of one another, an alkoxy radical of 1 to 5 carbon atoms, a -COOR₆ radical, a nitro radical, an amino radical or a radical of formula -NH-CO-Alk, -NH-CO-Ph or -NH-SO₂Alk;
in which Alk, Ph and R₆ are as defined in Claim 1;
in the base or salt form and in the hydrate or solvate form.

4. Compound of formula I according to any one of Claims 1 to 3, in which R₂ represents an alkoxy radical of 1 to 5 carbon atoms or a -COOR₆ radical where R₆ is as defined in Claim 1,
in the base or salt form and in the hydrate or solvate form.

5. Compound of formula I according to any one of Claims 1 to 4, in which R₃ represents a nitro radical, an amino radical or a radical of formula -NH-CO-Alk, -NH-CO-Ph or -NH-SO₂Alk, where Alk and Ph are as defined in Claim 1,
in the base or salt form and in the hydrate or solvate form.

6. Compound of formula I according to any one of Claims 1 to 5, in which:
• R, present on the 6, 7 or 8 positions of the imidazo[1,5-a]pyridine, represents a hydrogen atom, a hydroxyl radical, a -COOR₆ radical or a radical of formula:
• -O-Alk-COOR₆
• -CO-NR₄R₅, or
• -CO-NH-CH(R₇)-COOR₆
in which R₇ represents a hydrogen atom, an alkyl radical of 1 to 5 carbon atoms or a radical of formula -Alk-OR₆ and Alk, R₄, R₅ and R₆ are as defined in Claim 1;
• R₁ represents a hydrogen atom, a halogen atom, a -COOR₆ radical or a radical of formula:
• -NH-CO-Ph
• a phenyl radical optionally substituted by one or two groups chosen from halogen atoms, alkoxy radicals of 1 to 5 carbon atoms and -COOR₆ radicals or
• a thienyl radical optionally substituted by one or two groups chosen from alkoxy radicals of 1 to 5 carbon atoms and -COOR₆ radicals, in which Ph and R₆ are as defined in Claim 1;
• R₂ represents an alkoxy radical of 1 to 5 carbon atoms or a -COOR₆ radical where R₆ is as defined in Claim 1; and
• R₃ represents an amino radical,
in the base or salt form and in the hydrate or solvate form.

7. Process for the preparation of the compounds of formula I according to any one of the preceding claims, **characterized in that**:
A) the compound of formula II: 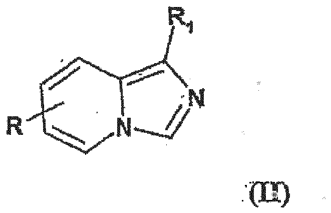 in which R is as defined for the compound of formula I according to Claim 1 but R is other than a radical capable of reacting with the compound of formula III, such as a hydroxyl radical, a carboxyl radical or an -NR₄R₅ radical, and R is other than an -NH-CO₂R₆ radical or than a -CONR₄R₅ radical,
is condensed with the compound of formula III: 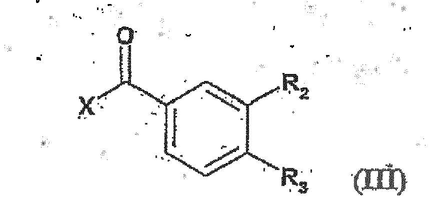 in which X represents a halogen atom and R₂ and R₃ represent, independently of one another, an alkoxy radical of 1 to 5 carbon atoms, a nitro radical or a -COOR₆ radical in which R₆ represents an alkyl radical of 1 to 5 carbon atoms, in order to obtain:
- the compounds of formula Ia, which are compounds of formula I in which R₂ or R₃ represents a nitro radical, or
- the compounds of formula Ib, which are compounds of formula I in which R₂ or R₃ represents a -COOR₆ radical in which R₆ represents an alkyl radical of 1 to 5 carbon atoms,
and, subsequently,
a) the compounds of formula Ia are subjected to a reduction reaction, in order to obtain the compounds of formula Id: 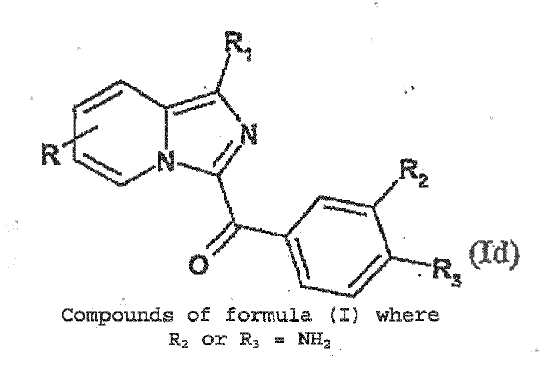 in which R and R₁ are as defined for the of formula Ia and R₂ or R₃ represents an amino radical;
the compounds of formula Id can subsequently be subjected to an alkylation, acylation or sulphonylation reaction in order to obtain the compounds of formula Ig: 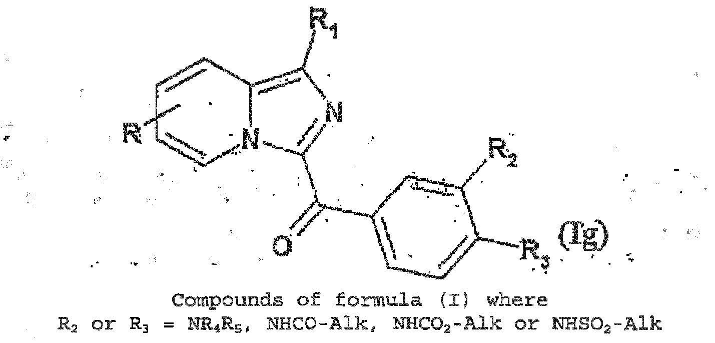 in which R and R₁ are as defined for the compound of formula Id and or R₃ represents an -NR₄R₅ radical, an -NHCOAlk radical, an -NHCO₂Alk radical or an -NHSO₂Alk radical;
b) or the compounds of formula Ib are subjected to a saponification reaction in order to obtain the compounds of formula Ie: 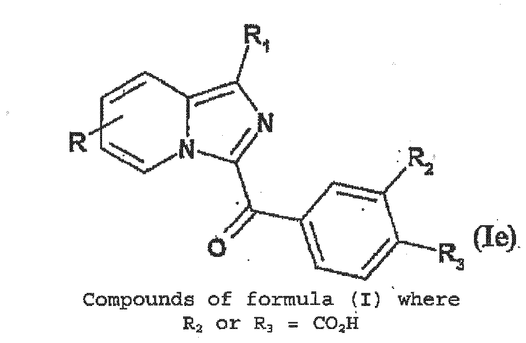 in which R and R₁ are as defined for the compound of formula Ib and R₂ or R₃ represents a carboxyl radical, the compounds of formula Ie can subsequently be subjected to a coupling reaction after activation of the carboxyl functional group, in the presence of a base, and then addition of an amine of formula HNR₄R₅ or of hydroxylamine in order to obtain the compounds of formula Ih: 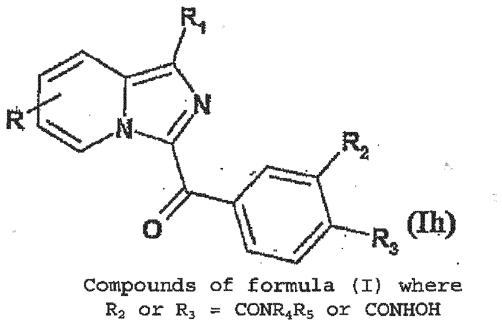 in which R and R₁ are as defined for the compounds of formulate and R₂ or R₃ represents a -CONR₄R₅ or -CONHOH radical;
OR
B) the compound of formula II as defined above in part A) is condensed with the compound of formula III': 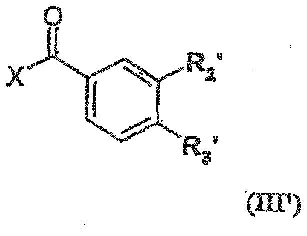 in which X represents a halogen atom and R₂' and R₃' together form, with the carbon atoms of the phenyl ring to which they are attached, a 6-membered carbon ring comprising a nitrogen atom and another heteroatom,
in order to obtain the compounds of formula Ic: 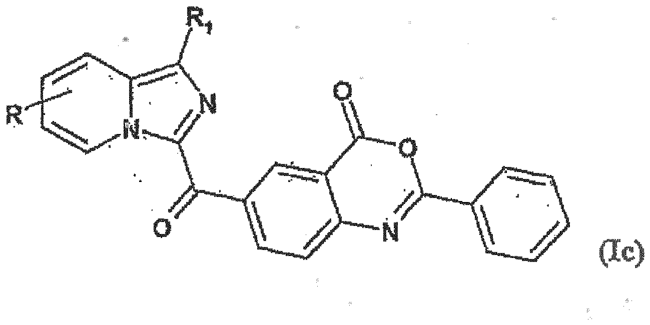 in which R and R₁ are as defined for the compound of formula II,
said compounds of formula Ic subsequently being subjected to an alcoholysis reaction in order to give the compounds of formula If: 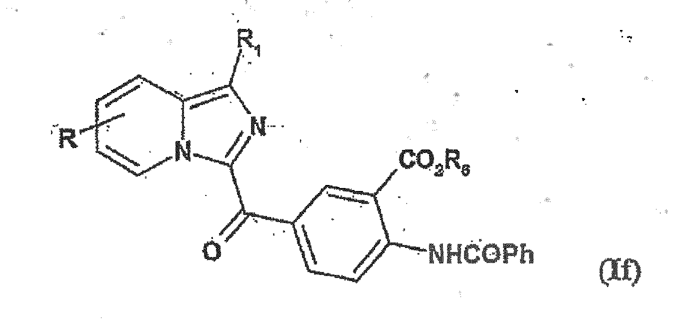 in which R and R₁ are as defined for the compound of formula II and R₆ is as defined for the compound of formula I,
the compounds If can subsequently be saponified in order to obtain the compounds o formulae Id or Ie in which R and R₁ are as defined for the compound of formula II, R₂ represents a -COOH radical and R₃ represents an radical;
OR
C) the compound of formula I in which R₁ represents a hydrogen atom, as obtained above in part A), is subjected to a bromination reaction in order to obtain the compounds of formula Ii: 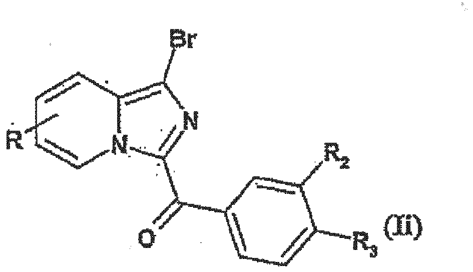 in which R, R₂ and R₃ are as defined for the compound of formula I according to Claim 1, when R₂ and R₃ do not together form a heteroaryl, and R₁ represents a bromine atom,
the compounds of formula Ii for which R is other than a bromine atom or than an iodine atom can be subjected, in the presence of a palladium catalyst, of a ligand and of a base:
a) either to an imination reaction with a benzophenone imine, followed by an acid hydrolysis reaction, in order to obtain the compounds of formula Ij: 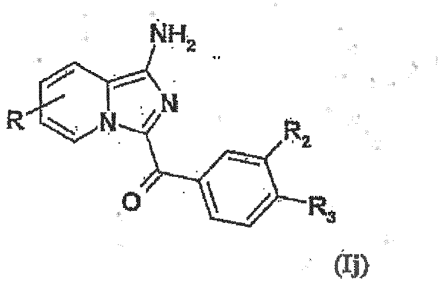 in which R is as defined for the compounds Ii and R₂ and R₃ are as defined for the compound of formula Ii and R₁ represents an -NH₂ radical,
b) or to a cyanation reaction with zinc cyanide in order to obtain the compounds of formula Ik: 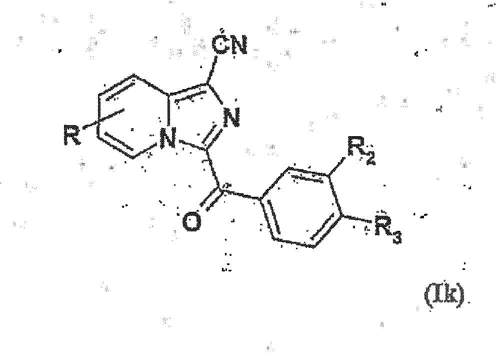 in which R, R₂ and R₃ are as defined for the compounds Ii and R₁ represents a -CN radical,
- the compounds of formula Ik can subsequently be subjected to a basic hydrolysis, reaction in order to obtain the compounds of formula Im: 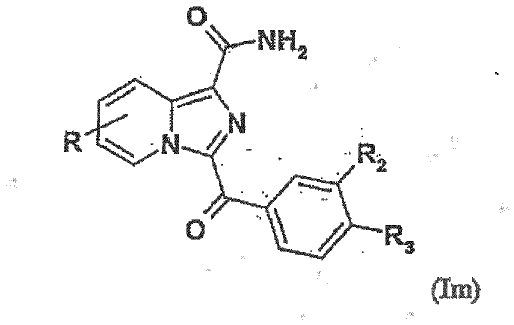 in which R, R₂ and R₃ are as defined for the compounds of formula Ik and R₁ represents a -CONH₂ radical,
- or alternatively the compounds of formula Ik are subjected to a Pinner reaction with a primary alcohol in the presence of hydrogen chloride gas to result in the corresponding imidoester, which, by acid hydrolysis, results in the compounds of formula In: 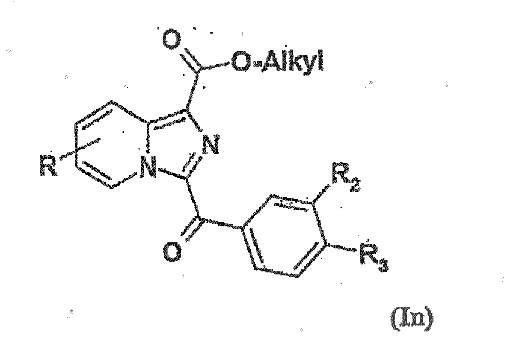
in which R, R₂ and R₃ are as defined for the compounds of formula Ik and R₁ represents a -CO₂Alk radical where Alk is as defined in Claim 1,
it being possible for the compounds of formula In themselves to be subjected to a saponification reaction in order to obtain the compounds of formula Io: 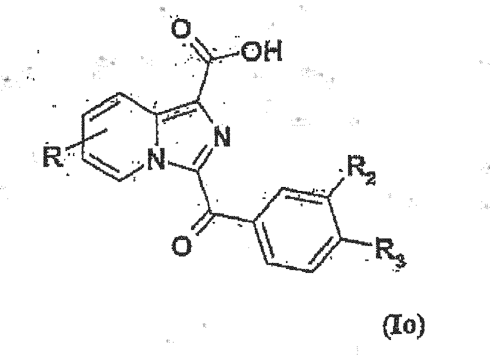 in which R, R₂ and R₃ are as defined for the compounds of formula Ik and R₁ represents a -CO₂H radical,
c) or to a Suzuki reaction with phenylboronic or heteroarylboronic derivatives in order to obtain the compounds of formula Is: 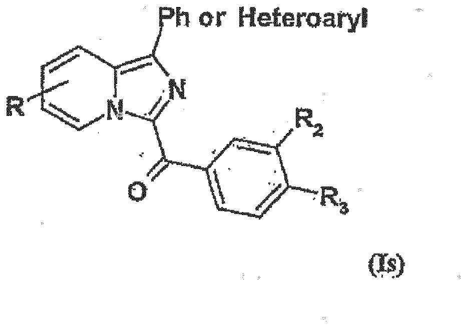 in which R, R₂ and R₃ are as defined for the compounds Ii and R₁ represents a substituted phenyl radical or an optionally substituted 5- or 6-membered heteroaryl;
OR
D) the compounds of formula Ij in which R₁ represents an amino radical are subjected to an acylation or sulphonylation reaction in order to obtain the compounds of formula Ip: 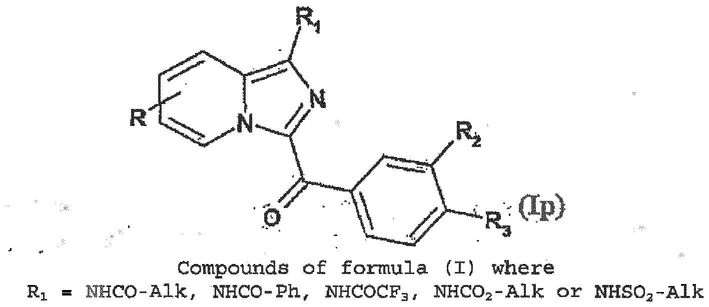 in which R, R₂ and R₃ are as defined for the compounds of formula Ij and R₁ represents an -NHCOAlk, -NHCO₂Alk, -NHSO₂Alk, -NHCOPh or -NHCOCF₃, radical in which Alk and Ph are as defined for the compound of formula I according to Claim 1, it being possible for the compounds of formula Ip in which R₁ represents an -NHCOCF₃ radical to be themselves subjected to an alkylation and then deprotection reaction, optionally followed by another alkylation reaction, in order to obtain the compounds of formula Iq: 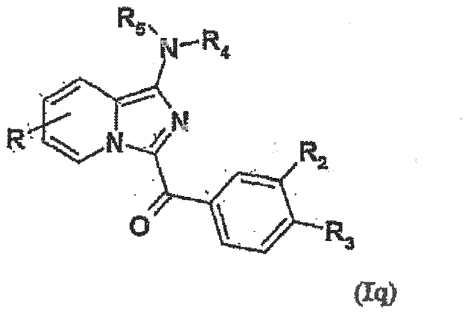 in which R, R₂ and R₃ are as defined for the compounds of formula Ij and R₄ and R₅ are as defined for the compound of formula I;
OR
E) the compounds of formula Ir in which R represents a -CO₂R₆ radical where R₆ represents an Alk radical as obtained above in part A) are subjected to an acid or basic hydrolysis reaction in order to obtain the compounds of formula It: 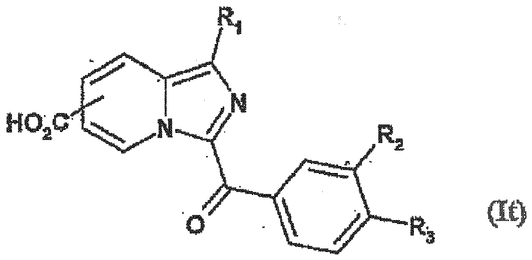 in which R₁, R₂ and R₃ are as defined for the compound of formula Ir and R represents a -COOH radical
the compounds of formula It can subsequently be subjected:
a) either to a coupling reaction after activation of the carboxyl functional group, in the presence of a base, and then addition of an amine of formula HNR₄R₅ or H₂N-CH(R₇)-(CH₂)ₘ-COOR₆ where R₆ represents an Alk radical as defined in Claim 1, in order to obtain the compounds of formula Iu: 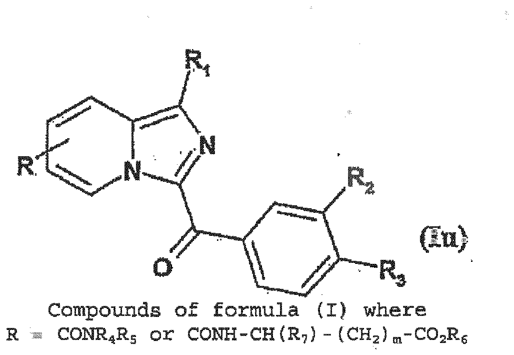 in which R₁, R₂ and R₃ are as defined for the compounds of formula It,
and, when R is a -CONH-CH(R₇)-(CH₂)ₘ-COOR₆ radical where R₆ represents an Alk radical as defined in Claim 1, these compounds can be saponified in order to obtain the compounds of formula Iu where R is a -CONH-CH(R₇)-(CH₂)ₘ-COOR₆ radical where R₆ represents a hydrogen atom and R₁, R₂ and R₃ are defined above,
b) or to Curtius rearrangements by the action of diphenylphosphoryl azide in the presence of triethylamine at reflux in an inert solvent and then addition of an alcohol of formula Alk-OH in order to obtain the compounds of formula Iv: 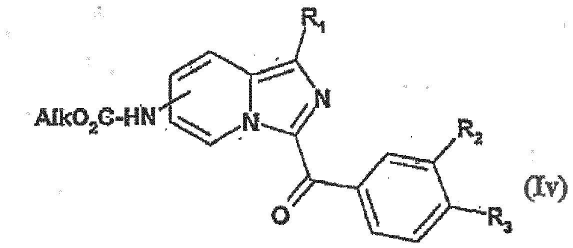 in which R₁, R₂ and R₃ are as defined for the compounds of formula It and R represents an -NHCO₂Alk radical,
the compounds of formula Iv in which R represents an -NH-CO₂-Alk radical where Alk represents a -tBu radical can subsequently result in the compounds of formula Iw in which R₁, R₂, R₃, R₄ and R₅ are as defined for the compound of formula I according to Claim 1: 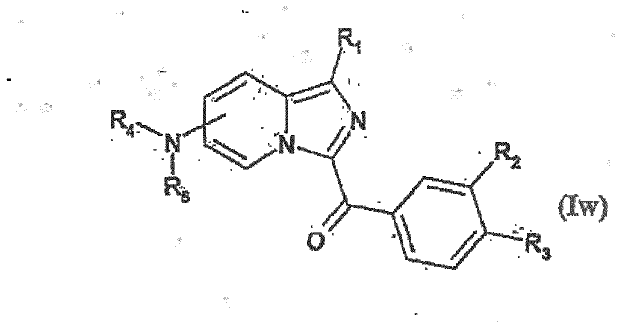
- by deprotection in an acid medium, the compounds of formula Iw where R represents an -NH₂ radical are obtained,
- by alkylation followed by deprotection and by an optional second alkylation, the compounds of formula Iw where R represent an -NR₄R₅ radical can be obtained,
the compounds of formula Iw where R represents an -NH₂ radical can be either acylated or sulphonylated in order to obtain the compounds of formula Ix: 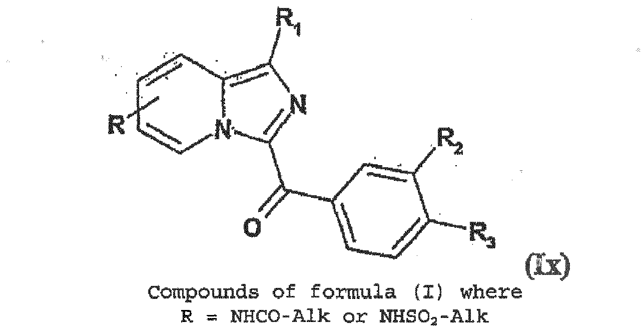 in which R₁, R₂ and R₃ are as defined for the compound of formula Iw R represents an -NHCOAlk or -NHSO₂Alk radical;
**OR**
F) the compounds of formula Iy: 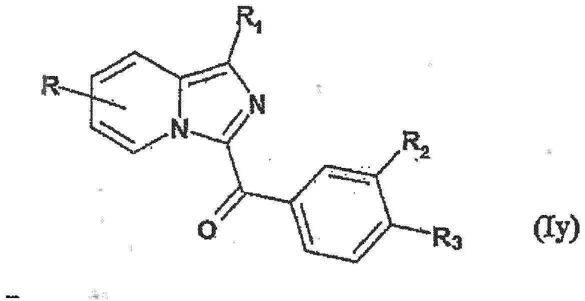 in which R represents an -O-benzyl radical and R₁, R₂ and R₃ are as defined in the compounds of formula I according to Claim 1, are subjected to a debenzylation reaction in a protic solvent in the presence of palladium-on-charcoal in order to obtain the compounds of formula Iz: 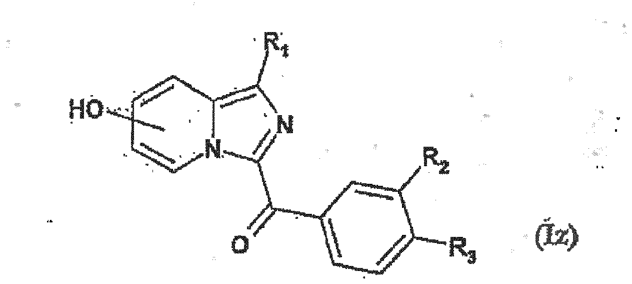 in which R₁, R₂ and R₃ are as defined for the compounds of formula Iy an R represents a hydroxyl radical, and, when R₂ or R₃ represents a nitro functional group, the compounds of formula Id in which R₂ or R₃ represents an NH₂ radical and R₁ is as defined in the compounds of formula I are obtained,
the compounds of formula Iz can subsequently be subjected to a selective O-alkylation reaction by the action at ambient temperature of an alkyl halide in a polar solvent in the presence of an alkaline carbonate in order to obtain the compounds of formula Iz' 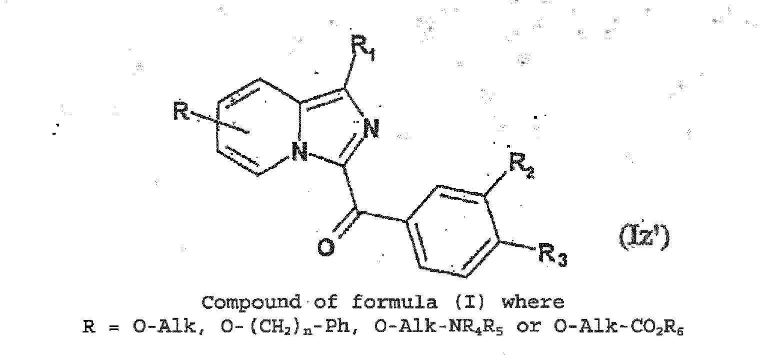 in which R₁, R₂ and R₃ are as defined for the compounds of formula Iz,
and, when R is an -O-Alk-COOR₆ radical where R₆ represents an Alk radical as defined for the compounds of formula I, these compounds can be saponified in order to obtain the compounds of formula Iz' where R is an -O-Alk-COOR₆ radical where R₆ represents a hydrogen atom and R₁, R₂ and R₃ are as defined above.

8. Medicament, **characterized in that** it comprises a compound of formula I according to any one of Claims 1 to 6 or an addition salt of this compound with a pharmaceutically acceptable acid or base or also a hydrate or a solvate of the compound of formula I.

9. Pharmaceutical composition, **characterized in that** it comprises a compound of formula I according to any one of Claims 1 to 6 or a pharmaceutically acceptable salt, a hydrate or a solvate of this compound, and at least one pharmaceutically acceptable excipient.

10. Use of a compound of formula I according to any one of Claims 1 to 6 in the preparation of a medicament intended for the treatment of cancers, in particular carcinomas having a high degree of vascularization, such as lung, breast, prostate and oesophageal carcinomas, cancers which induce metastases, such as colon cancer and stomach cancer, melano as, gliomas, lymphomas and leukemias.

11. Use according to Claim 10, **characterized in that** the compound of formula I is used in combination with one or more anticancer active principle(s) and/or with radiotherapy.

12. Use according to Claim 10, in the preparation of a medicament intended for the treatment of cardiovascular diseases, such as atherosclerosis or, post-angioplasty restenosis, diseases related to the complications which appear subsequent to the fitting of endovascular prostheses and/or aortocoronary bypasses or other vascular grafts of cardiac hypertrophy, or vascular complications of diabetes, such as diabetic retinopathy.

13. Use according to Claim 10, in the preparation of a medicament intended for the treatment of chronic inflammatory diseases, such as rheumatoid arthritis or IBD.

14. Use according to Claim 10, in the preparation of a medicament intended for the treatment of osteoarthritis, achondroplasia (ACH), hypochondroplasia (HCH) and TD (thanatophoric dysplasia).

15. Use according to Claim 10, in the preparation of a medicament intended for the treatment of obesity.

16. Use according to Claim 10, in the preparation of a medicament intended for the treatment of macular degeneration, such as age-related macular degeneration (AMD).

## Patentansprüche

1. Verbindung der Formel I: 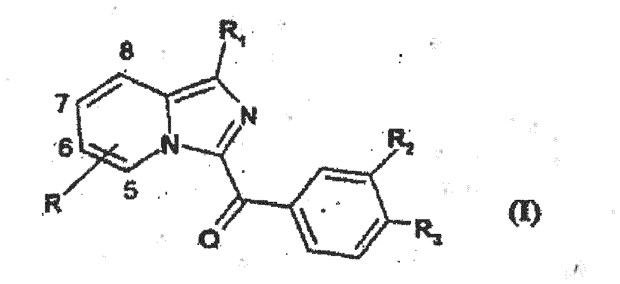 in der:
• R, der an den Positionen 5, 6, 7 oder 8 des Imidazo[1,5-a]pyridins vorliegt, für ein Wasserstoffatom, ein Halogenatom, einen Alkylrest mit 1 bis 5 Kohlenstoffatomen, einen Hydroxyrest, einen Alkoxyrest mit 1 bis 5 Kohlenstoffatomen, einen Rest -COOR₆ oder einen Rest der folgenden Formel steht:
• -NR₄R₅
• -NH-SO₂-Alk
• -NH-CO-Alk
• -NR₆-CO₂-Alk
• -O-Alk-COOR₆
• -O-Alk-NR₄R₅
• -O-(CH₂)ₙ-Ph
• -CO-NR₄R₅ oder
• -CO-NH-CH(R₇)-(CH₂)ₘ-COOR₆, worin:
• Alk für einen Alkylrest oder einen Alkylenrest mit 1 bis 5 Kohlenstoffatomen steht,
• n für eine ganze Zahl von bis 5 steht,
• m für eine ganze Zahl von 0 bis 4 steht,
• R₄ und R₅ unabhängig voneinander für ein Wasserstoffatom, einen Alkylrest mit 1 bis 5 Kohlenstoffatomen oder einen Benzylrest stehen,
• R₆ für ein Wasserstoffatom oder einen Alkylrest mit 1 bis 5 Kohlenstoffatomen steht,
• R₇ für ein Wasserstoffatom, einen Alkylrest mit 1 bis 5 Kohlenstoffatomen oder einen Rest der folgenden Formel steht:
• -Alk-CONR₄R₅
• -Alk-OR₆
• -Alk-NR₄R₅
• -Ph oder
• -CH₃Ph und
• Ph für einen Phenylrest steht, der gegebenenfalls mit einer oder mehreren Gruppen substituiert ist, die ausgewählt sind aus Halogenatomen, Alkoxyresten mit 1 bis 5 Kohlenstoffatomen und Resten -COOR₆, wobei R₆ wie vorstehend definiert ist;
• R₁ für ein Wasserstoffatom, ein Halogenatom, einen Cyanorest, einen Rest -COOR₆ oder einen Rest der folgenden Formel steht:
• -NR₄R₅
• -NH-SO₂-Alk
• -NH-CO-CF₃
• -NH-CO-Ph
• -NH-CO-Alk
• -NH-CO₂-Alk
• -CONR₄R₅
• einen Phenylrest, der gegebenenfalls mit einer oder mehreren Gruppen substituiert ist, die ausgewählt sind aus Halogenatomen, Alkylresten mit 1 bis 5 Kohlenstoffatomen, Alkoxygruppen mit 1 bis 5 Kohlenstoffatomen und Resten -COOR₆,
• einen 5-gliedrigen Heteroarylrest, der ein Heteroatom enthält, das ausgewählt wird aus einem Schwefelatom, eine Sauerstoffatom oder einem Stickstoffatom, und der gegebenenfalls ein zweites Stickstoffatom umfasst, wobei der Heteroaryl gegebenenfalls mit einer oder mehreren Gruppen substituiert ist, die ausgewählt sind aus Halogenatomen, Alkylresten mit 1 bis 5 Kohlenstoffatomen, Alkoxyresten mit 1 bis 5 Kohlenstoffatomen und Resten -COOR₆, oder
• einen 6-gliedrigen Heteroarylrest, der 1 oder 2 Stickstoffatome enthält und gegebenenfalls mit einer oder mehreren Gruppen substituiert ist, die ausgewählt sind aus Halogenatomen, Alkylresten mit 1 bis 5 Kohlenstoffatomen, Alkoxyresten mit 1 bis 5 Kohlenstoffatomen und Resten -COOR₆,
worin Alk, Ph, R₄, R₅, und R₆ wie vorstehend definiert sind;
• R₂ und R₃ unabhängig voneinander für einen Hydroxyrest, einen Alkoxyrest mit 1 bis 5 Kohlenstoffatomen, einen Aminorest, einen Rest -COOR₆, einen Nitrorest oder einen Rest der folgenden Formel stehen:
• -NR₄R₅
• -NH-CO-Alk
• -NH-CO-Ph
• -NH-CO₂-Alk
• -NH-SO₂-Alk
• -CO-NR₄R₅ oder
• -CO-NHOH,
worin Alk, Ph, R₄, R₅ und R₆ wie vorstehend definiert sind;
oder R₂ und R₃ auch zusammen mit den Kohlenstoffatomen des Phenylrings, an den sie gebunden sind, einen Kohlenstoffring mit 6 Ringgliedern bilden, der ein Stickstoffatom und ein anderes Heteroatom, wie ein Sauerstoffatom, enthält,
in Basen- oder Salzform sowie als Hydrat oder Solvat.

2. Verbindung der Formel I nach Anspruch 1, in der:
• R, der an den Positionen 6, 7 oder 8 des Imidazo[1,5-a]pyridins vorliegt, für ein Wasserstoffatom, einen Alkylrest mit 1 bis 5 Kohlenstoffatomen, einen Alkoxyrest mit 1 bis 5 Kohlenstoffatomen, einen Hydroxyrest, einen Rest -COOR₆ oder einen Rest der folgenden Formel steht:
• -NR₄R₅
• -NH-SO₂-Alk
• -NH-CO-Alk
• -NR₆-CO₂-Alk
• -O-Alk-COOR₆
• -O-Alk-NR₄R₅
• -O-CH₂-Ph
• -CO-NR₄R₅ oder
• -CO-NH-CH(R₇)-(CH₂)ₘ-COOR
worin Alk, Ph, R₄, R₅, R₆, R₇ und m wie im Anspruch 1 definiert sind;
• R₁ für ein Wasserstoffatom, ein Halogenatom, einen Cyanorest, einen Rest -COOR₆ oder einen Rest der folgenden Formel steht:
• -NR₄R₅
• -NH-SO₂-Alk
• -NH-CO-CF₃
• -NH-CO-Ph
• -NH-CO-Alk
• -CO-NR₄R₅
• einen Phenylrest, der gegebenenfalls mit einer oder zwei Gruppen substituiert ist, die ausgewählt sind aus Halogenatomen, Alkoxyresten mit 1 bis 5 Kohlenstoffatomen und Resten -COOR₆,
• einen 5-gliedrigen Heteroarylrest, der ein Heteroatom enthält, das ausgewählt wird aus einem Schwefelatom, einem Sauerstoffatom oder einem Stickstoffatom, und der gegebenenfalls ein zweites Stickstoffatom umfasst, wobei der Heteroaryl gegebenenfalls mit einer oder zwei Gruppen substituiert ist, die ausgewählt sind aus Halogenatomen, Alkylresten mit 1 bis 5 Kohlenstoffatomen, Alkoxyresten mit 1 bis 5 Kohlenstoffatomen und Resten -COOR₆, oder
• einen 6-gliedrigen Heteroarylrest, der 1 oder 2 Stickstoffatome enthält und gegebenenfalls mit einer oder zwei Gruppen substituiert ist, die ausgewählt sind aus Halogenatomen, Alkylresten mit 1 bis 5 Kohlenstoffatomen, Alkoxyreste mit 1 bis 5 Kohlenstoffatomen und Resten -COOR₆,
wobei Alk, Ph und R₆ wie im Anspruch 1 definiert sind;
• R₂ und R₃ unabhängig voneinander für einen Alkoxyrest mit 1 bis 5 Kohlenstoffatomen, einen Rest -COOR₆, einen Aminorest, einen Nitrorest oder einen Rest der folgenden Formel stehen:
• -NR₄R₅
• -NH-CO-Alk
• -NH-CO-Ph
• -NH-SO₂-Alk,
worin Alk, Ph, R₄, R₅ und R₆ wie im Anspruch 1 definiert sind,
in Basen- oder Salzform sowie als Hydrat oder Solvat.

3. Verbindung der Formel I nach Anspruch 1 oder 2, in der:
• R, der an den Positionen 6, 7 oder 8 des Imidazo[1,5-a]pyridins vorliegt, für ein Wasserstoffatom, einen Alkoxyrest mit 1 bis 5 Kohlenstoffatomen, einen Hydroxyrest, einen Rest -COOR₆ oder einen Rest der folgenden Formel steht:
• -NR₄R₅
• -NH-SO₂-Alk
• -NH-CO-Alk
• -NR₆-CO₂-Alk
• -O-Alk-COOR₆
• -CO-NR₄R₅ oder
• -CO-NH-CH(R₇)-(CH₂)ₘ-COOR₆,
worin m für 0 oder 1 steht, R₇ für ein Wasserstoffatom, einen Alkylrest mit 1 bis 5 Kohlenstoffatomen oder einen Rest der Formel -Alk-OR₆ oder -CH₂-Ph steht und Alk, R₄, R₅ und R₆ wie im Anspruch 1 definiert sind;
• R₁ für ein Wasserstoffatom, ein Halogenatom, einen Cyanorest, einen Rest -COOR₆ oder einen Rest der folgenden Formel steht:
• -NR₄R₅
• -NH-SO₂-Alk
• -NH-CO-Ph
• -NH-CO-Alk
• einen Phenylrest, der gegebenenfalls mit einer oder zwei Gruppen substituiert ist, die ausgewählt sind aus Halogenatomen, Alkoxyresten mit 1 bis 5 Kohlenstoffatomen und Resten -COOR₆,
• einen Heteroarylrest, der aus Thienyl-, Furyl- und Pyrrolylresten ausgewählt wird, wobei der Heteroaryl gegebenenfalls mit einer oder zwei Gruppen substituiert ist, die ausgewählt sind aus Alkoxyreste mit 1 bis 5 Kohlenstoffatomen und Resten -COOR₆, oder
• einen Pyridinylrest, der gegebenenfalls mit einer oder zwei Gruppen substituiert ist, die ausgewählt sind aus Alkoxyresten mit 1 bis 5 Kohlenstoffatomen und Resten -COOR₆,
worin Alk, Ph, R₄ und R₆ wie im Anspruch 1 definiert sind;
• R₂ und R₃ unabhängig voneinander für einen Alkoxyrest mit 1 bis 5 Kohlenstoffatomen, einen Rest -COOR₆, einen Nitrorest, einen Aminorest oder einen Rest der Formel-NH-CO-Alk, H-CO-Ph oder -NH-SO₂-Alk stehen,
worin Alk, Ph und R₆ wie im Anspruch 1 definiert sind,
in Basen- oder Salzform sowie als Hydrat oder Solvat.

4. Verbindung der Formel I nach einem der Ansprüche 1 bis 3, worin R₂ für einen Alkoxyrest mit 1 bis 5 Kohlenstoffatomen oder einen Rest -COOR₆ steht, wobei R₆ die im Anspruch 1 definierte Bedeutung hat,
in Basen- oder Salzform sowie als Hydrat oder Solvat.

5. Verbindung der Formel I nach einem der Ansprüche 1 bis 4, worin R₃ für einen Nitrorest, einen Aminorest oder einen Rest der Formel -NH-CO-Alk, -NH-CO-Ph oder -NH-SO₂-Alk steht, wobei Alk und Ph wie im Anspruch 1 definiert sind,
in Basen- oder Salzform sowie als Hydrat oder Solvat.

6. Verbindung der Formel I nach einem der Ansprüche 1 bis 5, in der:
• R, der an den Positionen 6, 7 oder 8 des Imidazo[1,5-a]pyridins vorliegt, für ein Wasserstoffatom, einen Hydroxyrest, einen Rest -COOR₆ oder einen Rest der folgenden Formel steht:
• -O-Alk-COOR₆
• -CO-NR₄R₅ oder
• -CO-NH-CH (R₇) -COOR₆,
worin R₇ für ein Wasserstoffatom, einen Alkylrest mit 1 bis 5 Kohlenstoffatomen oder einen Rest der Formel -Alk-OR₆ steht und Alk, R₄, R₅ und R₆ wie im Anspruch 1 definiert sind;
• R₁ für ein Wasserstoffatom, ein Halogenatom, einen Rest -COOR₆ oder einen Rest der folgenden Formel steht:
• -NH-CO-Ph
• einen Phenylrest, der egebenenfalls mit einer oder zwei Gruppen substituiert ist, die aus gewählt sind aus Halogenatomen, Alkoxyresten mit 1 bis 5 Kohlenstoffatomen und Resten -COOR₆, oder
• einen Thienylrest, der gegebenenfalls mit einer oder zwei Gruppen substituiert ist, die ausgewählt sind aus Alkoxyresten mit 1 bis 5 Kohlenstoffatomen und Resten -COOR₆,
worin Ph und R₆ wie im Anspruch 1 definiert sind;
• R₂ für einen Alkoxyrest mit 1 bis 5 Kohlenstoffatomen oder einen Rest -COOR₆ steht, worin R₆ wie im Anspruch 1 definiert ist, und
• R₃ für einen Aminorest steht,
in Basen- oder Salzform sowie als Hydrat oder Solvat.

7. Verfahren zur Herstellung von Verbindungen der Formel I nach einem der vorhergehenden Ansprüche, **dadurch gekennzeichnet, dass** man
A) die Verbindung der Formel II: 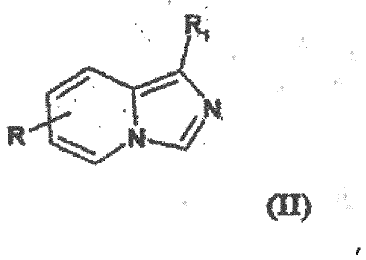 in der R die für die Verbindung der Formel I nach Anspruch 1 definierte Bedeutung hat; jedoch R kein Rest ist, der mit der Verbindung der Formel III reagieren kann, wie ein Hydroxyrest, ein Carboxyrest oder ein Rest -NR₄R₅, und R kein Rest -NH-CO₂R₆ oder kein Rest -CONR₄R₅ ist,
mit der Verbindung der Formel III kondensiert: 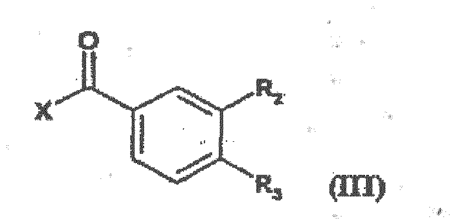 in der X für ein Halogenatom steht und R₂ und R₃ unabhängig voneinander für einen Alkoxyrest mit 1 bis 5 Kohlenstoffatomen, einen Nitrorest oder einen Rest -COOR₆ stehen, worin R₆ für einen Alkylrest mit 1 bis 5 Kohlenstoffatomen steht, so dass:
- die Verbindungen der Formel Ia erhalten werden, d.h. Verbindungen der Formel I, in der R₂ oder R₃ für einen Nitrorest stehen, oder
- die Verbindungen der Formel Ib erhalten werden, d.h. Verbindungen der Formel I, in der R₂ oder R₃ für einen Rest -COOR₆ stehen, worin R₆ für einen Alkylrest mit 1 bis 5 Kohlenstoffatomen steht,
und anschließend
a) die Verbindungen der Formel Ia einer Reduktionsreaktion unterzieht, so dass die Verbindungen der Formel Id erhalten werden: 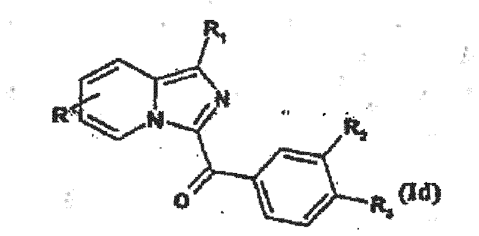 Verbindungen der Formel (I), worin
R₂ oder R₃ = NH₂,
in der R und R₁ wie für die Verbindung der Formel Ia definiert sind und R₂ oder R₃ für einen Aminorest steht;
die Verbindungen der Formel Id können anschließend einer Alkylierungs-, Acylierungs- oder Sulfonierungsreaktion unterzogen werden, so dass die Verbindungen der Formel Ig erhalten werden: 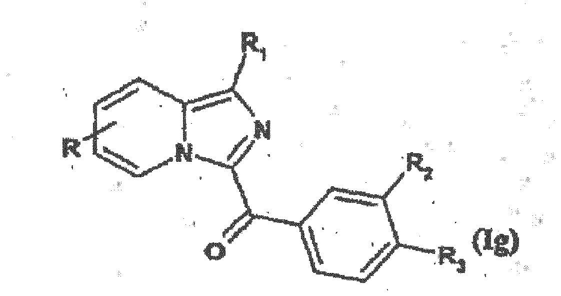 Verbindungen der Formel (I), worin R₂ oder R₃ = NR₄R₅, NHCO-Alk, NHCO₂-Alk oder NHSO₂-Alk,
in der R und R₁ wie für die Verbindung der Formel Id definiert sind und R₂ oder R₃ für einen Rest -NR₄R₅, einen Rest -NHCOAlk, einen Rest -NHCO₂Alk oder einen Rest -NHSO₂Alk steht;
b) oder die Verbindungen der Formel Ib einer Verseifungsreaktion unterzieht, so dass die Verbindungen der Formel Ie erhalten werden: 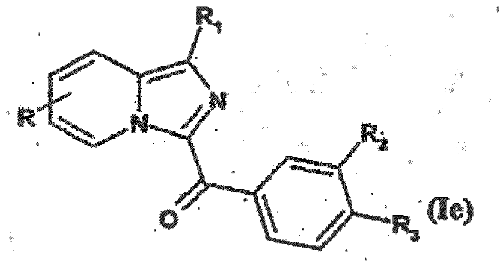 Verbindungen der Formel (I), worin
R₂ oder R₃ = CO₂H,
in der R und R₁ wie für die Verbindung der Formel Ib definiert sind und R₂ oder R₃ für einen Carboxyrest steht;
die Verbindungen Formel Ie können anschließend einer Kupplungsreaktion nach Aktivierung der Carboxyfunktion in Gegenwart einer Base und mit anschließender Zugabe eines Amins der Formel HNR₄R₅ oder von Hydroxylamin unterzogen werden, so dass die Verbindungen der Formel Ih erhalten.werden: 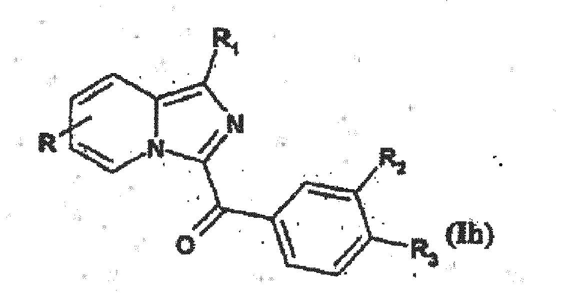 Verbindungen der Formel (I), worin
R₂ oder R₃ = CONR₄R₅ oder CONHOH,
in der R und R₁ wie für die Verbindung der Formel Ie definiert sind und R₂ oder R₃ für einen Rest CONR₄R₅ oder CONHOH steht;
ODER
B) die Werbindung der Formel II, wie vorstehend unter Punkt A) definiert, mit der Verbindung der Formel III' kondensiert: 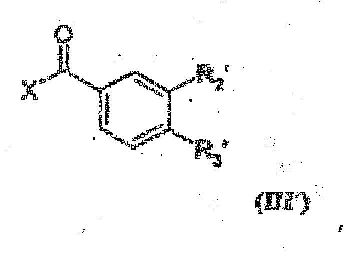 in der X für ein Halogenatom steht und R_{2'} und R₃' zusammen mit den Kohlenstoffatomen des Phenylkerns, an den sie gebunden sind, einen Kohlenstoffring mit 6 Ringgliedern bilden, der ein Stickstoffatom und ein anderes Heteroatom enthält,
so dass die Verbindungen der Formel Ic erhalten werden: 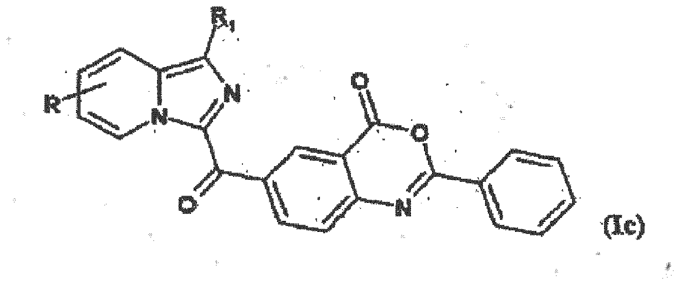 in der R und R₁ wie für die Verbindung der Formel II definiert sind,
wobei die Verbindungen der Formel Ic anschließend einer Alkoholysereaktion unterzogen werden, so dass die Verbindungen der Formel If erhalten werden : 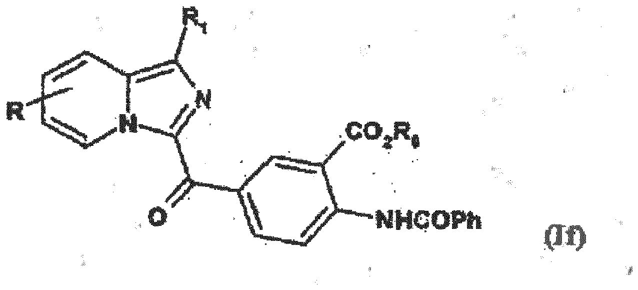 in der R und R₁ wie für die Verbindung der Formel II definiert sind und R₆ wie für die Verbindung der Formel I definiert ist,
die Verbindungen der Formel If können anschließend verseift werden, so dass die Verbindungen der Formeln Id oder Ie erhalten werden, in denen R und R₁ wie für die Verbindung der Formel II definiert sind, R₂ für einen Rest -COOH steht und R₃ für einen Rest -NH₂ steht;
ODER
C) die Verbindung der Formel I, in der R₁ für ein Wasserstoffatom steht, wie sie vorstehend unter Punkt A) erhalten wird, einer Bromierungsreaktion unterzieht, so dass die Verbindungen der Formel Ii erhalten werden: 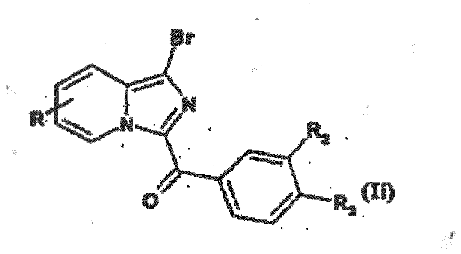 in der R, R₂ und R₃ wie für die Verbindung der Formel I nach Anspruch 1 definiert sind, wenn R₂ und R₃ nicht zusammen einen Heteroaryl bilden und R₁ für ein Bromatom steht,
die Verbindungen der Formel Ii, in denen R kein Brom- oder Iodatom ist, können in Gegenwart eines Katalysator, eines Liganden und einer Base:
a) entweder einer Iminierungsreaktion mit Benzophenonimin und danach einer sauren Hydrolysereaktion unterzogen werden, so dass die Verbindungen der Formel Ij erhalten werden: 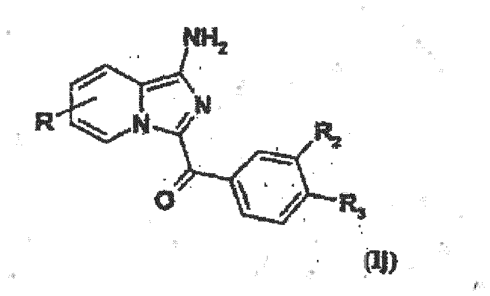 in der R wie für die Verbindungen Ii definiert ist und R₂ und R₃ die für die Verbindung der Formel Ii definierte Bedeutung haben und R₁ für einen Rest -NH₂ steht,
b) oder einer Cyanierungsreaktion mit Zinkcyanid unterzogen werden, so dass Verbindungen der Formel Ik erhalten werden: 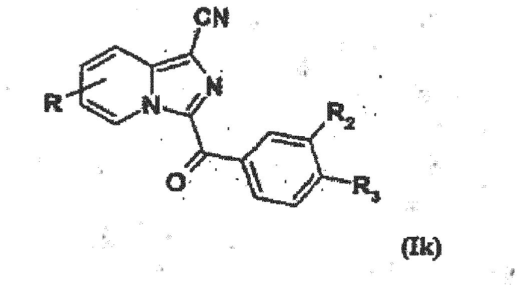 in der R, R₂ und R₃ die für die Verbindungen Ii definierten Bedeutungen haben und R₁ für einen Rest -CN steht,
- die Verbindungen der Formel Ik können anschließend einer basischen Hydrolysereaktion unterzogen werden, so dass die Verbindungen der Formel Im erhalten werden: 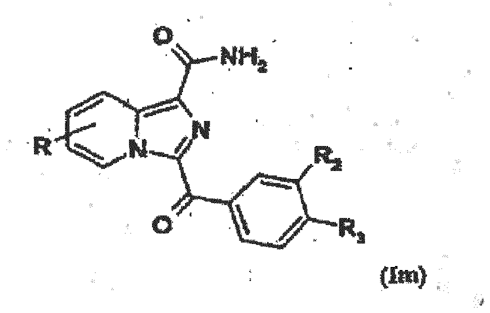 in der R, R₂ und R₃ die für die Verbindungen der Formel Ik definierten Bedeutungen haben und R₁ für einen Rest -CONH₂ steht,
- oder die Verbindungen der Formel Ik werden einer Pinner-Reaktion mit einem primären Alkohol in Gegenwart von Chlorwasserstoffgas unterzogen, wobei der entsprechende Imidoester erhalten wird, der durch saure Hydrolyse zu den Verbindungen Formel In führt: 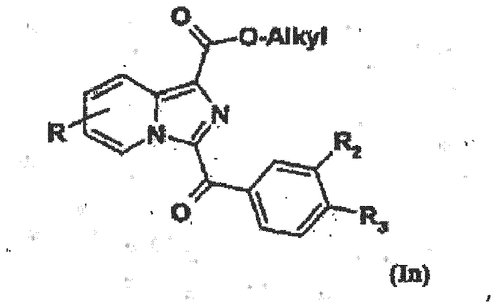
in der R, R₂ und R₃ die für die Verbindungen der Formel Ik definierten Bedeutungen haben und R₁ für einen Rest -CO₂Alk steht, wobei Alk wie im Anspruch 1 definiert ist,
wobei die Verbindungen der Formel In ihrerseits einer Verseifungsreaktion unterzogen werden können, so dass die Verbindungen der Formel Io erhalten werden: 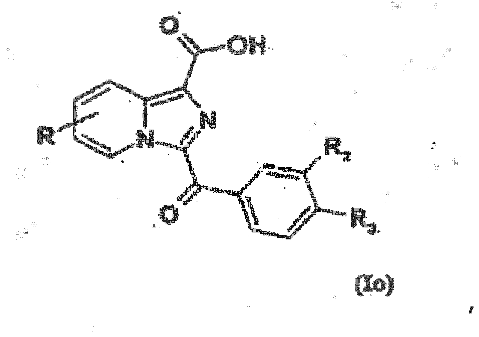 in der R, R₂ und R₃ die für die Verbindungen der Formel Ik definierten Bedeutungen haben und R₁ für einen Rest -CO₂H steht,
c) oder einer Suzuki-Reaktion mit Phenylboron- oder Heteroarylboron-Derivaten unterzogen werden können, so dass die Verbindungen der Formel Is erhalten werden: 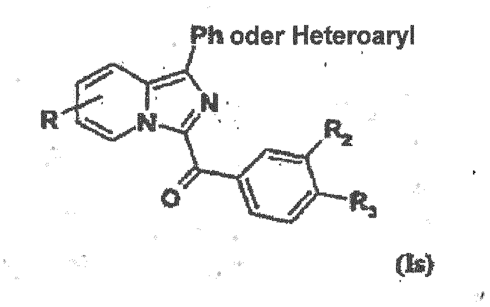 in der R, R₂ und R₃ die für die Verbindungen der Formel Ii definierten Bedeutungen haben und R₁ für einen substituierten Phenylrest oder einen gegebenenfalls substituierten 5- oder 6-gliedrigen Heteroaryl steht,
ODER
D) die Verbindungen der Formel Ij, in der R₁ für einen Aminorest steht, einer Acylierungs- oder Sulfonierungsreaktion unterzieht, so dass die Verbindungen der Formel Ip erhalten werden: 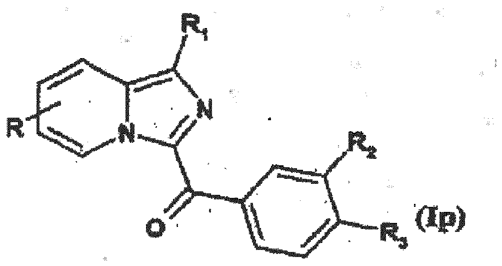 Verbindungen der Formel (I), worin R₁ = NHCO-Alk, NHCO-Ph, NHCCCF₃, NHCO₂-Alk oder NHSO₂-Alk,
in der R, R₂ und R₃ die für die Verbindungen der Formel Ij definierten Bedeutungen haben und R₁ für einen Rest -NHCOAlk, -NHCO₂Alk, -NHSO₂Alk, -NHCOPH oder NHCOCF₃ steht, in denen Alk und Ph wie für die Verbindung der Formel I nach Anspruch 1 definiert sind,
wobei die Verbindungen Formel Ip, in der R₁ für einen Rest -NHCOCF₃ steht, ihrerseits einer Alkylierungsreaktion mit anschließender Entfernung der Schutzgruppe, gegebenenfalls gefolgt von einer weiteren Alkylierungsreaktion, unterzogen werden können, so dass die Verbindungen Formel Iq erhalten werden: 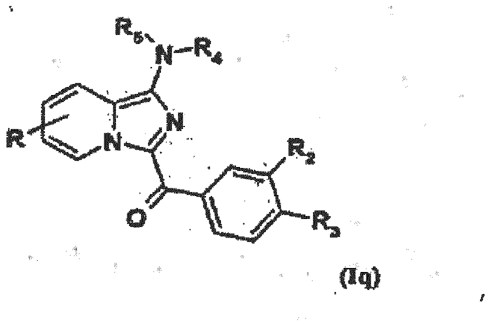 in der R, R₂, R₃ die für die Verbindungen der Formel Ij definierten Bedeutungen haben und R₄ und R₅ wie für die Verbindung der Formel I definiert sind;
ODER
E) die Verbindungen der Formel Ir, in der R für einen Rest -CO₂R₆ steht, wobei R₆ für einen Rest Alk steht, wie sie vorstehend unter Punkt A) erhalten werden, einer sauren oder basischen Hydrolysereaktion unterzieht, so dass die Verbindungen der Formel It erhalten werden: 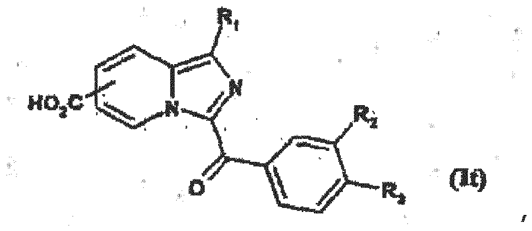 in der R₁, R₂, R₃ die für die Verbindung der Formel Ir definierten Bedeutungen haben und R für einen Rest -COO steht,
die Verbindungen der Formel It können anschließend:
a) entweder einer Kupplungsreaktion nach Aktivierung der Carboxyfunktion in Gegenwart einer Base und mit anschließender Zugabe eines Amins der Formel HNR₄R₅ oder H₂N-CH(R₇)-(CH₂)ₘ-COOR₆, wobei R₆ für einen Rest Alk, steht, wie im Anspruch 1 definiert, unterzogen werden, so dass die Verbindungen der Formel Iu erhalten werden: 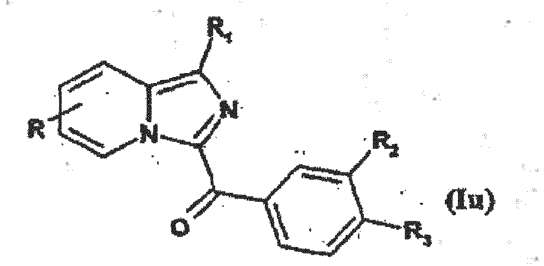 Verbindungen der Formel (I), worin R = CONR₄R₅ oder CONH-CH(R₇)-(CH₂)ₘ-CO₂R₆,
in der R₁, R₂, R₃ die für die Verbindungen der Formel It definierten Bedeutungen haben,
und wenn R ein Rest -CONH-CH(R₇)-(CH₂)ₘ-COOR₆ ist, wobei R₆ für einen Rest Alk steht, wie im Anspruch 1 definiert, können diese Verbindungen verseift werden, so die Verbindungen der Formel Iu erhalten werden, worin R ein Rest -CONH-CH(R₇)-(CH₂)ₘ-COCR₆ ist, wobei R₆ für ein Wasserstoffatom steht, und R₁, R₂, R₃ wie vorstehend definiert sind,
b) oder einer Curtius-Umlagerung durch Einwirkung von Diphenylphosphorylazid in Gegenwart von Triethylamin unter Rückfluss in einem inerten Lösungsmittel und durch anschließende Zugabe eines Alkohols der Formel Alk-OH unterzogen werden, so dass die Verbindungen der der Formel Iv erhalten werden; 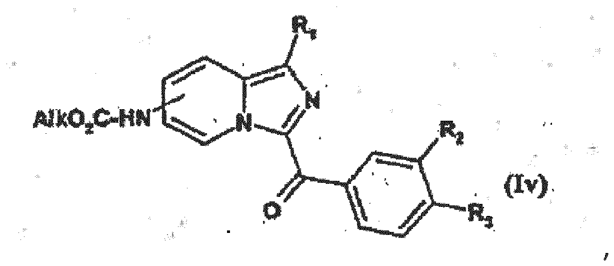 in der R₁, R₂, R₃ die für die Verbindungen der Formel It definierten Bedeutungen haben und R für einen Rest -NHCO₂Alk steht,
- die Verbindungen der Formel Iv, in denen R für einen Rest -NH-CO₂-Alk steht, wobei Alk für einen Rest
- tBu steht, können anschließend zu den Verbindungen der Formel Iw führen, in der R₁, R₂, R₃, R₄, R₅ wie für die Verbindung der Formel I nach Anspruch 1 definiert sind: 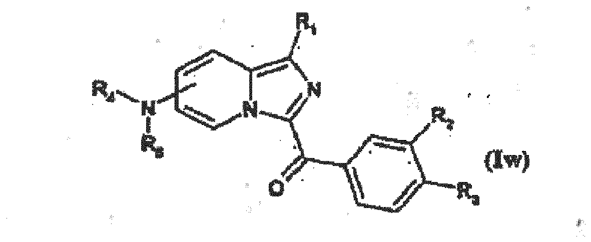
- durch Entfernen der Schutzgruppe in saurem Medium erhält man die Verbindungen der Formel Iw, worin R für einen Rest -NH₂ steht,
- durch Alkylierung und anschließende Entfernung der Schutzgruppe und gegebenenfalls eine zweite Alkylierung kann man die Verbindungen der Formel Iw erhalten, worin R für einen Rest -NR₄R₅ steht,
die Verbindungen der Formel Iw, worin R für einen Rest -NH₂ steht, können entweder acyliert oder sulfoniert werden, so dass die Verbindungen der Formel Ix erhalten werden: 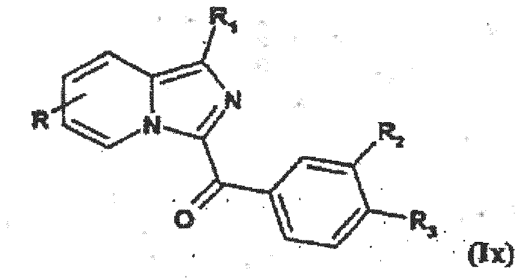 Verbindungen der Formel (I), worin
R = NHCO-Alk oder NHSO₂-Alk,
in der R₁, R₂, R₃ die für die Verbindung der Formel Iw definierten Bedeutungen haben und R für einen Rest -NHCOAlk oder -NHSO₂Alk steht,
ODER
F) die Verbindungen der Formel Iy: 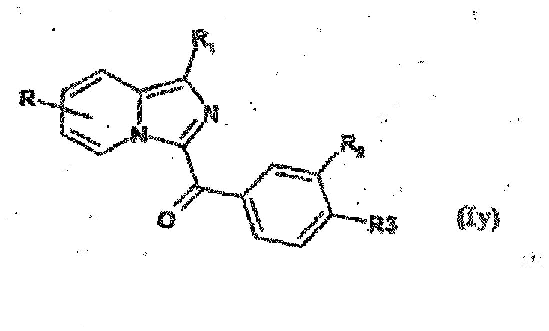 in der R für einen Rest -Obenzyl steht und R₁, R₂ und R₃ die für die Verbindungen der Formel I nach Anspruch 1 definierten Bedeutungen haben, einer Debenzylierungsreaktion in einem protischen Lösungsmittel in Gegenwart von Palladium auf Kohle unterzieht, um die Verbindungen der Formel Iz zu erhalten: 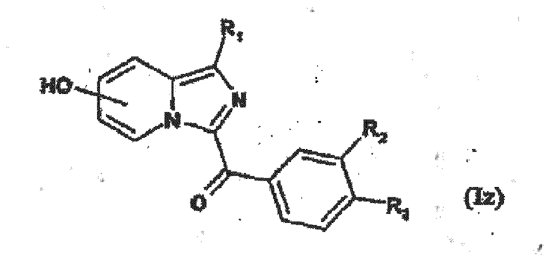 in der R₁, R₂ und R₃ die für die Verbindungen der Formel Iy definierten Bedeutungen haben und R für einen Hydroxyrest steht, und man, wenn R₂ oder R₃ für eine Nitrofunktion steht, die Verbindungen der Formel Id erhält, in der R₂ oder R₃ für einen Rest NH₂ steht und R₁ wie für die Verbindungen der Formel I definiert ist,
die Verbindungen der Formel Iz können abschließend einer selektiven O-Alkylierungsreaktion durch Einwirkung eines Alkylhalogenids bei Umgebungstemperatur in einem polaren Lösungsmittel in Gegenwart eines Alkalimetallcarbonats unterzogen werden, so dass die Verbindungen der Formel Iz, erhalten werden: 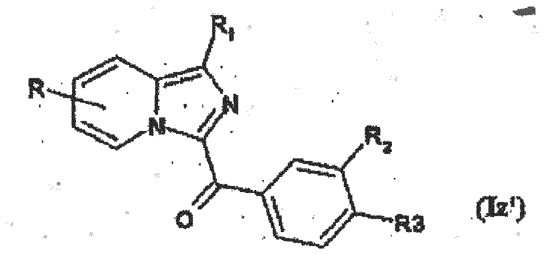 Verbindung der Formel I, worin: R = O-Alk, O-(CH₂)ₘ-Ph, oder O-Alk-CO₂R₆,
in der R₁, R₂ und R₃ die für die Verbindungen der Formel Iz definierten Bedeutungen haben,
und wenn R ein Rest -O-Alk-COOR₆ ist, worin R₆ für einen Rest Alk steht, wie für die Verbindungen der Formel I definiert, können diese Verbindungen verseift werden, so dass die Verbindungen der Formel Iz' erhalten werden, worin R ein Rest -O-Alk-COOR₆ ist, worin R₆ für ein Wasserstoffatom steht, und R₁, R₂ und R₃ wie vorstehend definiert sind.

8. Arzneimittel, **dadurch gekennzeichnet, dass** es eine Verbindung der Formel I nach einem der Ansprüche 1 bis 6 oder ein Additionssalz dieser Verbindung mit einer pharmazeutisch unbedenklichen Säure oder Base oder auch ein Hydrat oder ein Solvat der Verbindung der Formel I umfasst.

9. Pharmazeutische Zusammensetzung, **dadurch gekennzeichnet, dass** sie eine Verbindung der Formel I nach einem der Ansprüche 1 bis 6 oder ein pharmazeutisch unbedenkliches Salz, ein Hydrat oder ein Solvat dieser Verbindung sowie mindestens einen pharmazeutisch unbedenklichen Excipienten umfasst.

10. Verwendung einer Verbindung der Formel I nach einem der Ansprüche 1 bis 6 zur Herstellung eines Arzneimittels zur Behandlung von Krebserkrankungen, insbesondere von Karzinomen mit einem erheblichen Grad an Vaskularisierung, wie Karzinomen der Lunge, der Brust, der Prostata und des Ösophagus, Metastasen induzierenden Krebsarten, wie Dickdarmkrebs und Magenkrebs, Melanomen, Gliomen, Lymphomen und Leukämien.

11. Verwendung nach Anspruch 10, **dadurch gekennzeichnet, dass** man zu der Verbindung der Formel I einen oder mehrere Antikrebswirkstoff(e) und/oder eine Strahlentherapie hinzugesellt.

12. Verwendung nach Anspruch 10 zur Herstellung eines Arzneimittels zur Behandlung von kardiovaskulären Erkrankungen, wie Atherosklerose, Restenose nach Angioplastie, Erkrankungen, die mit Komplikationen, die nach dem Einsetzen endovaskulärer Prothesen und/oder aortokoronarer , Bypässe oder anderer Gefäßtransplantate für Herzhypertrophie auftreten, oder vaskulären Komplikationen von Diabetes, wie diabetischen Retinopathien, einhergehen.

13. Verwendung nach Anspruch 10 zur Herstellung eines Arzneimittels zur Behandlung von chronischen entzündlichen Erkrankungen, wie rheumatoide Arthritis oder IBD.

14. Verwendung nach Anspruch 10 zur Herstellung eines Arzneimittels zur Behandlung von Osteoarthritis, Achondroplasien (ACH), Hypochondroplasien (HCH) und TD (thanatophorer Dysplasie).

15. Verwendung nach Anspruch 10 zur Herstellung eines Arzneimittels zur Behandlung von Fettleibigkeit.

16. Verwendung nach Anspruch 10 zur Herstellung eines Arzneimittels zur Behandlung von Makuladegeneration, wie altersbedingte Makuladegeneration (AMD).
